# EUROPEAN PATENT APPLICATION

(11) **EP 4 756 030 A1**
(43) Date of publication of application: **10.06.2026**
(21) Application number: 24851783.1
(22) Date of filing: 02.08.2024
(51) Int. Cl.: C12N 15/13, A61K 35/17, A61P 35/00, A61P 37/04, C07K 14/725, C07K 16/28, C07K 19/00, C12N 5/10, C12N 5/0789, C12N 15/12, C12N 15/62

(54) **METHOD FOR PRODUCING IMMUNE CELLS**

(30) Priority: 04.08.2023 JP 2023127747
(71) Applicant: TOKYO UNIVERSITY OF SCIENCE FOUNDATION, Tokyo 162-8601 (JP)
(72) Inventor: IKAWA, Tomokatsu, Tokyo 162-8601 (JP); SHIGEHIRO, Tsukasa, Tokyo 162-8601 (JP); HARUYAMA, Munetada, Tokyo 103-8426 (JP)
(74) Representative: Mewburn Ellis LLP
(86) International application number: PCT/JP2024/027781
(87) International publication number: WO 2025/033358

(57) **Abstract**

Means capable of stably producing TCR-T cells and CAR-T/CAR-NK cells, and means for treating diseases such as tumors and autoimmune diseases by applying the same, are disclosed. One aspect of a production method of a TCR/CAR/CAAR-expressing immune cell according to the present invention is a method of producing a TCR/CAR/CAAR-expressing immune cell by introducing an exogenous receptor gene into multipotent blood progenitor cells and then inducing differentiation thereof into immune cells. Another aspect of the production method of a TCR/CAR/CAAR-expressing immune cell according to the present invention is a method of producing a TCR/CAR/CAAR-expressing immune cell by inducing differentiation of multipotent blood progenitor cells into immune cells and then introducing thereinto an exogenous receptor gene. As the multipotent blood progenitor cell, an induced leukocyte stem cell (iLS) or a T/NK progenitor cell can be preferably used.

## Description

### TECHNICAL FIELD

The present invention relates to a method of producing an immune cell expressing an exogenous receptor such as a T-cell receptor (TCR) or a chimeric antigen receptor (CAR) that specifically recognizes a desired antigen, a method of producing an immune cell therapy agent using the aforementioned method, a method of treating a disease using the immune cell, and a method of producing T/NK progenitor cells in large quantities, and the like.

### BACKGROUND ART

T cells (TCR-T cells, CAR-T cells) into which a tumor-specific TCR or a chimeric antigen receptor (CAR) has been introduced and NK cells (CAR-NK cells) into which a CAR has been introduced hold promise as new cancer treatment strategies. As methods for producing TCR-T cells, CAR-T cells, and CAR-NK cells used in immune cell therapy, there have been proposed a method of producing them by introducing a TCR gene or a CAR gene into T cells/NK cells collected from a patient themselves, T cells/NK cells derived from umbilical cord blood or the like, or a known T-cell line/NK-cell line, as well as a method of preparing CAR-NK cells by introducing a CAR gene into iPS cells and then inducing differentiation into NK cells (Non-Patent Document 1). In commercially available CAR-T therapy (Kymriah (registered trademark), Yescarta (registered trademark)), CAR-T cells are produced from leukocytes collected from a patient themselves by leukapheresis.

However, it has been pointed out that TCR-T cells and CAR-T/CAR-NK cells are exhausted in a patient's body and cannot maintain sufficient cytotoxic activity. From the viewpoint of maintaining therapeutic effects by repeating cell transplantation as appropriate, it is essential to develop a method of stably producing these tumor-specific immune cells, but the development has been insufficient.

On the other hand, various techniques for expanding hematopoietic cells while maintaining multipotency and self-renewal ability are known. One of them is a method capable of expanding mouse and human multipotent blood progenitor cells in large quantities, which was developed by the group of the present inventors and is disclosed in Non-Patent Documents 2 and 3 and Patent Document 1. In this method, E2A in cells is inhibited by introducing Id3 into hematopoietic stem/progenitor cells (HSPCs), and then the cells are cultured under conditions that induce differentiation into B cells. As a result, multipotent blood progenitor cells having the ability to differentiate into leukocytes (T cells, B cells, myeloid cells, etc.) and self-renewal ability are expanded in large quantities. Since the multipotent blood progenitor cells obtained in this way primarily produce leukocytes when transplanted into a living body, they were named induced leukocyte stem (iLS) cells. It has been shown that iLS cells can be expanded *ex vivo* for at least several months.

The present inventors have also reported a method of generating and expanding T/NK progenitor cells (pT/NK cells) having the ability to differentiate into T cells and NK cells in a study using mice (Non-Patent Documents 2, 4, and 5). In this method, mouse hematopoietic stem/progenitor cells are cultured in the presence of a Notch ligand, with IL-7, SCF, and Flt3-L added to the medium at 10 ng/mL each. As a result, pT/NK cells having self-renewal ability while maintaining multipotency (ability to differentiate into T cells, NK cells, and myeloid cells) are generated. It has also been confirmed by this method that mouse pT/NK cells can be expanded *ex vivo* for at least several months.

As a method developed by another group, for example, a method is known in which mouse hematopoietic stem cells are expanded and cultured in an undifferentiated state by adding polyvinyl alcohol to the medium instead of serum albumin and culturing the hematopoietic stem cells (Non-Patent Document 6). Also in this method, mouse hematopoietic stem cells can be expanded and cultured in an undifferentiated state for one month or longer.

### PRIOR ART DOCUMENT(S)

### PATENT DOCUMENT(S)

Patent Document 1: WO 2009/113595 A1

### NON-PATENT DOCUMENT(S)

Non-Patent Document 1: Guozhu Xie et al., EBioMedicine 59 (2020) 102975
Non-Patent Document 2: Ikawa et al. Science, vol.329, pp.93-96, 2 July 2010
Non-Patent Document 3: Ikawa et al. Stem Cell Rep, vol. 5, pp. 716-727, November 10, 2015
Non-Patent Document 4: Ikawa et al. J Exp Med. vol. 190, pp.1617-1625, December 6, 1999
Non-Patent Document 5: Ikawa et al. PNAS, vol.98, pp.5164-5169, April 24, 2001
Non-Patent Document 6: Adam C. Wilkinson et al., Nature, vol. 571, pp. 117-121 (2019)

### SUMMARY OF THE INVENTION

### PROBLEMS TO BE SOLVED BY THE INVENTION

One object of the present invention is to provide a means capable of stably producing TCR-T cells and CAR-T/CAR-NK cells. A further object of the present invention is to provide a means for treating a disease by applying the aforementioned means. In addition, as another problem of the present invention, it has been newly confirmed that there is a limit to the expansion of human T/NK progenitor cells in a test in which the methods described in Non-Patent Documents 2, 4, and 5 using mice were applied to human hematopoietic stem cells. Therefore, the object of the present invention also includes developing a new method with improved expansion efficiency of human T/NK progenitor cells for application to human immune cell therapy.

### MEANS FOR SOLVING THE PROBLEMS

As a result of intensive research, the present inventors have found that TCR- or CAR-expressing immune cells can be efficiently and stably prepared by introducing a TCR gene or a CAR gene into multipotent blood progenitor cells such as induced leukocyte stem cells or T/NK progenitor cells, expanding the cells, and inducing their differentiation into immune cells such as T cells, NK cells, or macrophages. The present inventors have also found that TCR- or CAR-expressing immune cells can be produced in large quantities by preparing these multipotent blood progenitor cells by the expansion method of induced leukocyte stem cells or T/NK progenitor cells developed by the group of the present inventors. Furthermore, the present inventors have found that TCR- or CAR-expressing immune cells can be efficiently and stably prepared even when the multipotent blood progenitor cells prepared by the aforementioned expansion method are differentiated into immune cells and then a TCR or CAR gene is introduced thereinto. In addition, based on previous research by the present inventors, it has been revealed that, in the preparation of mouse T/NK progenitor cells, the expansion efficiency is further improved by increasing the concentrations of IL-7, SCF, and Flt3-L to about 25 to 50 ng/mL, which is higher than the 10 ng/mL reported in Non-Patent Documents 2, 4, and 5. However, in the preparation of human T/NK progenitor cells, when these cytokines were used at high concentrations of 10 to 50 ng/mL each as in mouse cells, the expansion efficiency was limited to about 10 to 50 times, and furthermore, a problem was also found in that many cells were generated whose differentiation did not stop at the T/NK progenitor cell stage but proceeded further to NK cells. As a result of intensive studies to solve these problems, the inventors found that the expansion efficiency of human pT/NK cells is markedly increased by reducing the concentrations of SCF, Flt3-L, and IL-7 to less than 10 ng/mL in the preparation step of human T/NK progenitor cells. They further found that expansion of 300 to 600 times is achieved under culture conditions in combination with TPO or M-CSF, and that human CAR-NK cells prepared from human T/NK progenitor cells produced under the said culture conditions have good anti-tumor effects. By further proceeding with research based on these findings, the present inventors have completed the present invention including the following aspects.

[1] A method of producing an immune cell expressing an exogenous receptor that is a T-cell receptor (TCR), a chimeric antigen receptor (CAR), or a chimeric autoantigen receptor (CAAR), the method comprising the steps of:
   preparing multipotent blood progenitor cells from a cell population comprising hematopoietic stem cells;
   preparing multipotent blood progenitor cells expressing said exogenous receptor by introducing a gene encoding said exogenous receptor into the multipotent blood progenitor cells; and
   inducing differentiation of the multipotent blood progenitor cells expressing said exogenous receptor into immune cells.
[2] A method of producing an immune cell expressing an exogenous receptor that is a TCR, a CAR, or a CAAR, the method comprising the steps of:
   preparing multipotent blood progenitor cells from a cell population comprising hematopoietic stem cells;
   inducing differentiation of the multipotent blood progenitor cells into immune cells; and
   introducing a gene encoding said exogenous receptor into the immune cells,
   wherein the multipotent blood progenitor cell is an induced leukocyte stem cell or a T/NK progenitor cell.
[3] The method according to [1], wherein the multipotent blood progenitor cell is an induced leukocyte stem cell or a T/NK progenitor cell.
[4] The method according to [1] or [2], wherein the multipotent blood progenitor cell is an induced leukocyte stem cell, and in the step of preparing multipotent blood progenitor cells, induced leukocyte stem cells are prepared by culturing a cell population comprising hematopoietic stem cells under conditions that induce differentiation into B cells while inhibiting a function of transcription factor E2A in the cells.
[5] The method according to [4], wherein the inhibition of the function of E2A is performed by forced expression of a differentiation inhibitory factor Id in the hematopoietic stem cells.
[6] The method according to [1] or [2], wherein the multipotent blood progenitor cell is a T/NK progenitor cell, and in the step of preparing multipotent blood progenitor cells, T/NK progenitor cells are prepared by culturing a cell population comprising hematopoietic stem cells in the presence of a Notch ligand.
[7] The method according to [6], wherein the cell population comprising hematopoietic stem cells is cultured in a medium comprising SCF, TPO, and G-CSF at concentrations of 100 ng/mL or more each in the absence of a Notch ligand, and then cultured in the presence of a Notch ligand.
[8] The method according to any one of [1] to [7], wherein the immune cell is a T cell, an NK cell, a macrophage, or a B cell.
[9] The method according to [8], wherein the exogenous receptor is a TCR and the immune cell is a T cell.
[10] The method according to [8], wherein the exogenous receptor is a CAR or a CAAR, and the immune cell is a T cell, an NK cell, or a macrophage.
[11] The method according to any one of [1] to [10], wherein the exogenous receptor is an antigen-specific receptor and the antigen is a tumor antigen.
[12] A method of producing an immune cell therapy agent for a disease treatable by immune cell therapy (e.g., a tumor or an autoimmune disease), the method comprising the steps of:
   preparing multipotent blood progenitor cells from a cell population comprising hematopoietic stem cells;
   introducing a gene encoding an exogenous receptor into the multipotent blood progenitor cells to prepare multipotent blood progenitor cells expressing the exogenous receptor, wherein the exogenous receptor is a CAAR, or a TCR or CAR that specifically recognizes a target antigen (e.g., a tumor antigen);
   inducing differentiation of the multipotent blood progenitor cells expressing said exogenous receptor into T cells to prepare T cells expressing said exogenous receptor (TCR-T, CAR-T, or CAAR-T cells);
   subjecting the TCR-T, CAR-T, or CAAR-T cells to TCR stimulation to induce differentiation into CD8-positive cytotoxic TCR-T, CAR-T, or CAAR-T cells; and
   preparing the CD8-positive cytotoxic TCR-T or CAR-T cells as an immune cell therapy agent for a disease caused by cells expressing said antigen (e.g., a tumor expressing a tumor antigen), or preparing the CD8-positive cytotoxic CAAR-T cells as an immune cell therapy agent for an autoimmune disease.
[13] A method of producing an immune cell therapy agent for a disease treatable by immune cell therapy (e.g., a tumor or an autoimmune disease), the method comprising the steps of:
   preparing multipotent blood progenitor cells from a cell population comprising hematopoietic stem cells;
   inducing differentiation of the multipotent blood progenitor cells into T cells;
   subjecting the T cells to TCR stimulation and then introducing thereinto a gene encoding an exogenous receptor, wherein the exogenous receptor is a CAAR, or a TCR or CAR that specifically recognizes a target antigen (e.g., a tumor antigen), and thereby preparing CD8-positive cytotoxic TCR-T, CAR-T, or CAAR-T cells; and
   preparing the CD8-positive cytotoxic TCR-T or CAR-T cells as an immune cell therapy agent for a disease caused by cells expressing said antigen (e.g., a tumor expressing a tumor antigen), or preparing the CD8-positive cytotoxic CAAR-T cells as an immune cell therapy agent for an autoimmune disease,
   wherein the multipotent blood progenitor cell is an induced leukocyte stem cell or a T/NK progenitor cell.
[14] A method of producing an immune cell therapy agent for a disease treatable by immune cell therapy (e.g., a tumor or an autoimmune disease), the method comprising the steps of:
   preparing multipotent blood progenitor cells from a cell population comprising hematopoietic stem cells;
   introducing a gene encoding a chimeric receptor into the multipotent blood progenitor cells, wherein the chimeric receptor is a CAAR, or a CAR that specifically recognizes a tumor antigen, and thereby preparing multipotent blood progenitor cells expressing said chimeric receptor;
   inducing differentiation of the multipotent blood progenitor cells expressing said chimeric receptor into NK cells or macrophages to prepare NK cells (CAR-NK or CAAR-NK cells) or macrophages (CAR-macrophages or CAAR-macrophages), each expressing said chimeric receptor; and
   preparing the CAR-NK cells or CAR-macrophages as an immune cell therapy agent for a disease caused by cells expressing said antigen (e.g., a tumor expressing a tumor antigen), or preparing the CAAR-NK cells or CAAR-macrophages as an immune cell therapy agent for an autoimmune disease.
[15] The method according to [14], further comprising a step of subjecting the prepared NK cells or macrophages expressing said chimeric receptor to receptor stimulation, wherein the chimeric receptor-expressing NK cells or macrophages after the receptor stimulation are prepared as the immune cell therapy agent.
[16] A method of producing an immune cell therapy agent for a disease treatable by immune cell therapy (e.g., a tumor or an autoimmune disease), the method comprising the steps of:
   preparing multipotent blood progenitor cells from a cell population comprising hematopoietic stem cells;
   inducing differentiation of the multipotent blood progenitor cells into NK cells or macrophages;
   subjecting the NK cells or the macrophages to receptor stimulation and then introducing thereinto a gene encoding a chimeric receptor, wherein the chimeric receptor is a CAAR, or a CAR that specifically recognizes a target antigen (e.g., a tumor antigen), and thereby preparing NK cells (CAR-NK or CAAR-NK cells) or macrophages (CAR-macrophages or CAAR-macrophages), each expressing said chimeric receptor; and
   preparing the CAR-NK cells or CAR-macrophages as an immune cell therapy agent for a disease caused by cells expressing said antigen (e.g., a tumor expressing a tumor antigen), or preparing the CAAR-NK cells or CAAR-macrophages as an immune cell therapy agent for an autoimmune disease,
   wherein the multipotent blood progenitor cell is an induced leukocyte stem cell or a T/NK progenitor cell.
[17] A method of treating a disease treatable by immune cell therapy (e.g., a tumor or an autoimmune disease) in a patient, comprising the steps of:
   preparing multipotent blood progenitor cells from a cell population comprising hematopoietic stem cells;
   introducing a gene encoding an exogenous receptor into the multipotent blood progenitor cells to prepare multipotent blood progenitor cells expressing the exogenous receptor, wherein the exogenous receptor is a CAAR, or a TCR or CAR that specifically recognizes a target antigen (e.g., a tumor antigen);
   inducing differentiation of the multipotent blood progenitor cells expressing said exogenous receptor into T cells to prepare TCR-T, CAR-T, or CAAR-T cells that are T cells expressing said exogenous receptor;
   subjecting the TCR-T, CAR-T, or CAAR-T cells to TCR stimulation to induce differentiation into CD8-positive cytotoxic TCR-T, CAR-T, or CAAR-T cells; and
   administering the CD8-positive cytotoxic TCR-T or CAR-T cells to a patient having a disease caused by cells expressing said antigen (e.g., a tumor expressing a tumor antigen), or administering the CD8-positive cytotoxic CAAR-T cells to a patient with an autoimmune disease.
[18] A method of treating a disease treatable by immune cell therapy (e.g., a tumor or an autoimmune disease) in a patient, comprising the steps of:
   preparing multipotent blood progenitor cells from a cell population comprising hematopoietic stem cells;
   inducing differentiation of the multipotent blood progenitor cells into T cells;
   subjecting the T cells to TCR stimulation and then introducing thereinto a gene encoding an exogenous receptor, wherein the exogenous receptor is a CAAR, or a TCR or CAR that specifically recognizes a target antigen (e.g., a tumor antigen), and thereby preparing CD8-positive cytotoxic TCR-T, CAR-T, or CAAR-T cells; and
   administering the CD8-positive cytotoxic TCR-T or CAR-T cells to a patient having a disease caused by cells expressing said antigen (e.g., a tumor expressing a tumor antigen), or administering the CD8-positive cytotoxic CAAR-T cells to a patient with an autoimmune disease,
   wherein the multipotent blood progenitor cell is an induced leukocyte stem cell or a T/NK progenitor cell.
[19] A method of treating a disease treatable by immune cell therapy (e.g., a tumor or an autoimmune disease) in a patient, comprising the steps of:
   preparing multipotent blood progenitor cells from a cell population comprising hematopoietic stem cells;
   introducing a gene encoding a chimeric receptor into the multipotent blood progenitor cells to prepare multipotent blood progenitor cells expressing the chimeric receptor, wherein the chimeric receptor is a CAAR, or a CAR that specifically recognizes a target antigen (e.g., a tumor antigen);
   inducing differentiation of the multipotent blood progenitor cells expressing said chimeric receptor into NK cells or macrophages to prepare chimeric receptor-expressing immune cells that are CAR-NK cells, CAAR-NK cells, CAR-macrophages or CAAR-macrophages; and
   administering the CAR-NK cells or CAR-macrophages to a patient having a disease caused by cells expressing said antigen (e.g., a tumor expressing a tumor antigen), or administering the CAAR-NK cells or CAAR-macrophages to a patient with an autoimmune disease.
[20] The method according to [19], further comprising a step of subjecting the prepared chimeric receptor-expressing immune cells to receptor stimulation, wherein the chimeric receptor-expressing immune cells after the receptor stimulation are administered to the patient.
[21] A method of treating a disease treatable by immune cell therapy (e.g., a tumor or an autoimmune disease) in a patient, comprising the steps of:
   preparing multipotent blood progenitor cells from a cell population comprising hematopoietic stem cells;
   inducing differentiation of the multipotent blood progenitor cells into NK cells or macrophages;
   subjecting the NK cells or macrophages to receptor stimulation and then introducing thereinto a gene encoding a chimeric receptor, wherein the chimeric receptor is a CAAR, or a CAR that specifically recognizes a target antigen (e.g., a tumor antigen), and thereby preparing CAR-NK cells, CAAR-NK, CAR-macrophages or CAAR-macrophages; and
   administering the CAR-NK cells or CAR-macrophages to a patient having a disease caused by cells expressing said antigen (e.g., a tumor expressing a tumor antigen), or administering the CAAR-NK cells or CAAR-macrophages to a patient with an autoimmune disease,
   wherein the multipotent blood progenitor cell is an induced leukocyte stem cell or a T/NK progenitor cell.
[22] A method of treating a disease (e.g., a tumor) caused by cells expressing a target antigen in a patient, comprising the steps of:
   preparing CAR-macrophages by the following (1) or (2):
      (1) preparing multipotent blood progenitor cells from a cell population comprising hematopoietic stem cells, introducing a gene encoding a CAR that specifically recognizes a target antigen (e.g., a tumor antigen) into the multipotent blood progenitor cells to thereby prepare multipotent blood progenitor cells expressing said CAR, and then inducing differentiation of the CAR-expressing multipotent blood progenitor cells into macrophages;
      (2) preparing induced leukocyte stem cells or T/NK progenitor cells from a cell population comprising hematopoietic stem cells, inducing differentiation thereof into macrophages, subjecting the macrophages to receptor stimulation, and then introducing thereinto a gene encoding said CAR;
   preparing CAR-T cells by the following (3) or (4):
      (3) preparing multipotent blood progenitor cells from a cell population comprising hematopoietic stem cells, introducing a gene encoding said CAR into the multipotent blood progenitor cells to thereby prepare multipotent blood progenitor cells expressing said CAR, inducing differentiation of the CAR-expressing multipotent blood progenitor cells into T cells, and then subjecting the T cells to TCR stimulation to thereby induce differentiation into CD8-positive cytotoxic TCR-T or CAR-T cells;
      (4) preparing induced leukocyte stem cells or T/NK progenitor cells from a cell population comprising hematopoietic stem cells, inducing differentiation thereof into T cells, subjecting the T cells to TCR stimulation, and then introducing thereinto a gene encoding said CAR; and
   administering the CAR-macrophages and the CAR-T cells in combination to a patient having a disease caused by cells expressing said antigen (e.g., a tumor expressing a tumor antigen).
[23] A method of treating a disease (e.g., a tumor) caused by cells expressing a target antigen in a patient, comprising the steps of:
   preparing CAR-macrophages by the following step (1) or (2):
      (1) preparing multipotent blood progenitor cells from a cell population comprising hematopoietic stem cells, introducing a gene encoding a CAR that specifically recognizes a target antigen (e.g., a tumor antigen) into the multipotent blood progenitor cells to thereby prepare multipotent blood progenitor cells expressing said CAR, and then inducing differentiation of the CAR-expressing multipotent blood progenitor cells into macrophages;
      (2) preparing induced leukocyte stem cells or T/NK progenitor cells from a cell population comprising hematopoietic stem cells, inducing differentiation thereof into macrophages, subjecting the macrophages to receptor stimulation, and then introducing a gene encoding said CAR;
   preparing CAR-NK cells by the following step (5) or (6):
      (5) preparing multipotent blood progenitor cells from a cell population comprising hematopoietic stem cells, introducing a gene encoding said CAR into the multipotent blood progenitor cells to thereby prepare multipotent blood progenitor cells expressing said CAR, and then inducing differentiation of the CAR-expressing multipotent blood progenitor cells into NK cells;
      (6) preparing induced leukocyte stem cells or T/NK progenitor cells from a cell population comprising hematopoietic stem cells, inducing differentiation thereof into NK cells, subjecting the NK cells to receptor stimulation, and then introducing a gene encoding said CAR; and
   administering the CAR-macrophages and the CAR-NK cells in combination to a patient having a disease caused by cells expressing said antigen (e.g., a tumor expressing a tumor antigen).
[24] The method according to [23], wherein the step (5) further comprises, after inducing differentiation of the CAR-expressing multipotent blood progenitor cells into NK cells to prepare the CAR-NK cells, subjecting the CAR-NK cells to receptor stimulation.
[25] The method according to any one of [22] to [24], wherein the step (1) further comprises, after inducing differentiation of the CAR-expressing multipotent blood progenitor cells into macrophages to prepare the CAR-macrophages, subjecting the CAR-macrophages to receptor stimulation.

In [12] to [25] above, preferably, the target antigen is a tumor antigen, and the disease caused by cells expressing the antigen is a tumor expressing said tumor antigen.

The present invention further provides the following methods, cells, and the like.

[26] A method of producing human T/NK progenitor cells, comprising a step of culturing a cell population comprising human hematopoietic stem cells in the presence of a Notch ligand in a medium comprising SCF, Flt3-L, and IL-7 at concentrations of 1 ng/mL or more and less than 10 ng/mL each.

[27] The method according to [26], wherein the medium further comprises at least one of TPO and M-CSF.

[28] The method according to any one of [26] to [27], wherein the cell population comprising human hematopoietic stem cells is a cell population obtained after culturing a cell population comprising human hematopoietic stem cells in a medium comprising SCF, TPO, and G-CSF at concentrations of 100 ng/mL or more each in the absence of a Notch ligand.

[29] A method of producing human NK cells, comprising the steps of:
producing human T/NK progenitor cells by the method according to any one of [26] to [28]; and
culturing the produced human T/NK progenitor cells in a medium comprising IL-7, Flt3-L, SCF, and IL-15 in the absence of a Notch ligand to induce differentiation into NK cells.

[30] The method of producing said immune cell according to claim 6 or 7, wherein the T/NK progenitor cell is a human T/NK progenitor cell, and in the step of preparing multipotent blood progenitor cells, human T/NK progenitor cells are produced from a cell population comprising human hematopoietic stem cells by the method according to any one of [26] to [28].

[31] The method of producing said immune cell according to [30], wherein the immune cell is a human NK cell, and in the step of inducing differentiation of the multipotent blood progenitor cells into immune cells, differentiation into human NK cells is induced by culturing the human T/NK progenitor cells in a medium comprising IL-7, Flt3-L, SCF, and IL-15 in the absence of a Notch ligand.

[32] A cultured human T/NK progenitor cell, characterized by being CD122-positive. Here, the cultured human T/NK progenitor cell is preferably a cell that further satisfies 1, 2, 3, 4, 5, or 6 surface antigen expression conditions selected from the group consisting of CD3-negative, CD4-negative, CD8-negative, CD56-negative, CD161-negative, and CD7-positive.

[33] A cultured human NK cell, characterized by being more immature and having a lower degree of exhaustion than human umbilical cord blood NK cells isolated from human umbilical cord blood, due to lower expression levels of maturity indicators CD16 and KIR and exhaustion marker TIGIT compared to those of said human umbilical cord blood NK cells.

The aforementioned method of producing an immune cell therapy agent for a disease treatable by immune cell therapy according to the present invention includes the following aspects.
[i-1] The method of producing an immune cell therapy agent according to any one of [12] to [16], wherein: the multipotent blood progenitor cell is a human T/NK progenitor cell; and in the step of preparing multipotent blood progenitor cells, human T/NK progenitor cells are produced from a cell population comprising human hematopoietic stem cells by the method according to any one of [26] to [28].
[i-2] The method of producing an immune cell therapy agent according to any one of [14] to [16], wherein: the multipotent blood progenitor cell is a human T/NK progenitor cell; the immune cell is a human NK cell; and in the step of preparing multipotent blood progenitor cells, human T/NK progenitor cells are produced from a cell population comprising human hematopoietic stem cells by the method according to any one of [26] to [28]; and in the step of inducing differentiation of the multipotent blood progenitor cells into immune cells, differentiation into human NK cells is induced by culturing the human T/NK progenitor cells in a medium comprising IL-7, Flt3-L, SCF, and IL-15 in the absence of a Notch ligand.

The aforementioned method of treating a disease treatable by immune cell therapy in a patient according to the present invention includes the following aspects.
[ii-1] The method of treating a disease treatable by immune cell therapy in a patient according to any one of [17] to [21], wherein: the multipotent blood progenitor cell is a human T/NK progenitor cell; and in the step of preparing multipotent blood progenitor cells, human T/NK progenitor cells are produced from a cell population comprising human hematopoietic stem cells by the method according to any one of [26] to [28].
[ii-2] The method of treating a disease in a patient according to any one of [19] to [21], wherein: the multipotent blood progenitor cell is a human T/NK progenitor cell; the immune cell is a human NK cell; and in the step of preparing multipotent blood progenitor cells, human T/NK progenitor cells are produced from a cell population comprising human hematopoietic stem cells by the method according to any one of [26] to [28]; and in the step of inducing differentiation of the multipotent blood progenitor cells into immune cells, differentiation into human NK cells is induced by culturing the human T/NK progenitor cells in a medium comprising IL-7, Flt-3L, SCF, and IL-15 in the absence of a Notch ligand.

The aforementioned method of treating a disease caused by cells expressing a target antigen in a patient according to the present invention includes the following aspects.
[iii-1] The method of treating a disease caused by cells expressing a target antigen in a patient according to any one of [22] to [25], wherein: the multipotent blood progenitor cell is a human T/NK progenitor cell; and preparation of the multipotent blood progenitor cell from a cell population comprising hematopoietic stem cells is performed by the method according to any one of [26] to [28].
[iii-2] The method of treating a disease caused by cells expressing a target antigen in a patient according to [23] or [24], wherein: the multipotent blood progenitor cell is a human T/NK progenitor cell; the NK cell is a human NK cell; preparation of the multipotent blood progenitor cell from a cell population comprising hematopoietic stem cells is performed by the method according to any one of [26] to [28]; and induction of differentiation into the NK cell is performed by culturing the human T/NK progenitor cell in a medium comprising IL-7, Flt3-L, SCF, and IL-15 in the absence of a Notch ligand.

### EFFECT OF THE INVENTION

According to the present invention, TCR/CAR/CAAR-expressing immune cells such as TCR-T, CAR-T, CAAR-T, CAR-NK, and CAAR-NK cells can be stably produced. By preparing multipotent blood progenitor cells by the expansion method of induced leukocyte stem cells or T/NK progenitor cells developed by the group of the present inventors, TCR/CAR/CAAR-expressing immune cells can be stably produced in large quantities. TCR/CAR-expressing immune cells prepared using an antigen-specific receptor that specifically recognizes a tumor antigen or an antigen specific to a pathogen such as a bacterium or a virus exhibit cytotoxic activity against tumor cells or pathogen-infected cells in a patient's body, and thus can be used for the treatment of tumors or infectious diseases as an immune cell therapy agent for tumors or infectious diseases. In addition, CAAR-expressing immune cells prepared using a CAAR designed based on an arbitrary self-antigen exhibit cytotoxic activity against pathogenic B cells that recognize the self-antigen in a patient's body, and thus can be used for the treatment of autoimmune diseases as an immune cell therapy agent for autoimmune diseases caused by the self-antigen or pathogenic B cells. According to the present invention, it is also possible to stably produce such an immune cell therapy agent in large quantities, which greatly contributes to the treatment of diseases such as tumors, infectious diseases, and autoimmune diseases.

### BRIEF DESCRIPTION OF THE DRAWINGS

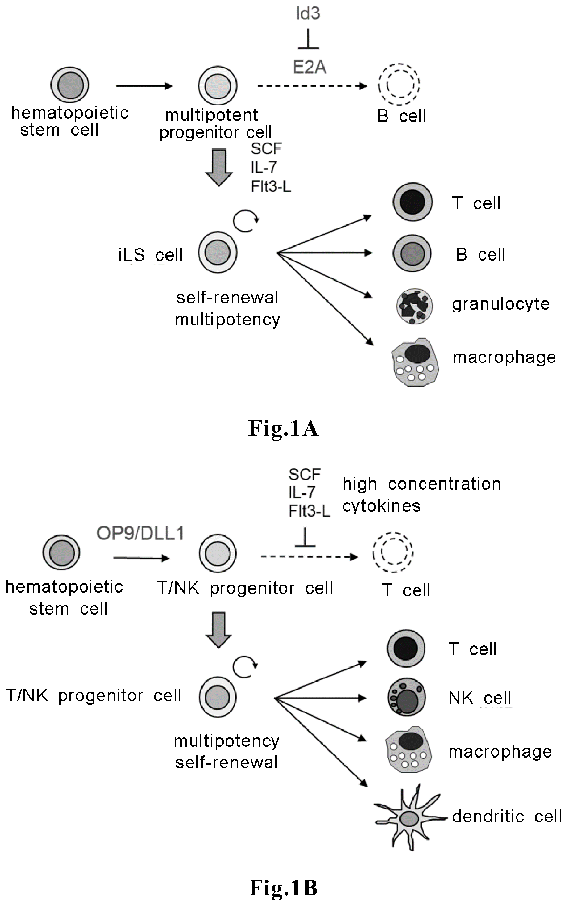

[FIG. 1A] A diagram illustrating induced leukocyte stem (iLS) cells. iLS cells are multipotent blood progenitor cells having self-renewal ability and have the ability to differentiate into leukocytes such as T cells and B cells.
[FIG. 1B] A diagram illustrating T/NK progenitor cells (pT/NK cells). Hematopoietic stem/progenitor cells are cultured in the presence of high-concentration cytokines to expand CD34-positive hematopoietic stem cells. Then, the cells are cultured using OP9/delta-like-1 (DLL1) cells or the like under Notch signal stimulation, to thereby generate pT/NK having self-renewal ability while maintaining multipotency (ability to differentiate into T cells, NK cells, and myeloid cells).

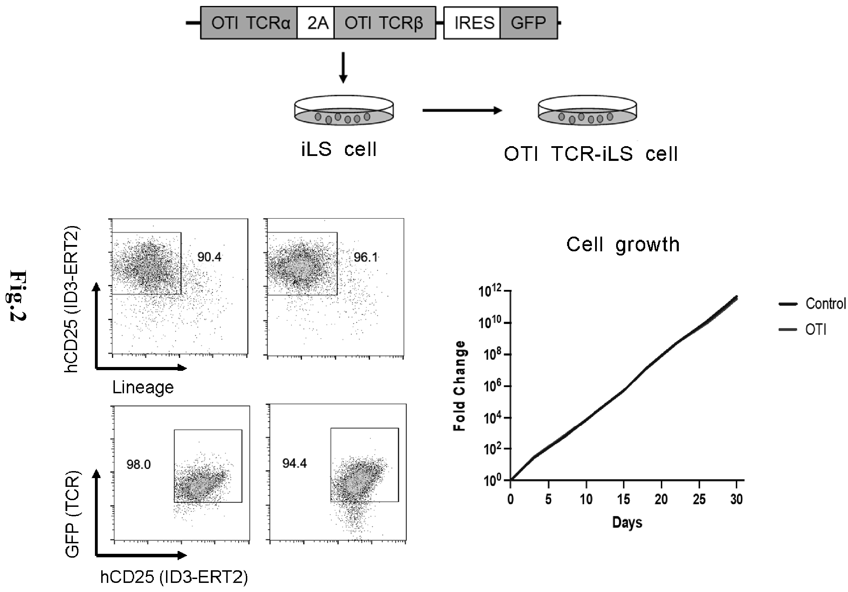

[FIG. 2] (Top) A schematic diagram of the method for producing iLS cells expressing OTI TCR. (Bottom left) FCM analysis of iLS cells transduced with the OTI TCR gene. OTI TCR-expressing iLS (OTI TCR-iLS) cells that are Lineage marker (-), human CD25 (+), and GFP (+) were detected at a high frequency. (Bottom right) A graph showing the proliferation, expressed as a fold change, of OTI TCR-iLS cells and control cells (iLS cells spin-infected with an empty retrovirus not containing the OTI TCR gene) cultured under iLS cell culture conditions.

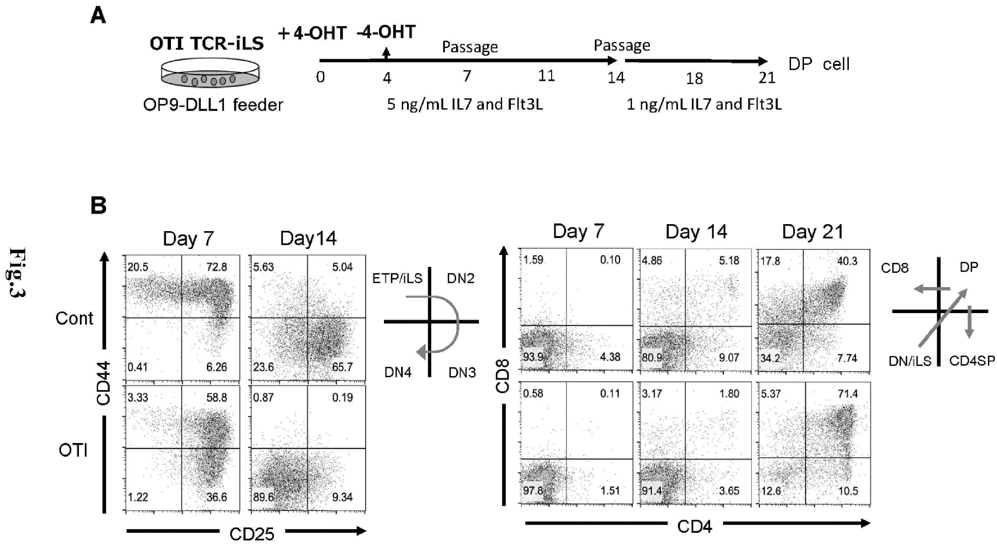

[FIG. 3] (A) An outline of the process for inducing differentiation of T cells (CD4/CD8 double-positive (DP) cells) from OTI TCR-iLS cells. (B) FCM analysis performed on cells collected on days 7, 14, and 21 after the start of induction. DP cells appear on day 14 of culture, and DP cells are generated at a ratio of 70% or more on day 21 of culture.

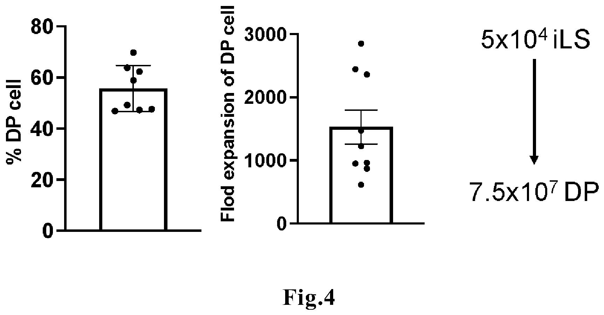

[FIG. 4] The ratio and expansion rate of DP cells generated from OTI TCR-iLS cells are shown. The ratio of DP cells is 50-60%, and iLS cells generate approximately 1500-fold more DP cells.

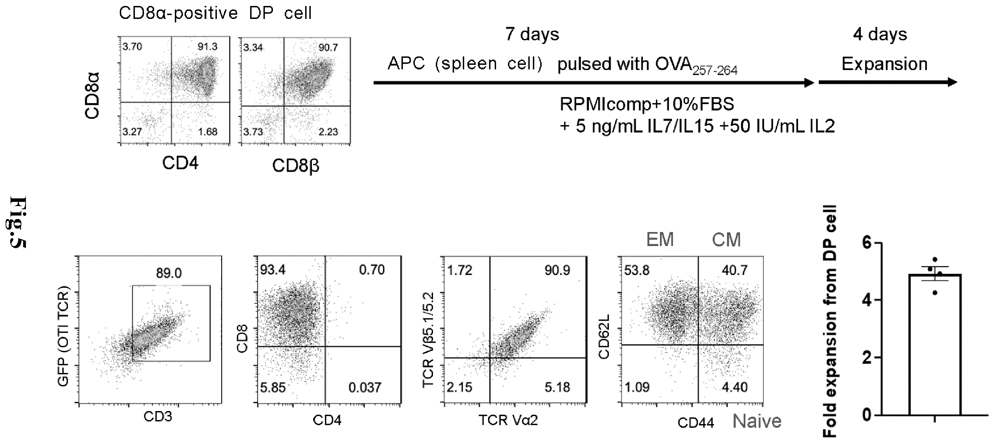

[FIG. 5] An outline of the process for inducing DP cells generated from OTI TCR-iLS cells into CD8-positive T cells. CD8-positive T cells are induced at a ratio of 90% or more by co-culturing DP cells with antigen-presenting cells (APCs) pulsed with OVA₂₅₇₋₂₆₄ for 7 days. These CD8-positive T cells express OTI TCR, and most of them are effector memory (EM) T cells and central memory (CM) T cells. During this process, the cells expand by about 5 times.

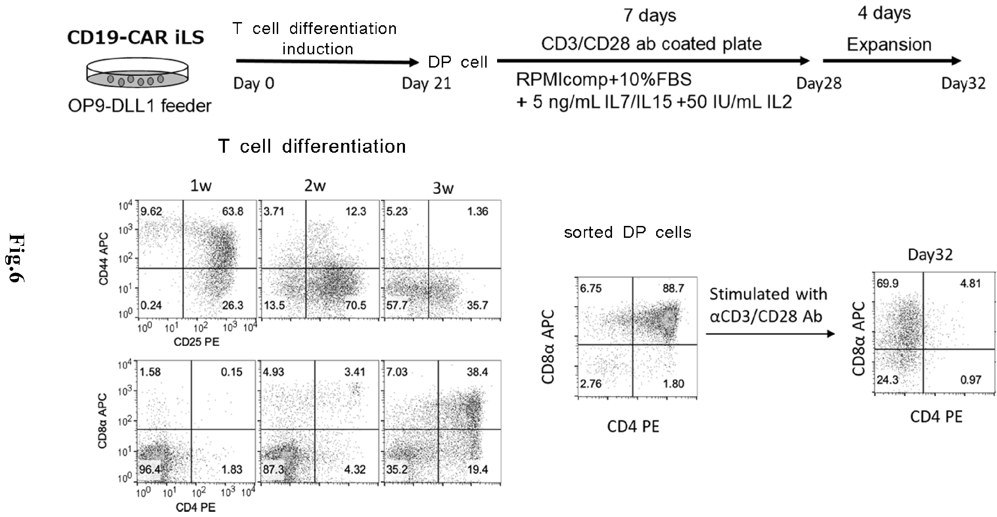

[FIG. 6] An outline of the process for inducing CD8-positive T cells from anti-CD19 CAR-expressing iLS cells (anti-CD19 CAR-iLS cells). Differentiation into DP cells is induced by co-culturing anti-CD19 CAR-iLS cells and OP9/DLL1 cells. DP cells are generated on day 21 of culture. Next, the DP cells are sorted and stimulated with an anti-CD3/CD28 antibody to be induced into CD8T positive cells. CD8-positive T cells are generated after 4 days of culture.

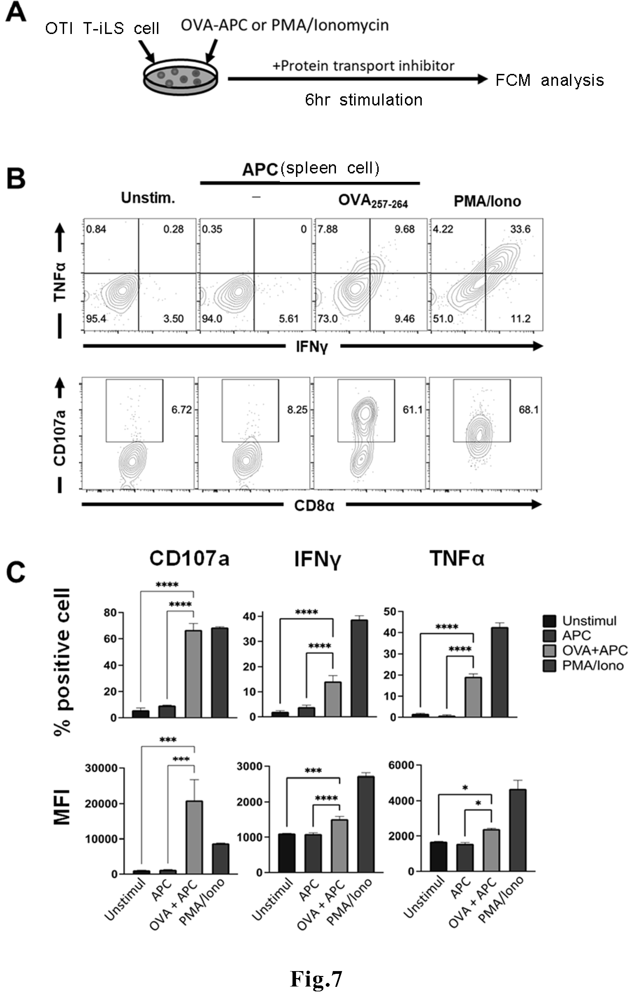

[FIG. 7] (A) A process outline of the antigen-specific cytokine release evaluation test of CD8-positive T cells derived from OTI TCR-iLS cells (OTI T-iLS cells). (B, C) Results of examining cytokine release by intracellular staining of OTI TCR-iLS cell-derived CD8-positive T cells stimulated with OVA antigen-presenting cells or with PMA + ionomycin. B shows a two-parameter histogram of FCM analysis, and C shows a graph thereof. Unstim. and Unstimul indicate cells without stimulation, APC indicates cells co-cultured with mouse spleen cells not presenting OVA, OVA + APC indicates cells stimulated with OVA antigen-presenting cells, and PMA/Iono indicates cells stimulated with PMA + ionomycin. MFI: median fluorescence intensity.

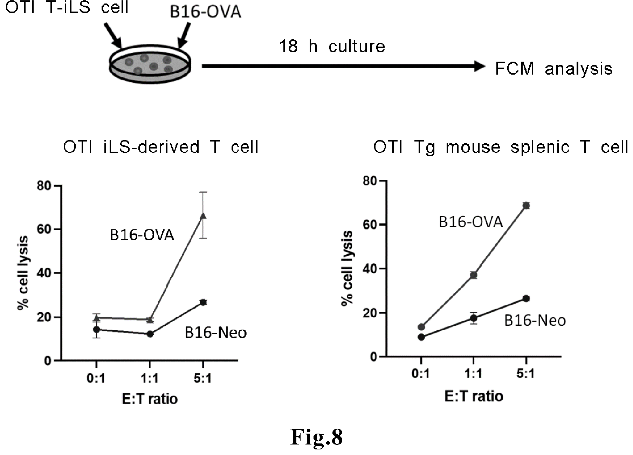

[FIG. 8] (Top) A process outline of the cytotoxic activity evaluation test of OTI T-iLS cells. (Bottom) Results of examining the cytotoxic activity of each effector cell, using B16 melanoma cell lines introduced with the OVA antigen (B16-OVA cells) and the Neo gene (B16-Neo cells) as target cells, and OTI T-iLS cell- and OTI Tg mouse spleen-derived T cells as effector cells.

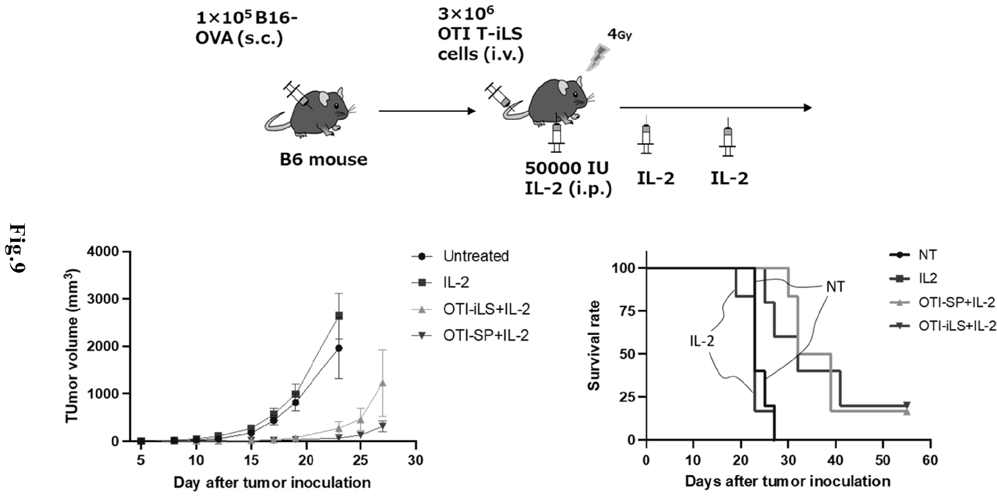

[FIG. 9] (Top) A process outline of the anti-tumor activity evaluation test of OTI T-iLS cells using B16-OVA cell-bearing mice. (Bottom left) Tumor volume of tumor-bearing mice in each treatment group. (Bottom right) Survival rate of tumor-bearing mice in each treatment group. Untreated and NT indicate no treatment, IL-2 indicates IL-2 treatment only, OTI-iLS + IL-2 indicates treatment with OTI T-iLS cells and IL-2, and OTI-SP + IL-2 indicates treatment with OTI Tg mouse spleen-derived T cells and IL-2.

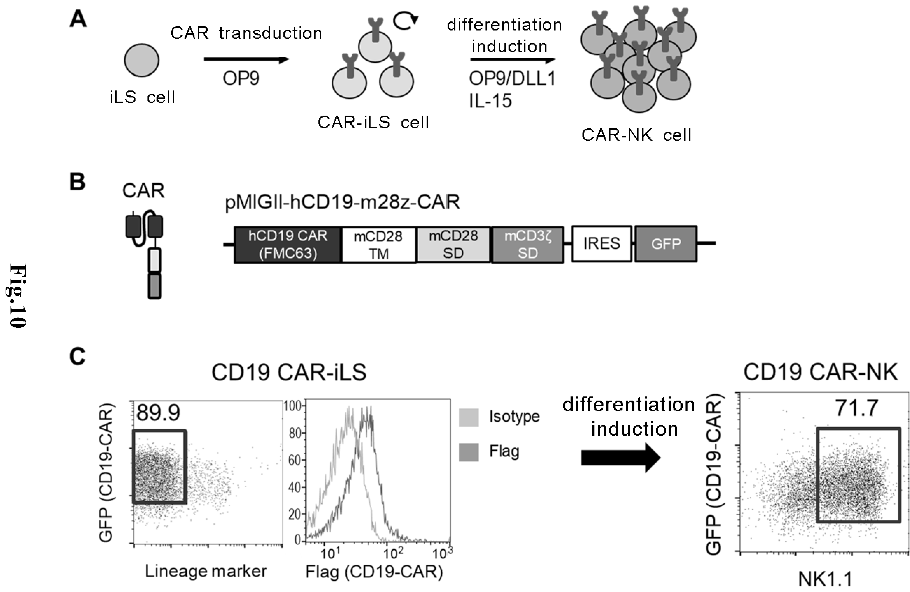

[FIG. 10] (A) An outline of the process for inducing CAR-NK cells from iLS cells. (B) A schematic diagram of the anti-CD19 CAR sequence for mice of a retroviral vector used in the Examples. (C) Results of FCM analysis of anti-CD19 CAR gene-transduced pT/NK cells (anti-CD19 CAR-pT/NK cells) and anti-CD19 CAR-transduced NK cells after induction of differentiation (anti-CD19 CAR-NK cells). It shows that anti-CD19 CAR-NK cells are generated at a high ratio (70% or more).

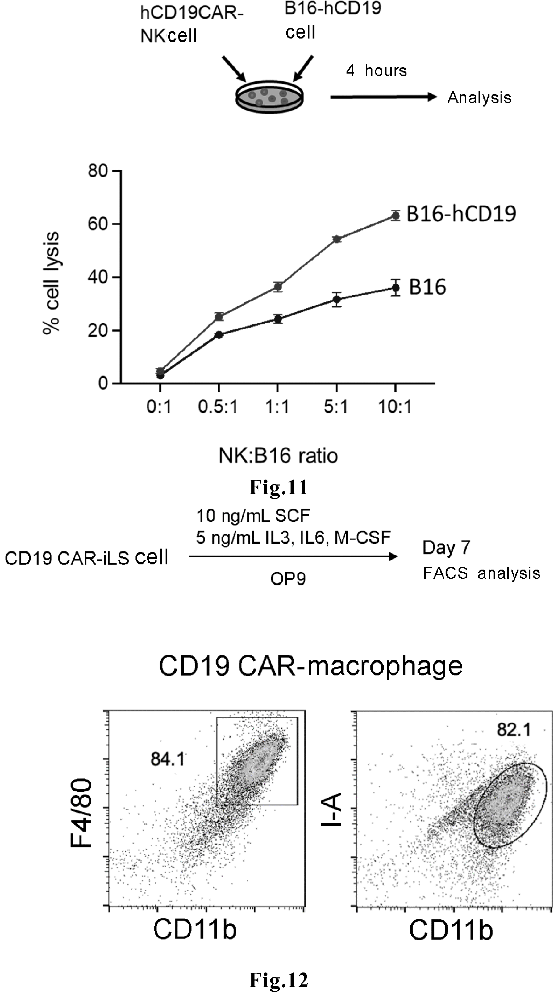

[FIG. 11] Cytotoxic activity of anti-CD 19 CAR-NK cells against B16 melanoma cells introduced with human CD19 (B16-hCD19 cells) and B16 melanoma cells not introduced with human CD19 (B16 cells).
[FIG. 12] Induction of differentiation into macrophages from anti-CD19 CAR-iLS cells. FCM analysis was performed one week after the start of induction (Day 7), and the generation of CD11b-positive F4/80-positive MHC class II (I-A)-positive macrophages was confirmed.

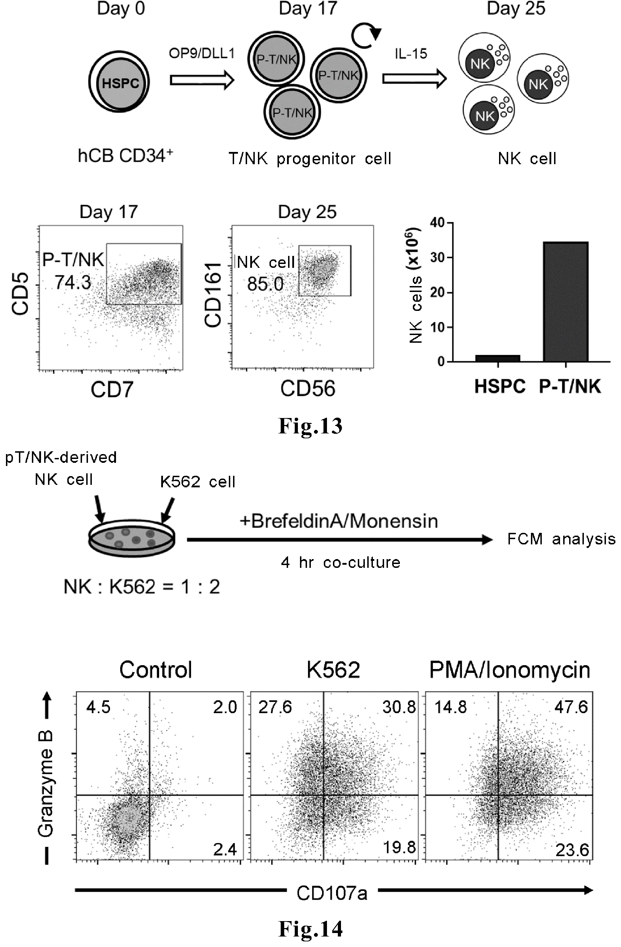

[FIG. 13] (Top) An outline of the process for producing pT/NK cells from human umbilical cord blood CD34-positive cells (hCB-CD34 positive cells) and inducing differentiation into NK cells. (Bottom) The expression of each surface antigen marker molecule was examined by FCM analysis on day 17 (left) and day 25 (middle) after the start of culture of hCB-CD34 positive cells. pT/NK cells isolated by a cell sorter from the cell population on day 17 of culture and human umbilical cord blood hematopoietic stem/progenitor cells (hCB-HSPCs) obtained by expanding hCB-CD34 positive cells in Stemline II medium were cultured under NK cell differentiation induction conditions, and the numbers of generated NK cells were compared (right).
[FIG. 14] NK cells derived from pT/NK cells were stimulated by co-culture with a K562 leukemia cell line (K562 cells), which are target cells, or by treatment with PMA + ionomycin, and cytokine release ability was analyzed by FCM.

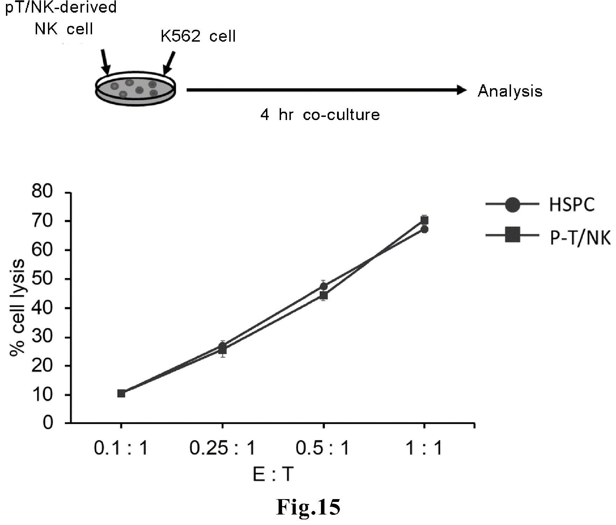

[FIG. 15] Cytotoxic activity of pT/NK cell-derived NK cells and hCB-HSPC-derived NK cells against K562 cells.

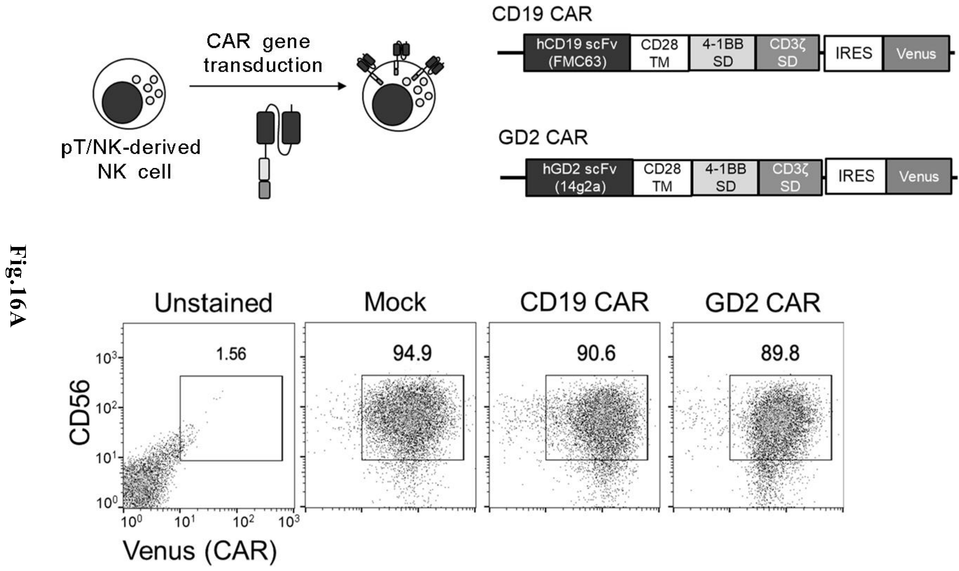

[FIG. 16A] A schematic diagram of the anti-CD19 CAR sequence and anti-GD2 CAR sequence for humans of a lentiviral vector used for the production of pT/NK cell-derived anti-CD19 CAR-NK cells and anti-GD2 CAR-NK cells (top). It was confirmed by FCM analysis for marker protein Venus that the expression of the transduced gene was maintained (bottom). Mock indicates cells transduced with Venus alone by lentivirus.

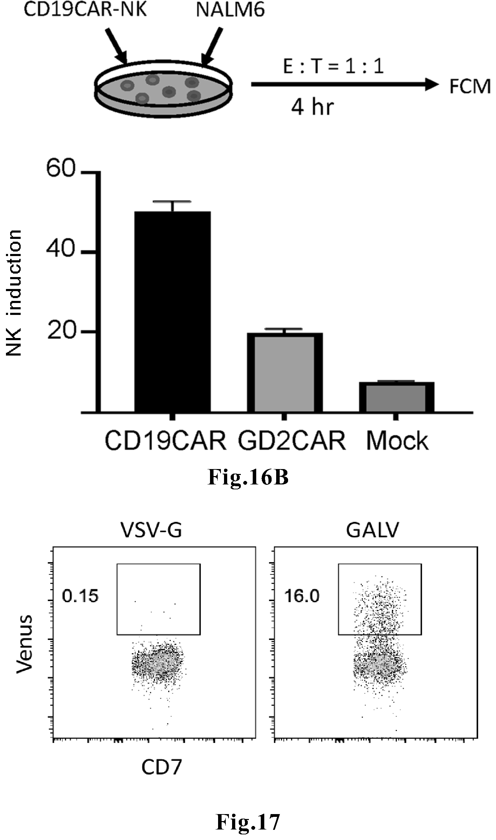

[FIG. 16B] Antigen-specific cytotoxic activity of pT/NK cell-derived anti-CD19 CAR-NK cells against a NALM6 leukemia cell line (NALM6 cells).
[FIG. 17] The gene transduction efficiency into pT/NK cells of lentiviruses produced using a VSV-G or GALV envelope was compared.

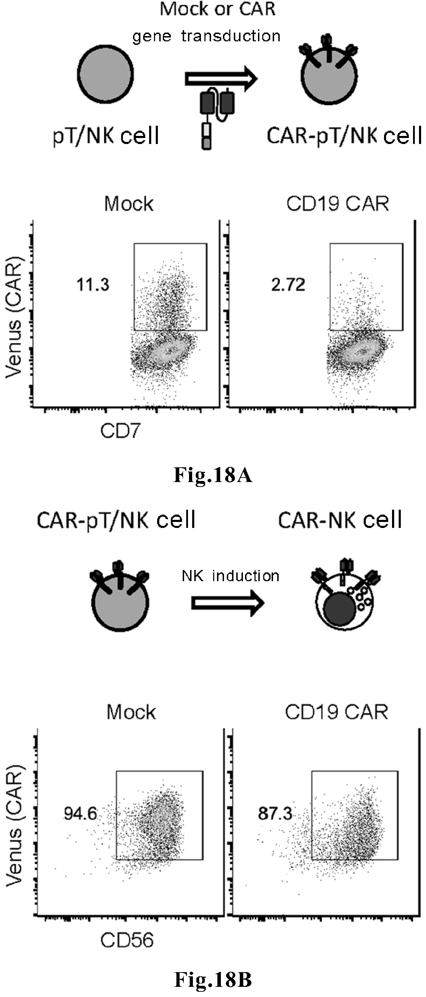

[FIG. 18A] Gene transduction into pT/NK cells was performed using a CAR gene-expressing lentivirus produced with a GALV envelope. It was confirmed by FCM analysis for marker protein Venus that the expression of the transduced gene was maintained. Mock indicates cells transduced with Venus alone by lentivirus.
[FIG. 18B] CAR-pT/NK cells obtained by gene transduction with a CAR gene-expressing lentivirus produced using a GALV envelope were induced to differentiate into NK cells, to thereby obtain pT/NK cell-derived NK cells stably expressing a CAR gene. Mock indicates cells transduced with Venus alone by lentivirus.

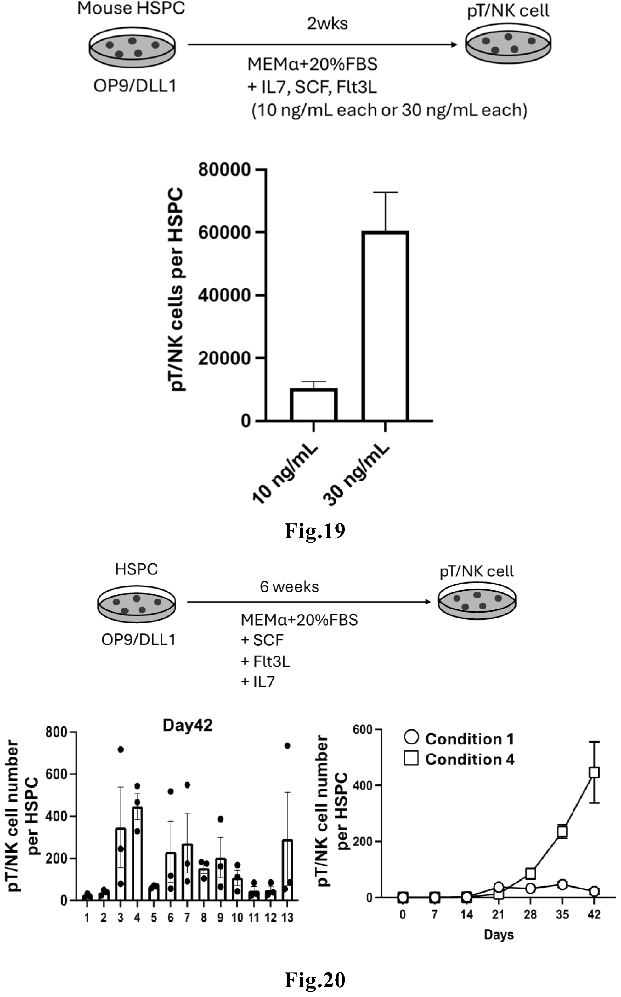

[FIG. 19] Results of measuring the number of pT/NK cells after culturing mouse bone marrow-derived hematopoietic stem/progenitor cells (Mouse HSPC, Lineage-Scal-ckit+: LSK) on OP9/DLL1 feeder cells for 2 weeks with SCF, IL-7, and Flt3-L added to the medium at a concentration of 10 ng/ml or 30 ng/ml each.
[FIG. 20] Results of investigating optimal cytokine concentrations for generating and expanding pT/NK cells from hCB-CD34 positive cells. The top row is an outline of the culture process. The bottom left shows the number of pT/NK cells measured after 6 weeks of culture at the cytokine concentrations of conditions 1 to 13 shown in Table 1. The bottom right is a graph showing the number of pT/NK cells obtained by culture under condition 1 and condition 4 over time.

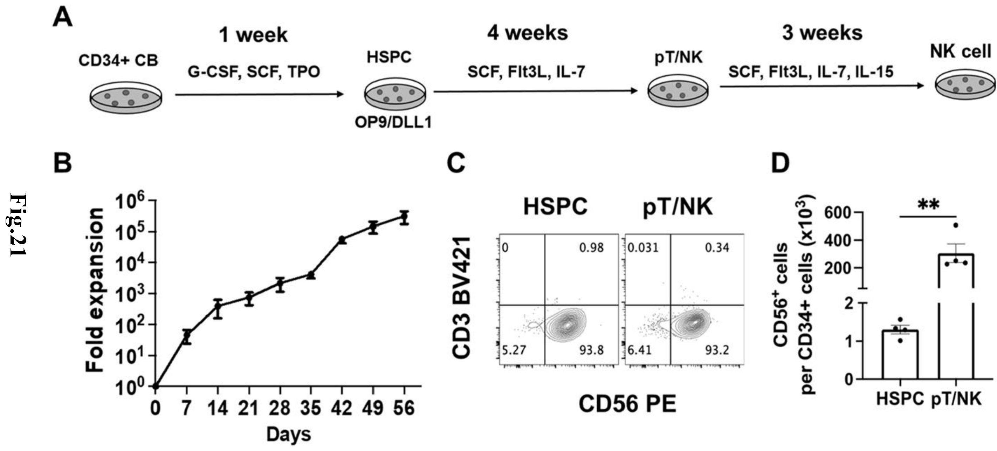

[FIG. 21] (A) An outline of the culture protocol in Example B-2. (B) Proliferation rate of cells from hCB-CD34 positive cells. (C) Results of FCM analysis after NK cell generation. (D) Number of produced NK cells when NK cells were directly induced from hCB-HSPCs and when the pT/NK cell expansion method was used.

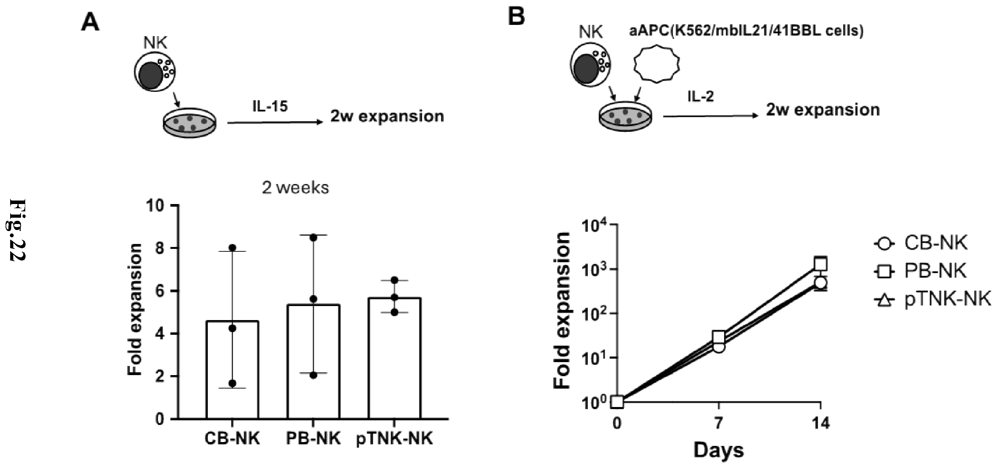

[FIG. 22] (A) Proliferation rate of NK cells collected from human umbilical cord blood (CB-NK cells), NK cells collected from human peripheral blood (PB-NK cells), and human pT/NK cell-derived NK cells after culturing for 2 weeks in MEMα medium supplemented with 10 ng/ml rhIL-15 in Example B-3. (B) Expansion rate when NK cells were expanded in MEMα medium containing 200 IU/ml rhIL-2 by mixing mitomycin-treated aAPCs and each type of NK cells at an E:T ratio of 1:2.

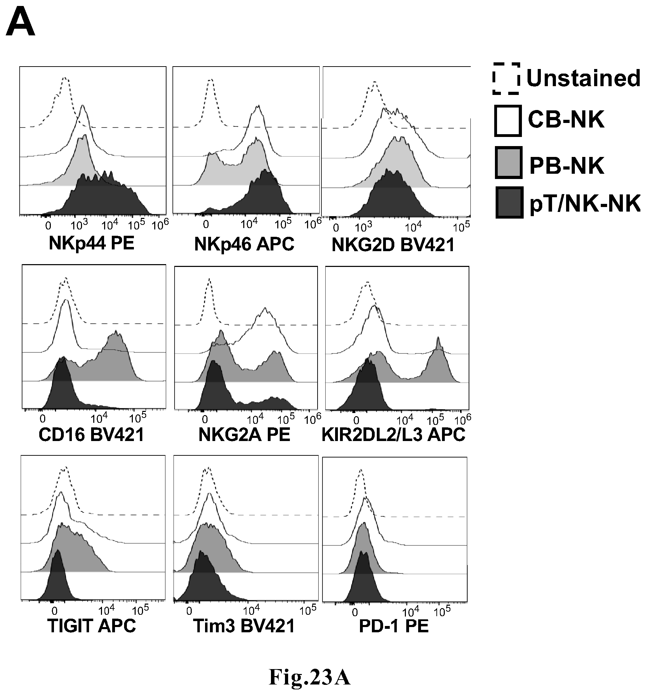

[FIG. 23A] Results of evaluating the expression level of NK cell-related receptors in pT/NK cell-derived NK cells by FCM analysis in Example B-4 (one-parameter histogram).

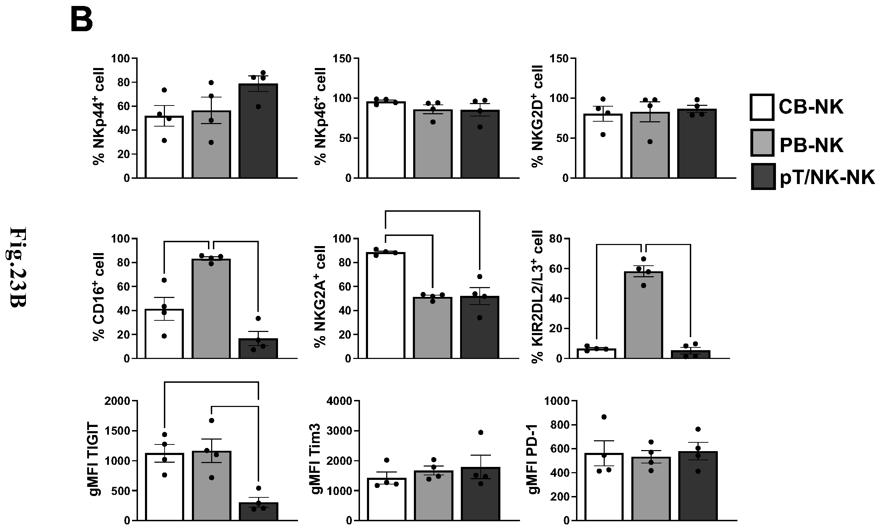

[FIG. 23B] Results of evaluating the expression level of NK cell-related receptors in pT/NK cell-derived NK cells by FCM analysis in Example B-4 (graphical representation of one-parameter histograms).

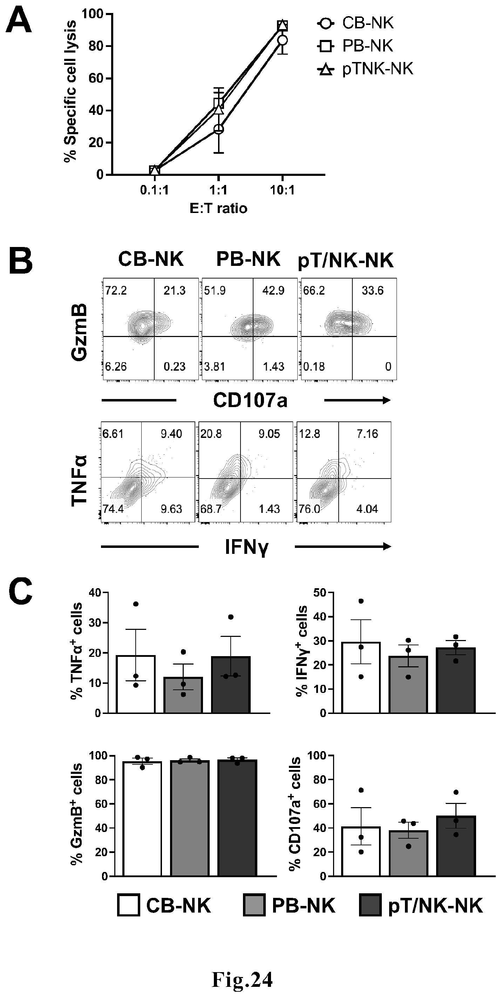

[FIG. 24] Results of comparing the cytotoxic activity of pT/NK cell-derived NK cells against K562 cells with PB-NK cells or CB-NK cells in Example B-5. (A) Results of FCM analysis of the dead cell ratio of K562 cells after mixing NK cells and K562 cells at various E:T ratios and co-culturing for 4 hours. (B, C) Evaluation of the expression level of effector molecules produced by activated NK cells. B is a two-parameter histogram, and C is a graphical representation thereof.

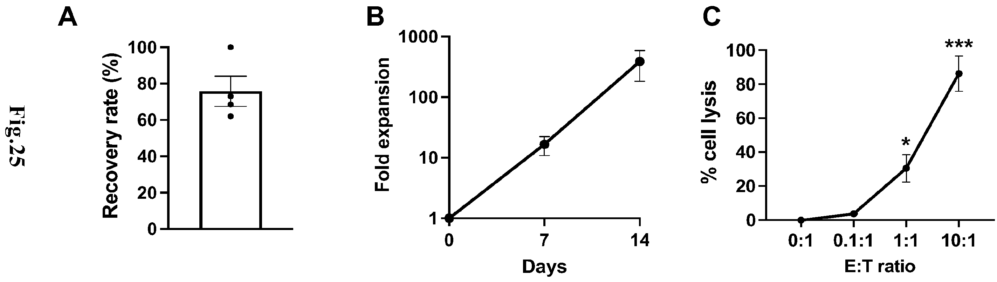

[FIG. 25] Results of evaluating the effect of cryopreservation of human pT/NK cell-derived NK cells in Example B-6. (A) Recovery rate of pT/NK cell-derived NK cells after freeze-thawing (number of cells after freezing / number of cells before freezing × 100). (B) Proliferation ability of human pT/NK cell-derived NK cells after freeze-thawing. (C) Cytotoxic activity of freeze-thawed pT/NK cell-derived NK cells against K562 cells.

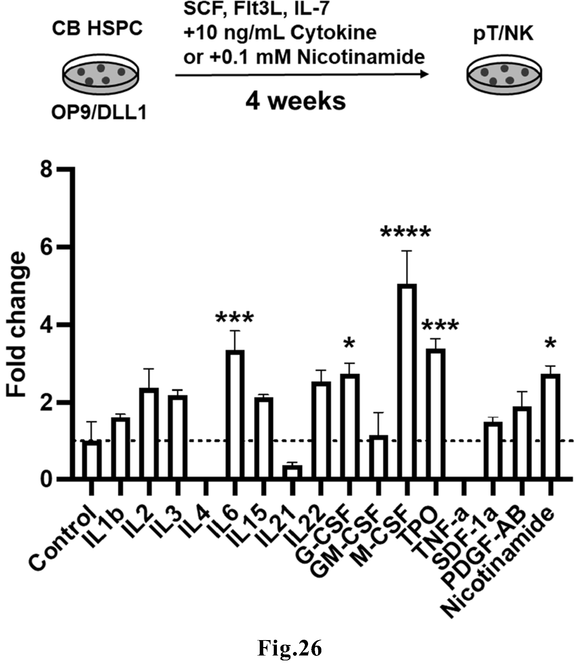

[FIG. 26] Results of a search for factors that promote differentiation and proliferation of hCB-HSPCs into pT/NK cells in Example B-7. The expansion rate of pT/NK cells was calculated by adding 10 ng/ml of each cytokine or 0.1 mM nicotinamide to a system in which hCB-HSPCs were cultured on OP9/DLL1 feeder cells in the presence of 5 ng/ml each of rhIL-7, rhFlt3-L, and rhSCF.

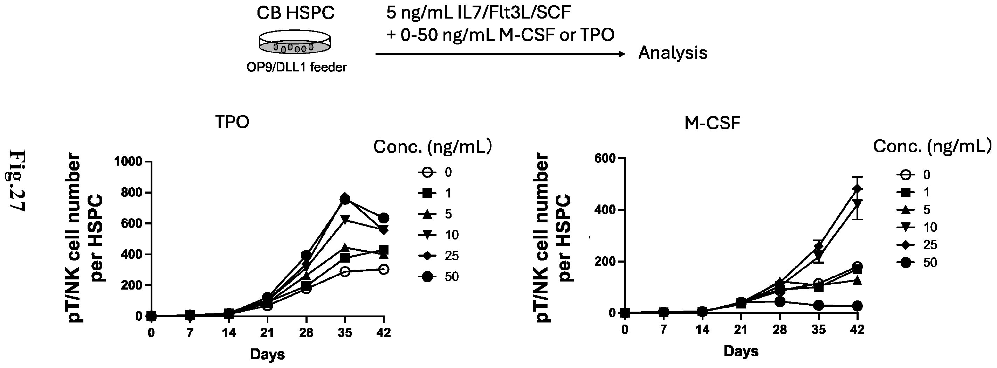

[FIG. 27] Results of investigating the concentration at which TPO or M-CSF promotes the generation of human pT/NK cells in Example B-8. TPO or M-CSF was added at a concentration of 0 to 50 ng/ml to a system in which hCB-HSPCs were cultured on OP9/DLL1 feeder cells in the presence of 5 ng/ml each of rhIL-7, rhFlt3-L, and rhSCF, and the number of pT/NK cells per HSPC was examined.

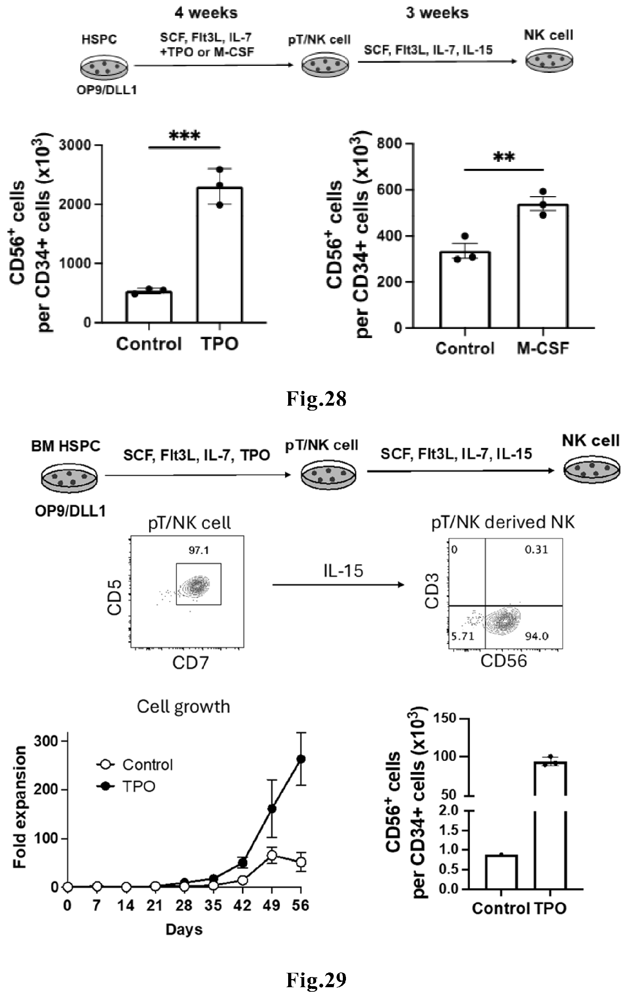

[FIG. 28] Results of evaluating the differentiation ability of human pT/NK cells expanded by adding TPO or M-CSF into NK cells in Example B-9.
[FIG. 29] Results of investigating whether TPO is also effective for inducing pT/NK cells from human bone marrow-derived HSPCs (hBM-HSPCs) in Example B-10. The top row is an outline of the culture protocol. The middle row shows FCM analysis of pT/NK cells generated and expanded from hBM-HSPCs and NK cells induced from these pT/NK cells. The bottom row shows the results of comparing the number of NK cells induced from pT/NK cells expanded for 5 weeks using this culture system with or without TPO.

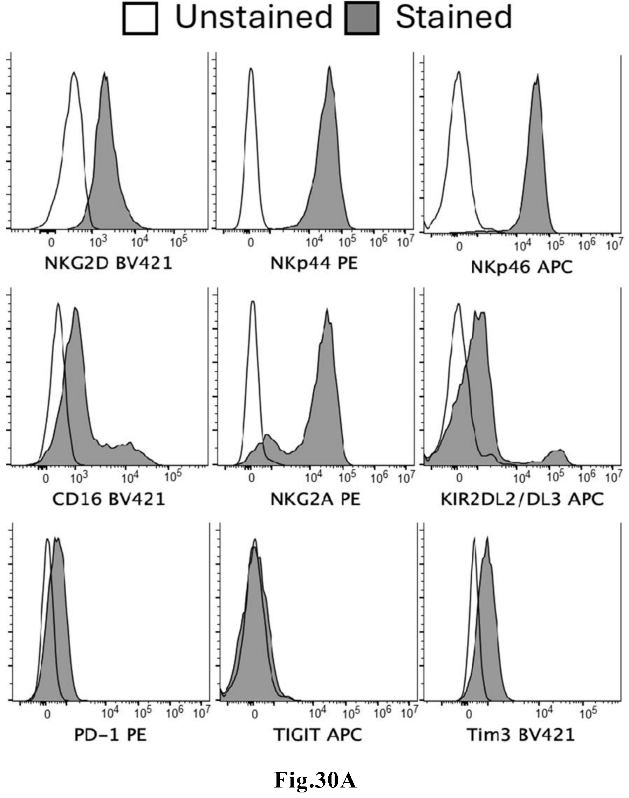

[FIG. 30A] Results of evaluating the expression level of NK cell-related receptors in pT/NK-derived NK cells induced from hBM-HSPCs by FCM analysis in Example B-11 (one-parameter histograms).

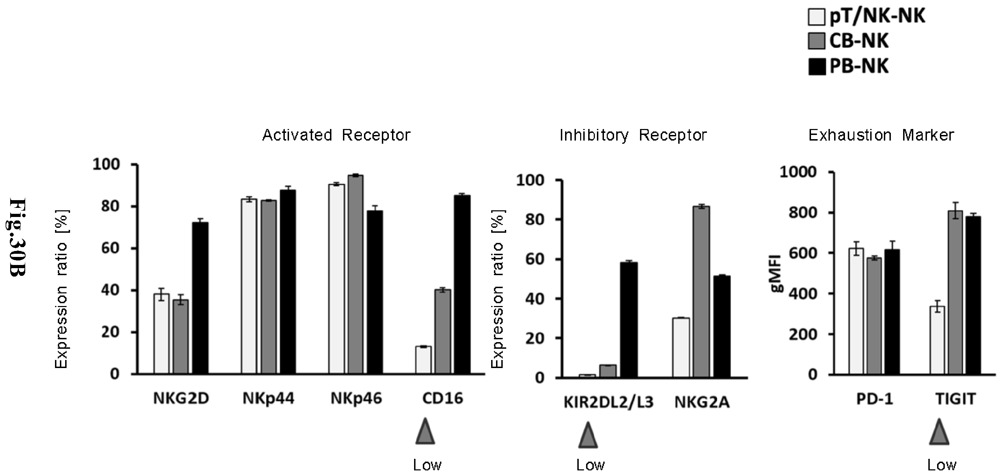

[FIG. 30B] Results of comparing the expression of activated receptors, inhibitory receptors, and exhaustion markers in human T/NK progenitor cell-derived NK cells (pT/NK-NK cells) induced from hCB-HSPCs, human CB-NK cells, and human PB-NK cells.
[FIG. 30C] RNA-seq data comparing gene expression in human pT/NK-NK cells induced from hCB-HSPCs, human CB-NK cells, and human PB-NK cells.

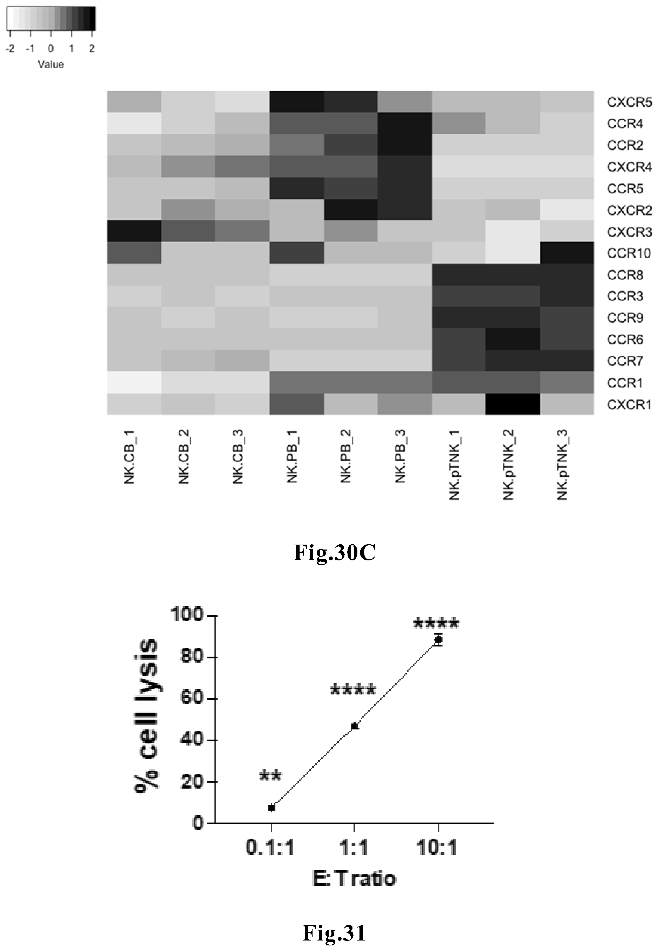

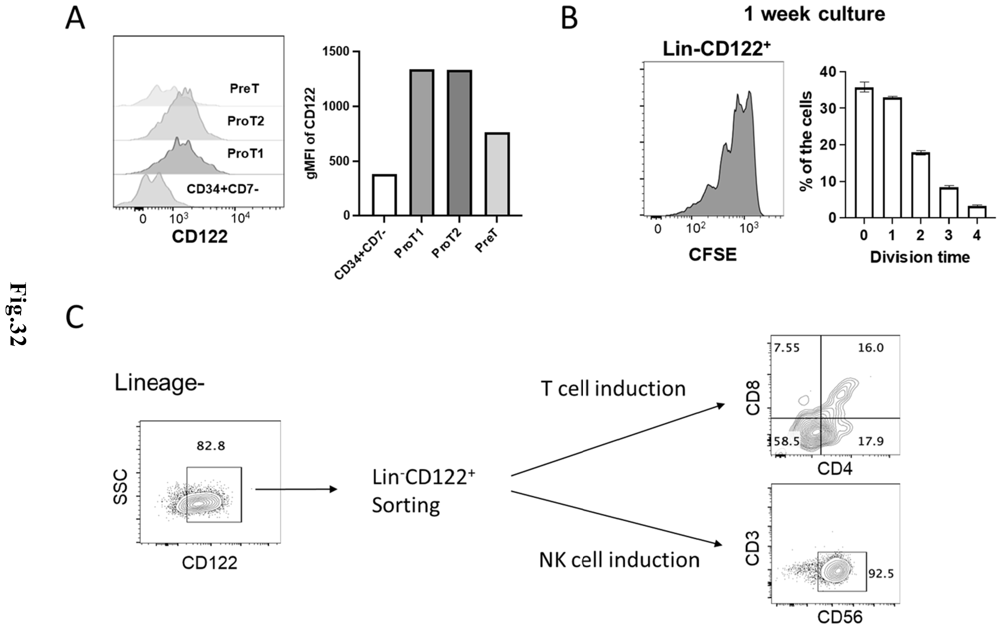

[FIG. 31] Results of evaluating the cytotoxic activity against K562 cells of pT/NK cell-derived NK cells induced from hBM-HSPCs in Example B-12. After mixing and culturing NK cells and K562 cells at various E:T ratios, the dead cell ratio of K562 cells was analyzed by FCM. [FIG. 32] Identification of the specific marker for pT/NK cells (Example B-13). (A) Expression of CD122 (IL-2 receptor β chain) in pT/NK cells was evaluated by FCM analysis. (B) CFSE assay performed to evaluate the proliferation ability of CD122-positive cells. (C) Evaluation of the differentiation ability of CD122-positive cells into T cells and NK cells.

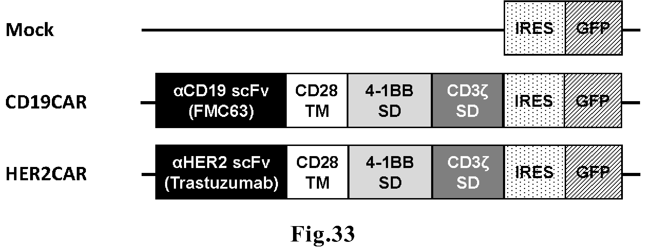

[FIG. 33] A schematic diagram of CAR sequences of retroviral vectors used for the production of human pT/NK cell-derived anti-CD19 CAR-NK cells and anti-HER2 CAR-NK cells constructed in Example C-1.

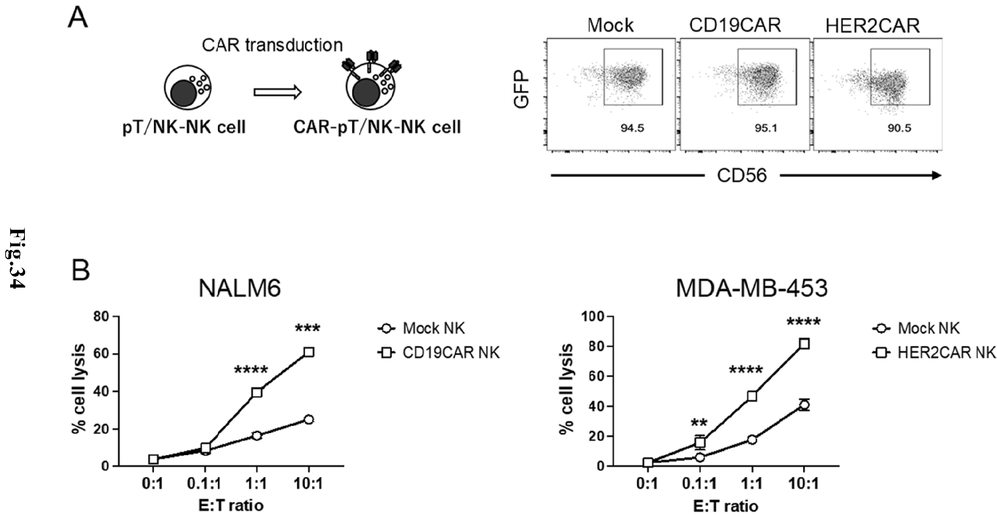

[FIG. 34] Generation of anti-CD 19 CAR-NK cells and anti-HER2 CAR-NK cells from human pT/NK cell-derived NK cells (Example C-2). (A) Human pT/NK cell-derived NK cells were transduced with each CAR gene using the vectors shown in FIG. 33, and GFP-positive CAR-NK cells were isolated by a cell sorter, followed by co-culturing with aAPCs to further expand the cells. After culture, the expression of GFP was analyzed by FCM, and stable expression of the transduced genes was confirmed. (B) Antigen-specific cytotoxic activity of anti-CD19 CAR-NK cells or anti-HER2 CAR-NK cells. After mixing with each target cell at various E:T ratios and co-culturing, the dead cell ratio of the target cells was measured by FCM analysis.

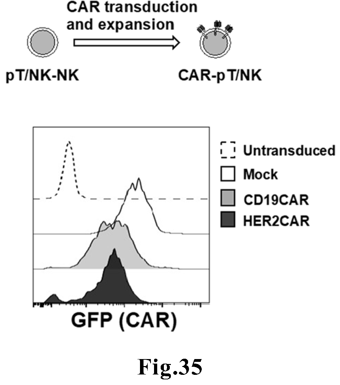

[FIG. 35] Transduction of CAR genes into human pT/NK cells (Example C-3). Human pT/NK cells were transduced with the anti-CD19 CAR gene or anti-HER2 CAR gene using the vectors shown in FIG. 33, and pT/NK cells expressing each CAR gene were isolated using GFP expression as an indicator and expanded. It was confirmed that the transduced genes were stably expressed in the expanded pT/NK cells.

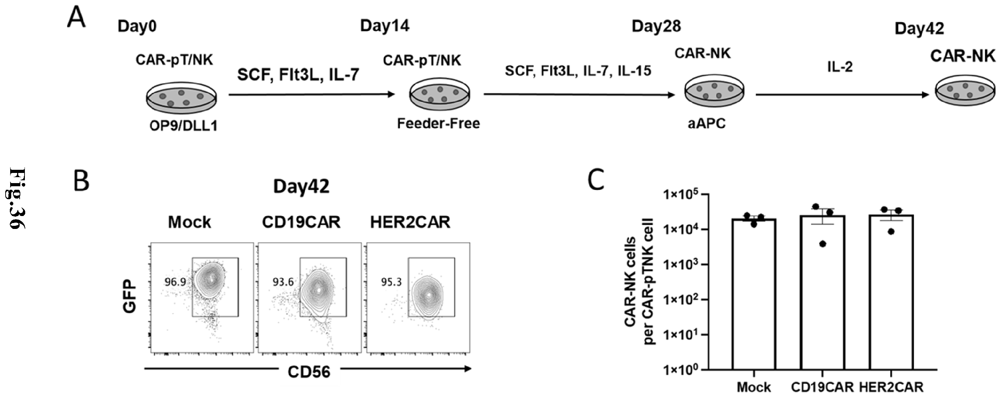

[FIG. 36] Induction of differentiation from human pT/NK cells transduced with the CAR gene into CAR-NK cells (Example C-4). (A) A scheme for producing CAR-NK cells from pT/NK cells transduced with the CAR gene. (B) The obtained CAR-NK cells were expanded by culturing in the presence of aAPCs and IL-2. CAR-pT/NK cell-derived CAR-NK cells after expansion were analyzed by FCM, and the production of CAR-NK cells stably expressing the transduced gene was confirmed. (C) About 1 x 10⁴ CAR-NK cells were produced per human pT/NK cell transduced with the CAR gene.

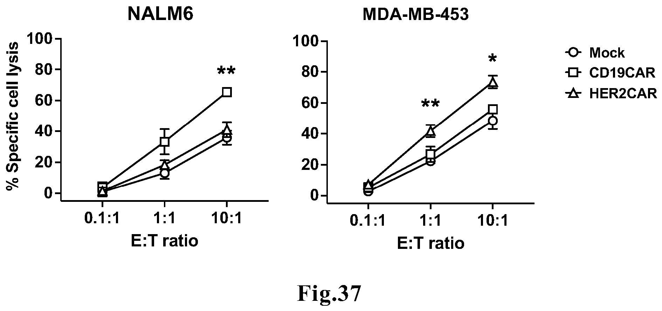

[FIG. 37] Evaluation of cytotoxic activity against target cells of CAR-NK cells derived from pT/NK cells transduced with the CAR gene (Example C-5).

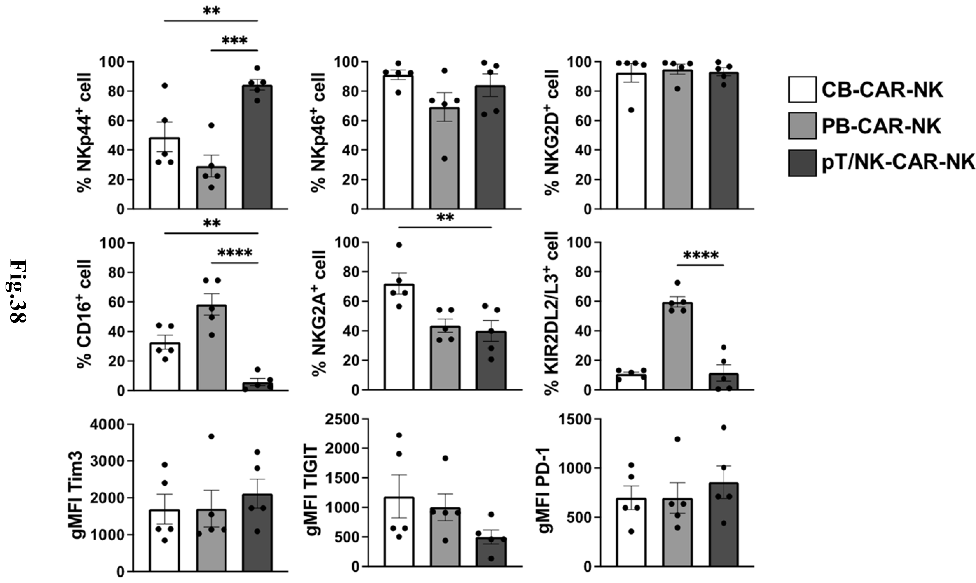

[FIG. 38] FCM analysis of representative NK cell-related receptors was performed for pT/NK cell-derived anti-HER2 CAR-NK cells (pT/NK-CAR-NK), CB-NK-derived anti-HER2 CAR-NK cells (CB-CAR-NK), and PB-NK-derived anti-HER2 CAR-NK cells (PB-CAR-NK) (Example C-6).

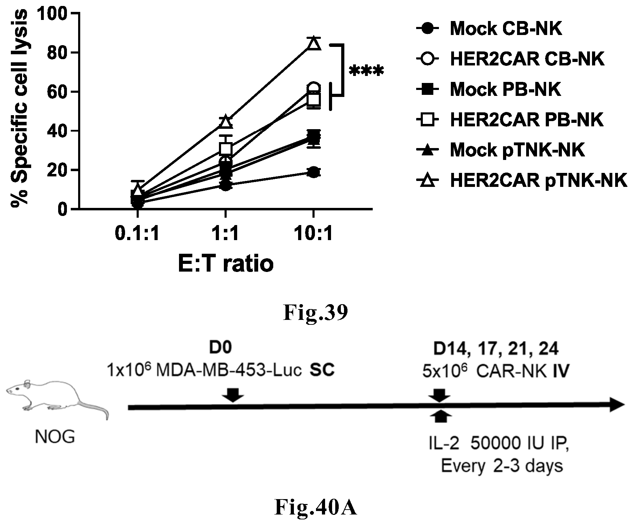

[FIG. 39] Cytotoxic activity of pT/NK cell-derived anti-HER2 CAR-NK cells, CB-NK-derived anti-HER2 CAR-NK cells, and PB-NK-derived anti-HER2 CAR-NK cells, each transduced with the CAR gene (Example C-7).
[FIG. 40A] An outline of the protocol for evaluating *in vivo* anti-tumor activity of human pT/NK cell-derived CAR-NK cells transduced with the CAR gene in Example C-8.

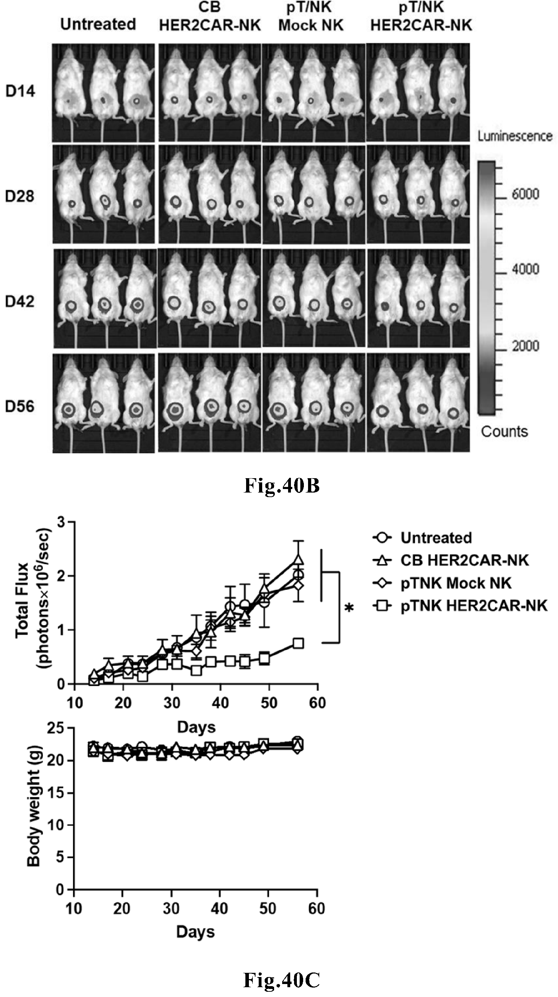

[FIG. 40B] Tumor growth in tumor-bearing mice administered with CAR-NK cells was measured by bioluminescence imaging.
[FIG. 40C] The top row shows the change over time in tumor growth in tumor-bearing mice administered with CAR-NK cells. The bottom row shows the change in body weight of each group of mice.

### MODE FOR CARRYING OUT THE INVENTION

In the present invention, "immune cell therapy" means a method of treating a disease by utilizing the biological defense function of immune cells to reduce or remove cells or tissues causing the disease. Representative examples of immune cell therapy include CAR-immune cell therapy in which a tumor is treated by reducing or removing cells expressing an antigen using immune cells expressing a CAR that specifically recognizes the antigen specifically expressed in cells contained in tumor tissue. The representative examples also include, for example, immunotherapy targeting a B-cell-specific antigen for the treatment of an autoimmune disease, and immune cell therapy in which bacterial or viral-infected cells are reduced or removed by immune cells targeting an antigen specific to the bacteria or virus to treat a bacterial or viral infection. "Disease caused by cells expressing a target antigen" includes such various diseases, and representative examples thereof include tumors, autoimmune diseases, bacterial infections, viral infections, and the like. The term "cells expressing a target antigen" includes not only a mode in which the cells express the target antigen from their inherent nuclear or mitochondrial genomes, but also a mode in which bacteria or viruses infected into the cells express the target antigen within the cells.

In the present invention, the term exogenous receptor means a T-cell receptor (TCR), a chimeric antigen receptor (CAR), or a chimeric autoantigen receptor (CAAR). In the present description, "TCR, CAR, or CAAR" may be referred to as "TCR/CAR/CAAR". The term "cells expressing an exogenous receptor" includes TCR/CAR/CAAR-expressing cells (the cells are, for example, multipotent blood progenitor cells, the same applies hereinafter), TCR/CAR-expressing cells, TCR/CAAR-expressing cells, CAR/CAAR-expressing cells, TCR-expressing cells, CAR-expressing cells, and CAAR-expressing cells.

In addition, in the present invention, the term antigen-specific receptor means a TCR or a CAR. In the present description, "TCR or CAR" may be referred to as "TCR/CAR". The term "cells expressing an antigen-specific receptor" includes TCR/CAR-expressing cells, TCR-expressing cells, and CAR-expressing cells. Expressions such as "TCR or CAR that specifically recognizes a tumor antigen" and "treatment of a tumor using the same" used in the present specification describe one mode of utilization of TCR/CAR-expressing cells of the present invention. TCR/CAR-expressing cells targeting an antigen expressed in cells other than tumor cells can be used for the treatment of a disease treated by reducing or removing cells expressing the antigen (e.g., an autoimmune disease, a bacterial infection, etc.). Therefore, the present invention including the above description is applicable to diseases other than tumors.

In addition, in the present invention, the term chimeric receptor means a CAR or a CAAR. In the present specification, "CAR or CAAR" may be referred to as "CAR/CAAR". The term "cells expressing a chimeric receptor" includes CAR/CAAR-expressing cells, CAR-expressing cells, and CAAR-expressing cells.

TCR is, as is well known in the art, a receptor present on the T-cell surface used by T cells to recognize an antigen, and is composed of a dimer of an α chain and a β chain, or a γ chain and a δ chain. Many T cells are αβ T cells having a TCR composed of a dimer of an α chain and a β chain (αβTCR), and αβTCR is usually used as a TCR to be expressed in immune cells in the present invention.

CAR is, as is well known in the art, a receptor artificially produced by linking an antigen-recognition portion derived from an antibody that specifically recognizes an antigen, a transmembrane domain derived from a cell surface molecule such as CD8 or CD28, one or more costimulatory domains derived from an intracellular signaling domain of a costimulatory receptor molecule such as CD28, 4-IBB, OX40, or 2B4, and an activation domain derived from an intracellular signaling domain of a CD3ζ molecule. As the antigen-recognition portion, a single-chain variable region fragment (scFv) having a structure in which a heavy chain variable region (VH) and a light chain variable region (VL) of an antibody that specifically recognizes a desired antigen are linked by an appropriate linker can be preferably used. The target antigen is not limited to a tumor antigen, and various antigens can be adopted as long as they are antigens characteristically or specifically expressed in cells for which a therapeutic effect is expected by reducing or removing specific cells. For example, a CAR against an antigen specifically expressed in B cells such as CD19 is useful not only for blood cancer but also for the treatment of an autoimmune disease. A suitable costimulatory domain can be appropriately selected according to the type of immune cell to be engineered to express a CAR. For example, Non-Patent Document 1 reports that a domain derived from 2B4 can be used as a costimulatory domain of a CAR that is made to be expressed in NK cells.

CAAR (Chimeric Auto-Antibody Receptor) is well known as a chimeric receptor similar to a CAR (see WO2022/196719, WO2016/07006, Ellebrecht et al., Science, 08 Jul 2016: Vol. 353, Issue 6295, pp. 179-184, etc.). A CAAR differs in that the antigen-recognition portion in the CAR is an antigen-derived portion derived from a self-antigen, but other molecular configurations are similar to those of the CAR. The antigen-derived portion is usually composed of an oligopeptide or a polypeptide containing a part of or all the amino acid sequence of a self-antigen causing an autoimmune disease. It has been reported that immune cells engineered to express a CAAR bind to a B-cell receptor (BCR) on the surface of a pathogenic B cell that recognizes a self-antigen, and show a therapeutic effect on an autoimmune disease by reducing pathogenic B cells causing the autoimmune disease. Examples of such autoimmune diseases and self-antigens include DSG3 in pemphigus vulgaris, nicotinic acetylcholine receptor α subunit in myasthenia gravis, and the like.

In the present invention, "culture in the presence of a Notch ligand" means culture conditions in which Notch signaling of cells can be stimulated and enhanced by culturing the cells under conditions where the Notch ligand and the cells to be cultured can come into contact with each other. As a form of the Notch ligand to be used, a Notch ligand protein may be adopted as a recombinant protein, cells (e.g., feeder cells) expressing the protein may be adopted, or a culture of the cells containing the Notch ligand protein (e.g., a culture supernatant, a cell lysate, etc.) may be adopted. Examples of a Notch ligand that can be preferably used include Delta-like ligands such as Delta-like 1 (DLL1, human gene is Gene ID: 28514, NCBI Reference Sequence: NM_005618.4, SEQ ID NOs: 19 and 20) and Delta-like 4 (DLL4, human gene is Gene ID: 54567, NCBI Reference Sequence: NM_019074.4, SEQ ID NOs: 21 and 22). The amino acid sequences of DLL1 and DLL4 that can be used as the Notch ligand in the present application are not limited to the sequences shown in SEQ ID NOs: 20 and 22, and proteins with amino acid sequences having 90% or more, for example, 95% or more, or 98% or more identity with these sequences can also be used as DLL1 and DLL4. Examples of a method of culturing the cell population in the presence of a Notch ligand include a method of culturing target cells on feeder cells expressing the Notch ligand, and a method of culturing the cell population in a culture vessel (a plate, a dish, etc.) whose inner surface is coated with a Notch ligand protein (which may be a fragment thereof containing at least an extracellular region) or a culture of feeder cells expressing the Notch ligand. Various feeder cells expressing a Notch ligand are known and can be easily obtained from cell banks and the like. Specific examples thereof include OP9 cells (OP9/DLL1 cells, etc.), Tst-4 cells (Tst-4/DLL1 cells, etc.), S17 cells (S17/DLL1 cells, etc.), and MS-5 cells (MS-5/DLL1 cells, etc.), each engineered for forced expression of a Notch ligand.

In the present invention, "culture in the absence of a Notch ligand" means culture in a culture system substantially not containing a Notch ligand. Co-culture with feeder cells that do not substantially express a Notch ligand corresponds to culture in the absence of a Notch ligand. In "culture in the absence of a Notch ligand" in the present invention, feeder cells that do not substantially express a Notch ligand may be used, but basically do not need to be used.

It is known that human T/NK progenitor cells, immature NK cells, and mature NK cells are cells defined by the following marker expression patterns. It is known that immature NK cells are similar to the phenotype of NK cells isolated from umbilical cord blood (umbilical cord blood NK cells), and that mature NK cells represent the phenotype of NK cells isolated from peripheral blood (peripheral blood NK cells).

Human T/NK progenitor cells: CD3-negative, CD4-negative, CD8-negative, CD56-negative, CD161-negative, and CD7-positive (CD3⁻ CD4⁻ CD8⁻ CD56⁻ CD161⁻ CD7⁺)

Human immature NK cells: CD3-negative, CD4-negative, CD8-negative, CD56-positive, CD161-positive, CD16-negative, and KIR-negative (CD3⁻ CD4⁻ CD8⁻ CD56⁺ CD161⁺ CD16⁻ KIR⁻)

Human mature NK cells: CD3-negative, CD4-negative, CD8-negative, CD56-positive, CD161-positive, CD16-positive, and KIR-positive (CD3⁻ CD4⁻ CD8⁻ CD56⁺ CD161⁺ CD16⁺ KIR⁺)

CD16 and KIR (killer cell immunoglobulin-like receptor) are known as maturation markers that serve as indicators of the maturity of human NK cells. The expression levels of CD16 and KIR in NK cells increase as maturation progresses. Among NK cells positive for the expression of CD16 and KIR, the lower the expression levels of these markers, the more immature the cells are evaluated to be.

Cultured human T/NK progenitor cells, which is one of the inventions provided by the present invention, are human T/NK progenitor cells isolated from a living body and cultured, or induced to differentiate from pluripotent cells such as multipotent blood progenitor cells *in vitro* and cultured, and are characterized by being CD122-positive. The fact that CD122 serves as a specific marker for human T/NK progenitor cells was first discovered by the present inventors in the following Examples. The cultured human T/NK progenitor cells of the present invention may be cells further satisfying 1, 2, 3, 4, 5, or 6 surface antigen expression conditions selected from the group consisting of CD3-negative, CD4-negative, CD8-negative, CD56-negative, CD161-negative, and CD7-positive. In one mode, the cultured human T/NK progenitor cells of the present invention are CD122-positive and CD3-negative. In another mode, the cultured human T/NK progenitor cells of the present invention are CD122-positive, CD3-negative, and CD4-negative. In yet another mode, the cultured human T/NK progenitor cells of the present invention are CD122-positive, CD3-negative, CD4-negative, and CD8-negative. In yet another mode, the cultured human T/NK progenitor cells of the present invention are CD122-positive, CD3-negative, CD4-negative, CD8-negative, and CD56-negative. In yet another mode, the cultured human T/NK progenitor cells of the present invention are CD122-positive, CD3-negative, CD4-negative, CD8-negative, CD56-negative, and CD161-negative. In yet another mode, the cultured human T/NK progenitor cells of the present invention are CD122-positive, CD3-negative, CD4-negative, CD8-negative, CD56-negative, CD161-negative, and CD7-positive. The expression of these surface antigen markers can be examined by well-known methods such as a flow cytometer using an antibody against each surface antigen marker and real-time PCR using a primer/probe for detecting the expression of each surface antigen marker.

### I. A method of producing an immune cell expressing an exogenous receptor

The method of producing an immune cell expressing an exogenous receptor that is a TCR, a CAR, or a CAAR according to the present invention is classified into two modes, depending on the difference in the step of introducing the exogenous receptor gene.

Mode A is a method of producing a TCR/CAR/CAAR-expressing immune cell by introducing an exogenous receptor gene into a multipotent blood progenitor cell and then inducing their differentiation into an immune cell, and comprises the steps of: preparing multipotent blood progenitor cells from a cell population comprising hematopoietic stem cells; preparing multipotent blood progenitor cells expressing the exogenous receptor by introducing a gene encoding the exogenous receptor into the multipotent blood progenitor cells; and inducing differentiation of the multipotent blood progenitor cells expressing the exogenous receptor into immune cells.

Mode B is a method of producing a TCR/CAR/CAAR-expressing immune cell by inducing differentiation of a multipotent blood progenitor cell into an immune cell and then introducing an exogenous receptor gene, and comprises the steps of: preparing multipotent blood progenitor cells from a cell population comprising hematopoietic stem cells; inducing differentiation of the multipotent blood progenitor cells into immune cells; and introducing a gene encoding the exogenous receptor into the immune cells.

Immune cells include T cells, NK cells, macrophages, and B cells.

When the exogenous receptor is a TCR, since TCR does not function in NK cells, macrophages and B cells even if the TCR alone is introduced, it is preferable to induce differentiation of the multipotent blood progenitor cells into T cells. However, a system has been developed to induce antigen-specific signal transduction by a TCR through the co-introduction of CD3 into NK cells along with the TCR (Mensali et al., eBioMed. 40. 106-117 (2019)). In this system, CD3 forms a complex with the TCR and transmits intracellular TCR signals. This technology enables the TCR to be made to function even in NK cells, macrophages, and B cells. Therefore, the present invention also encompasses production of TCR-expressing NK cells, macrophages or B cells, and in this case, a CD3 gene is also introduced into cells in combination with the TCR gene.

When the exogenous receptor is a CAR, since the CAR can function in various immune cells such as NK cells, macrophages and B cells, the cells can be preferably induced to differentiate into the various immune cells described above.

Since a CAAR is a chimeric receptor based on CAR technology, it can be said that the CAAR can also function in various immune cells such as NK cells, macrophages and B cells, similar to a CAR. Therefore, even when the exogenous receptor is a CAAR, the cells can be preferably induced to differentiate into the various immune cells described above.

### I-1. Preparation of multipotent blood progenitor cells (Mode A, Mode B)

A multipotent blood progenitor cell is a cell whose direction of differentiation is more restricted than that of a hematopoietic stem cell, but has multipotency capable of differentiating into a plurality of cell lineages and has self-renewal ability. In the present invention, an induced leukocyte stem cell (iLS) or a T/NK progenitor cell can be preferably used as the multipotent blood progenitor cell.

A cell population comprising hematopoietic stem cells can be obtained from umbilical cord blood, bone marrow fluid, or peripheral blood, and usually comprises hematopoietic stem cells and hematopoietic progenitor cells. Human hematopoietic stem cells are determined as CD34-positive, CD59-positive, CD90/Thy1-positive, CD38 weakly positive or negative, c-Kit negative or weakly positive, and Lineage marker negative (CD34⁺ CD59⁺ CD90/Thyl⁺ CD38^{low/-} c-Kit^{-/low} Lin⁻). For example, a cell population comprising hematopoietic stem cells (a cell population in which hematopoietic stem cells account for approximately 3% or more, for example, 7% or more) can be obtained by separating and recovering CD34+ cells by cell sorting or magnetic beads.

As described later, when the TCR/CAR/CAAR-expressing immune cells produced according to the present invention are used in the treatment of a tumor or an autoimmune disease, it is also possible to obtain a cell population comprising hematopoietic stem cells from bone marrow fluid or peripheral blood collected from a patient to prepare multipotent blood progenitor cells derived from the patient's own cells, in addition to preparation from umbilical cord blood which is generally not derived from the patient themselves.

When the multipotent blood progenitor cell is an iLS cell, its preparation method is known as described in Non-Patent Documents 2 and 3 and Patent Document 1, and iLS cells can be prepared by culturing a cell population comprising hematopoietic stem cells under conditions that induce differentiation into B cells while inhibiting the function of transcription factor E2A (also known as transcription factor 3 (TCF3)) in the cells.

Examples of a method of inhibiting the function of E2A include a method of enhancing a factor that binds to E2A and suppresses E2A from acting on its target gene, a method of introducing a dominant-negative mutant in which a functional domain of E2A is inactivated, and a method of inhibiting expression or activity of other transcription factor(s) that is/are under the control of E2A or function(s) cooperatively with E2A. However, a method of forced expression of a differentiation inhibitory factor Id (Inhibitor of DNA binding) in hematopoietic stem cells is preferable. Id proteins are known to dominantly-negatively inhibit E proteins such as E2A (Norton, J.D. et al., Trends Cell Biol. 8, 58-65 (1998); and Sayegh, C.E. et al., Nat. Immunol. 4, 586-593 (2003)). By forced expression of an Id protein in hematopoietic stem cells, the Id protein can bind to E2A and suppress E2A from acting on its target gene.

As the Id factor, any of the four types existing in mammals, Id1 to Id4, may be used. Gene sequence information for Id1 to Id4 is registered in databases such as GenBank and is easily available. For example, sequence information for the human Id1 gene is registered in GenBank under accession numbers NM_002165 (transcript variant 1) and NM_181353 (transcript variant 2), sequence information for the human Id2 gene is registered under NM_002166, sequence information for the human Id3 gene is registered under NM_002167, and sequence information for the human Id4 gene is registered under NM_001546. Among these, the human Id3 gene and protein sequences of NM_002167 used in the Examples below are shown in SEQ ID NOs: 5 and 6, respectively, of the Sequence Listing. Suitable primers can be designed based on these known sequence information, and DNA encoding Id can be amplified by performing RT-PCR using RNA extracted from cells or tissues expressing Id as a template. By incorporating the obtained DNA into an appropriate expression vector under the control of an appropriate promoter that exhibits constitutively strong promoter activity in mammalian cells (CMV promoter, SV40 promoter, LTR promoter, etc.) and introducing it into a cell population comprising hematopoietic stem cells, the Id protein can be forcedly expressed in the hematopoietic stem cells. As the expression vector, animal virus vectors such as retroviral vectors, adenoviral vectors, and lentiviral vectors can be preferably used, but plasmid vectors for mammalian cells can also be used. In order to sustain the expression of the Id factor in the cells, the DNA encoding Id is preferably introduced so as to be integrated into the chromosomes of the hematopoietic stem cells, and a retroviral vector or a lentiviral vector, which can integrate a transgene into the chromosome, can be preferably used. By infecting cells with these viral vectors carrying the DNA encoding Id by a spin infection method or the like, the DNA encoding Id can be integrated into the chromosome by random integration. However, it is also possible to introduce it using a viral vector that can exist stably extra-chromosomally, such as a persistent expression-type Sendai virus vector (e.g., J. Biol. Chem. 282, 27383-27391, 2007).

Modes of E2A inhibition by forced expression of the Id factor include constitutive E2A inhibition in which E2A is constitutively inhibited in cells, and controlled E2A inhibition in which E2A is inhibited only under certain conditions. When the multipotent blood progenitor cells into which a TCR gene or a CAR gene has been introduced are induced to differentiate into B cells in a later step, it is preferable to perform controlled E2A inhibition such that the E2A inhibition can be relieved. When the cells are induced to differentiate into immune cells other than B cells, constitutive E2A inhibition may be used, and controlled E2A inhibition can also be preferably used.

Constitutive E2A inhibition may be performed by expressing DNA encoding the Id factor under the control of an appropriate promoter that constitutively exhibits strong promoter activity in mammalian cells.

Examples of the method of controlled E2A inhibition includes a system for controlling E2A inhibition by 4-hydroxytamoxifen (4-OHT) using a ligand-binding domain of a human estrogen receptor, which is used in the Examples below. In this system, an ID-ER gene in which a ligand-binding domain sequence of a human estrogen receptor (hER) is linked downstream of DNA encoding the Id factor is introduced into cells, and an ID-ER protein in which the hER ligand-binding domain is fused to the C-terminal side of the Id factor is made to be expressed in the cells. In the absence of 4-OHT, the expressed ID-ER protein remains in the cytoplasm, but in the presence of 4-OHT, the ID-ER protein translocates into the nucleus and inhibits the transcription factor E2A (Non-Patent Document 3). Therefore, by culturing iLS cells with 4-OHT added to the medium, the iLS cells can be maintained without differentiation. When inducing differentiation into immune cells, it is not necessary to add 4-OHT to the medium, and differentiation induction may be performed in a medium not supplemented with 4-OHT.

As the sequence of the hER ligand-binding domain, the sequence at positions 370 to 1311 in the base sequence shown in SEQ ID NO: 1 can be preferably used. This sequence is a sequence of a mutant hER ligand-binding domain (ERT2) designed by modifying hER isoform 1 (SEQ ID NO: 7) of GenBank Accession No. NM_000125 such that several base substitutions are added to its ligand-binding domain sequence (positions 1078 to 2019 of SEQ ID NO: 7; the sequence of this 942-base region is shown in SEQ ID NO: 9). SEQ ID NO: 1 is the base sequence of an ID3-ERT2 gene, which is a preferred example of an ID-ER gene, in which the ERT2 sequence is fused to the full-length human ID3 gene cDNA. However, the sequence encoding the hER ligand-binding domain used for the ID-ER gene is not limited to this mutant sequence, and a sequence in which 10 or fewer bases are substituted or added in the base sequence of the ligand-binding domain of hER isoform 1 shown in SEQ ID NO: 9 can be used as the hER ligand-binding domain sequence. DNA having a structure in which the hER ligand-binding domain sequence is linked to the 3' end of the DNA encoding the Id factor via an appropriate spacer sequence can be used as the ID-ER gene.

DNA encoding an arbitrary protein that serves as a marker for gene introduction may be linked to the ID gene (DNA encoding the Id factor) or the ID-ER gene. By separating and recovering cells expressing the marker protein with a cell sorter from the cell population comprising hematopoietic stem cells after the gene introduction treatment, it is possible to obtain hematopoietic stem cells into which the ID gene or ID-ER gene has been introduced and which express the Id factor.

After obtaining hematopoietic stem cells in which E2A is inhibited by expression of the Id factor or the like, the cells are cultured under conditions that induce differentiation into B cells. When controlled E2A inhibition is performed, differentiation induction culture into B cells is performed under E2A inhibitory conditions (in the case of a system using the ligand-binding domain of the human estrogen receptor, in the presence of 4-OHT).

Culture under conditions that induce differentiation into B cells is culture on feeder cells having an ability to support differentiation into B cells, and an appropriate amount of cytokines is added to the medium. Examples of feeder cells having an ability to support differentiation into B cells include OP9 cells, TSt-4 cells, S17 cells, and MS-5 cells. As the cytokines, for example, IL-7, Flt3-L, SCF, and the like can be used at concentrations of about 0.1 to 20 ng/mL each.

When the multipotent blood progenitor cell is a T/NK progenitor cell (pT/NK cell), its preparation method is known as described in Non-Patent Documents 2, 4, and 5. By culturing a cell population comprising CD34-positive hematopoietic stem cells with the addition of an appropriate amount of cytokines in the presence of a Notch signal, pT/NK cells having the ability to differentiate into T cells and NK cells and self-renewal ability can be generated. As the cytokines, SCF, IL-7, and Flt3-L can be preferably used. In the case of mouse cells, pT/NK cells proliferate vigorously by adding SCF, IL-7, and Flt3-L to the medium at concentrations of 10 to 50 ng/ml each, particularly at high concentrations of 25 to 50 ng/ml each, and culturing in the presence of a Notch signal. On the other hand, when producing pT/NK cells in large quantities from human hematopoietic stem cells, it is preferable to use SCF, IL-7, and Flt3-L at lower concentrations than the above. Specifically, as shown in A-2-10, B-1, and B-2, etc., of the Examples below, pT/NK cells can be expanded in large quantities by adding the same cytokines to the medium at low concentrations of 1 ng/mL or more and less than 10 ng/ml each and culturing in the presence of a Notch signal. Further, in the case of human cells, by further adding TPO or M-CSF to the medium, it becomes possible to expand pT/NK cells several times more (see B-8 to B-10, etc., of the Examples). The production of pT/NK cells from human hematopoietic stem cells will be described in detail in "II.Method of producing human T/NK progenitor cells".

By culturing a cell population comprising CD34-positive hematopoietic stem cells in the presence of a Notch ligand, the Notch signal of the hematopoietic stem cells can be stimulated and enhanced. The definition of the term "culture in the presence of a Notch ligand," the form of the Notch ligand to be used, the Notch ligand that can be preferably used, and the culture method in the presence of a Notch ligand are as described above.

The human hematopoietic stem cells used in the above-described method may be subjected to an expansion operation in advance. Examples of the method for such expansion culture include a method in which a cell population comprising hematopoietic stem cells is cultured in the absence of a Notch ligand in a medium comprising SCF, TPO, and G-CSF at concentrations of 100 ng/mL or more each. By culturing hematopoietic stem cells in the presence of the high-concentration cytokines described above in the absence of a Notch ligand, CD34-positive hematopoietic stem cells proliferate. Such expansion culture can be applied to all methods using a cell population comprising hematopoietic stem cells in the present invention. That is, the cell population comprising hematopoietic stem cells used in the present invention may be a cell population after expansion of CD34-positive hematopoietic stem cells by the expansion culture described above.

### I-2. Introduction of an exogenous receptor gene into multipotent blood progenitor cells (Mode A)

In Mode A, a gene encoding an exogenous receptor (TCR gene, CAR gene, or CAAR gene) is introduced into multipotent blood progenitor cells such as iLS cells and pT/NK cells to prepare multipotent blood progenitor cells expressing the receptor. If desired, in addition to the exogenous receptor gene, other gene(s) (e.g., a CD3 gene) may be introduced into the multipotent blood progenitor cells.

When the exogenous receptor is an antigen-specific receptor, a target antigen can be selected according to the use of the multipotent blood progenitor cells or immune cells that express the TCR/CAR. When using these cells in the treatment of a tumor, a tumor antigen is selected. Specific examples of tumor antigens include GD2 (a glycolipid on the cell surface, specifically expressed in neuroblastoma), WT1 (a Wilms tumor gene, expressed in leukemia and various solid cancers), survivin (belongs to the inhibitor of apoptosis (IAP) family, expressed in many solid cancers and blood cancers), MAGE-A3 and MAGE-A4 (expressed in many cancer types such as melanoma, esophageal cancer, non-small cell lung cancer, head and neck cancer, and bladder cancer), tyrosinase (melanoma antigen), gp100 (melanoma antigen), CEA (expressed in colorectal cancer), Melan-A (melanoma antigen), TRP-2 (melanoma antigen), CDK4 (cyclin-dependent kinase 4, expressed in melanoma), NY-ESO-1 (frequently expressed in synovial sarcoma, also expressed in other tumors), HER2 (expressed in breast cancer, ovarian cancer, etc.), MUC-1 (highly expressed in many cancers such as breast cancer, lung cancer, pancreatic cancer, and colorectal cancer), p53 (expressed in various cancer types), and the like.

For any of the tumor antigens, sequence information such as gene sequences and amino acid sequences is known, and antibodies that specifically recognize the tumor antigens are known or can be produced by conventional methods. A CAR gene can be designed and produced using variable region sequences of an antibody that specifically recognizes a tumor antigen. Regardless of the type of antigen, when human-derived cells are engineered to express a CAR, it is preferable to use human protein-derived domains for the intracellular domains (costimulatory domain and activation domain) of the CAR.

A TCR gene that recognizes a desired antigen can be obtained from an antigen-specific killer T-cell clone. A killer T-cell clone specific for a desired antigen can be obtained by causing an antigen-presenting cell such as a dendritic cell to take up a desired antigen protein or a CTL epitope peptide thereof to prepare an antigen-presenting cell that presents the CTL epitope peptide on the cell surface, and co-culturing the antigen-presenting cell with T cells to bring both into contact with each other.

Furthermore, when an expression vector carrying a TCR gene that recognizes a desired antigen is already known, the TCR gene can be prepared by PCR amplification from such an expression vector.

Examples of antigens other than tumor antigens include viral antigens and parasite antigens. Specific examples of viral antigens include constituent proteins of viruses such as hepatitis viruses (HBV, HCV, etc.), human papillomavirus, and human immunodeficiency virus. Examples of parasite antigens include malaria parasite proteins and the like. Since information such as gene sequences and amino acid sequences is also known for these antigens, genes for TCRs and CARs that specifically recognize these antigens can be obtained in the same manner as described above.

When the exogenous receptor is a CAAR, multipotent blood progenitor cells or immune cells expressing the CAAR can be used for the treatment of an autoimmune disease. A self-antigen used for an antigen-derived portion of a CAAR can be selected from various self-antigens known as causes of autoimmune diseases. Representative examples of autoimmune diseases and self-antigens are as described above, but are not limited thereto. For example, in the case of treating pemphigus vulgaris, the antigen-derived portion of the CAAR can be designed based on DSG3, and in the case of treating myasthenia gravis, it can be designed based on the nicotinic acetylcholine receptor α subunit.

Introduction of an exogenous receptor gene or a CD3 gene into multipotent blood progenitor cells can be performed basically by the same method as the introduction of an ID gene or an ID-ER gene into hematopoietic stem cells. In gene introduction into human pT/NK cells, when a gene is introduced using a viral vector, the efficiency of gene introduction can be increased by using an envelope of Gibbonape leukemia virus (GALV) as an envelope of the virus. Specifically, a TCR/CAR/CAAR gene-expressing virus having a GALV envelope may be produced and used to infect pT/NK cells by a spin infection method or the like. In the present invention, for example, a protein with an amino acid sequence having 90% or more, for example, 95% or more, or 98% or more sequence identity with the amino acid sequence shown in SEQ ID NO: 16 or 24 can be used as the GAVL envelope. The amino acid sequence shown in SEQ ID NO: 24 is the amino acid sequence encoded by the envelope gene (SEQ ID NO: 23) of GALV SEATO strain registered in GenBank as AF055060.1. The amino acid sequence shown in SEQ ID NO: 16 is an amino acid sequence encoded by a synthetic gene GALV-MTR (SEQ ID NO: 15) (Caeser et al., Nat Commun. 2019 Oct 4;10(1):4543) of a GALV envelope in which the insertion efficiency of the GALV envelope protein into viral particles is increased by replacing the membrane domain and cytoplasmic domain of the GALV envelope (residues 627-685 in the amino acid sequence shown in SEQ ID NO: 24) with the membrane domain and cytoplasmic domain of a Mouse Leukemia virus (MuLV) envelope.

By separating and recovering cells that express a marker protein linked to a transgene using a cell sorter, cells into which an exogenous receptor gene has been introduced and which express the receptor can be isolated. The isolated receptor-expressing cells are expanded by culturing while appropriately exchanging medium and feeder cells while maintaining a multipotent state. When introducing an exogenous receptor gene into iLS cells produced by controlled E2A inhibition, gene introduction, isolation, and expansion are performed while culturing the iLS cells under E2A inhibitory conditions. When a system for controlling E2A inhibition by 4-OHT is used, receptor gene introduction, isolation, and expansion are performed using a medium containing 4-OHT.

### I-3. Induction of differentiation of multipotent progenitor cells expressing an exogenous receptor into immune cells (Mode A)

In Mode A, the expanded TCR/CAR/CAAR-expressing multipotent blood progenitor cells are induced to differentiate into immune cells such as T cells, NK cells, macrophages, or B cells. When using iLS cells produced by controlled E2A inhibition, it is not necessary to continue the E2A inhibitory conditions in this step. For example, when a system for controlling E2A inhibition by 4-OHT is used, a medium containing 4-OHT may be used at the start of the differentiation induction treatment, but the differentiation induction treatment may be subsequently proceeded using a medium not containing 4-OHT.

When the multipotent blood progenitor cells are iLS cells, the cells can be induced to differentiate into various immune cells such as T cells, NK cells, macrophages, and B cells. On the other hand, pT/NK cells have the ability to differentiate into T cells, NK cells, and myeloid cells (macrophages, etc.), but do not have the ability to differentiate into B cells. Therefore, when the multipotent blood progenitor cells are pT/NK cells, the cells are induced to differentiate into T cells, NK cells, or macrophages, preferably NK cells or macrophages, particularly preferably NK cells.

In general, when producing TCR-expressing T cells (TCR-T cells) and CAR-expressing T cells (CAR-T cells), iLS cells are preferably used. When producing NK cells expressing a CAR or a CAAR (CAR-NK cells, CAAR-NK cells) and macrophages expressing a CAR or a CAAR (CAR-macrophages, CAAR-macrophages), pT/NK cells and iLS cells, particularly pT/NK cells, are preferably used.

In one mode, the TCR/CAR/CAAR-expressing multipotent blood progenitor cells are TCR-expressing iLS cells, which are induced to differentiate into T cells to thereby obtain TCR-T cells.

In another mode, the TCR/CAR/CAAR-expressing multipotent blood progenitor cells are CAR-expressing iLS cells, which are induced to differentiate into T cells, NK cells, macrophages or B cells to thereby obtain CAR-T cells, CAR-NK cells, CAR-macrophages or CAR-expressing B cells (CAR-B cells), or are induced to differentiate into T cells, NK cells or macrophages to thereby obtain CAR-T cells, CAR-NK cells or CAR-macrophages. In yet another mode, the TCR/CAR/CAAR-expressing multipotent blood progenitor cells are CAAR-expressing iLS cells, which are induced to differentiate into T cells, NK cells, macrophages or B cells to thereby obtain CAAR-T cells, CAAR-NK cells, CAAR-macrophages or CAAR-B cells, or are induced to differentiate into T cells, NK cells or macrophages to thereby obtain CAAR-T cells, CAAR-NK cells or CAAR-macrophages.

In yet another mode, the TCR/CAR/CAAR-expressing multipotent blood progenitor cells are TCR/CAR/CAAR-expressing pT/NK cells, which are induced to differentiate into T cells to thereby obtain TCR/CAR/CAAR-T cells.

In yet another mode, the TCR/CAR/CAAR-expressing multipotent blood progenitor cells are CAR-expressing pT/NK cells, which are induced to differentiate into NK cells to thereby obtain CAR-NK cells.

In yet another mode, the TCR/CAR/CAAR-expressing multipotent blood progenitor cells are CAAR-expressing pT/NK cells, which are induced to differentiate into NK cells to thereby obtain CAAR-NK cells.

In yet another mode, the TCR/CAR/CAAR-expressing multipotent blood progenitor cells are CAR-expressing pT/NK cells, which are induced to differentiate into macrophages to thereby obtain CAR-macrophages.

In yet another mode, the TCR/CAR/CAAR-expressing multipotent blood progenitor cells are CAAR-expressing pT/NK cells, which are induced to differentiate into macrophages to thereby obtain CAAR-macrophages.

In yet another mode, the TCR/CAR/CAAR-expressing multipotent blood progenitor cells are TCR-expressing iLS cells, and the cells further express CD3. The cells are induced to differentiate into NK cells, macrophages or B cells to thereby obtain TCR-expressing NK cells, TCR-expressing macrophages or TCR-expressing B cells (all of which also express CD3), or are induced to differentiate into NK cells or macrophages to thereby obtain TCR-expressing NK cells or TCR-expressing macrophages (both of which also express CD3).

In yet another mode, the TCR/CAR/CAAR-expressing multipotent blood progenitor cells are TCR-expressing pT/NK cells, and the cells further express CD3. The cells are induced to differentiate into NK cells or macrophages to thereby obtain TCR-expressing NK cells or TCR-expressing macrophages.

### Induction of differentiation of TCR/CAR/CAAR-expressing multipotent blood progenitor cells into T cells

In the induction of differentiation of multipotent blood progenitor cells expressing a TCR, a CAR or a CAAR into T cells, first, the cells are cultured in a state where Notch signaling is stimulated, i.e., in the presence of a Notch ligand, with an appropriate amount of cytokines added to the medium. The method for stimulating Notch signaling is as described above, and includes a method of culturing on feeder cells expressing a Notch ligand (specific examples of such feeder cells are as described above) and a method of culturing in a culture vessel whose inner surface is coated with a Notch ligand protein (which may be a fragment thereof containing at least an extracellular region). As the cytokines, for example, IL-7, Flt3-L, and the like can be used at concentrations of about 0.1 to 10 ng/mL each. Culture is continued while appropriately performing medium exchange and, in the case of using feeder cells, exchange of the feeder cells. About 3 weeks after the start of induction, CD4/CD8 double-positive (DP) cells, which are immature T cells, are generated.

By subjecting the DP cells to TCR stimulation, differentiation into CD8-positive killer T cells (cytotoxic T cells) can be induced. Examples of a method for subjecting DP cells to TCR stimulation include a method of treating the DP cells with an anti-CD3 antibody and an anti-CD28 antibody, and, when the exogenous receptor is an antigen-specific receptor, a method of co-culturing the DP cells with antigen-presenting cells (such as dendritic cells) that present an antigen recognized by the introduced antigen-specific receptor. When the introduced exogenous receptor is a CAR or a CAAR using a costimulatory receptor molecule intracellular signaling domain derived from 4-IBB, a method of treating the DP cells with an anti-CD3 antibody and an anti-4-IBB antibody can also be used. By subjecting the DP cells to TCR stimulation by any of the methods described above in a medium containing appropriate cytokines (IL-7, IL-15, IL-2, etc.), CD8-positive CAR-T cells or CD8-positive CAAR-T cells can be generated and expanded.

### Induction of differentiation of TCR/CAR/CAAR-expressing multipotent blood progenitor cells into NK cells

The method for inducing differentiation of multipotent blood progenitor cells expressing a CAR or a CAAR into NK cells differs depending on the breadth of the differentiation potential of the multipotent blood progenitor cells.

In the case of iLS cells expressing a CAR or a CAAR, CAR/CAAR-NK cells can be generated by culturing the cells on feeder cells having an ability to support differentiation into B cells or under feeder-free conditions in a medium containing appropriate cytokines (e.g., IL-15 and IL-2).

In the case of pT/NK cells expressing a CAR or a CAAR, CAR/CAAR-NK cells can be generated by culturing the cells in a medium containing appropriate cytokines (e.g., IL-7, Flt3-L, SCF, and IL-15). In this culture, a Notch ligand may or may not be present. Culture in the absence of a Notch ligand is preferable.

Induction of differentiation of multipotent blood progenitor cells expressing a TCR and CD3 into NK cells can also be performed in the same manner as the induction of differentiation of multipotent blood progenitor cells expressing a CAR or a CAAR into NK cells.

Although the generated CAR/TCR/CAAR-NK cells have sufficient proliferation ability, the proliferation ability may be further enhanced by performing receptor stimulation if desired. Receptor stimulation of CAR/TCR/CAAR-NK cells can be achieved by a method such as co-culturing the CAR/TCR-NK cells with a K562 leukemia cell line expressing membrane-bound IL-21 (memIL-21) and 4-1BB ligand (41BBL), or culturing the CAR/TCR/CAAR-NK cells in the presence of cytokines such as IL-12, IL-15, IL-18, IL-21, and IL-2.

### Induction of differentiation of TCR/CAR/CAAR-expressing multipotent blood progenitor cells into macrophages

When inducing differentiation of multipotent blood progenitor cells expressing a CAR or a CAAR into macrophages, macrophages expressing a CAR or a CAAR (CAR/CAAR-macrophages) can be generated by culturing the cells on OP9 cells in a medium containing appropriate cytokines (e.g., SCF, M-CSF, IL-3, and IL-6).

Induction of differentiation of multipotent blood progenitor cells expressing a TCR and CD3 into macrophages can also be performed in the same manner as the induction of differentiation of multipotent blood progenitor cells expressing a CAR or a CAAR into macrophages.

Although the generated CAR/TCR/CAAR-macrophages have sufficient proliferation ability, the proliferation ability may be further enhanced by performing receptor stimulation if desired. Receptor stimulation of CAR/TCR/CAAR-macrophages can be achieved by a method such as culturing the CAR/TCR/CAAR-macrophages in the presence of M-CSF or GM-CSF.

### Induction of differentiation of TCR/CAR/CAAR-expressing multipotent blood progenitor cells into B cells

In the induction of differentiation of multipotent blood progenitor cells expressing a CAR or a CAAR into B cells, CAR/CAAR-B cells can be generated by culturing the cells on OP9 cells in a medium containing appropriate cytokines (e.g., IL-7 and SCF).

Induction of differentiation of multipotent blood progenitor cells expressing a TCR and CD3 into B cells can also be performed in the same manner as the induction of differentiation of multipotent blood progenitor cells expressing a CAR or a CAAR into B cells.

### I-4. Induction of differentiation of multipotent blood progenitor cells into immune cells (Mode B)

In Mode B, the prepared multipotent blood progenitor cells are induced to differentiate into immune cells. This induction of differentiation can be performed basically by the same method as in I-3. It is the same in Mode B that iLS cells can be induced to differentiate into various immune cells such as T cells, NK cells, macrophages and B cells, while pT/NK cells are induced to differentiate into T cells, NK cells or macrophages, preferably NK cells or macrophages, and particularly preferably NK cells. It is also the same that iLS cells are preferably used for production of TCR-expressing T cells (TCR-T cells), CAR-expressing T cells (CAR-T cells) and CAAR-expressing T cells (CAAR-T cells); and that pT/NK cells and iLS cells, particularly pT/NK cells, are preferably used for production of NK cells expressing a CAR or a CAAR (CAR-NK cells, CAAR-NK cells) and macrophages expressing a CAR or a CAAR (CAR-macrophages, CAAR-macrophages).

In one mode, the multipotent blood progenitor cells are iLS cells, and are induced to differentiate into T cells. In the next step, a TCR gene is introduced.

In another mode, the multipotent blood progenitor cells are iLS cells, and are induced to differentiate into T cells, NK cells, macrophages or B cells, or are induced to differentiate into T cells, NK cells or macrophages. In the next step, a CAR gene is introduced.

In yet another mode, the multipotent blood progenitor cells are iLS cells, and are induced to differentiate into T cells, NK cells, macrophages or B cells, or are induced to differentiate into T cells, NK cells or macrophages. In the next step, a CAAR gene is introduced.

In yet another mode, the multipotent blood progenitor cells are pT/NK cells, and are induced to differentiate into T cells. In the next step, a TCR gene is introduced.

In yet another mode, the multipotent blood progenitor cells are pT/NK cells, and are induced to differentiate into NK cells. In the next step, a CAR gene is introduced.

In yet another mode, the multipotent blood progenitor cells are pT/NK cells, and are induced to differentiate into NK cells. In the next step, a CAAR gene is introduced.

In yet another mode, the multipotent blood progenitor cells are pT/NK cells, and are induced to differentiate into macrophages. In the next step, a CAR gene is introduced.

In yet another mode, the multipotent blood progenitor cells are pT/NK cells, and are induced to differentiate into macrophages. In the next step, a CAAR gene is introduced.

In yet another mode, the multipotent blood progenitor cells are iLS cells, and are induced to differentiate into NK cells, macrophages or B cells, or are induced to differentiate into NK cells or macrophages. In the next step, a TCR gene is introduced, and a CD3 gene is further introduced.

In yet another mode, the multipotent blood progenitor cells are pT/NK cells, and are induced to differentiate into NK cells or macrophages. In the next step, a TCR gene is introduced, and a CD3 gene is further introduced.

### I-5. Introduction of an exogenous receptor gene into immune cells (Mode B)

When introducing a TCR, a CAR or a CAAR into immune cells, receptor stimulation (referred to as TCR stimulation in the case of T cells) according to the type of immune cell is required, and the exogenous receptor gene is introduced into the immune cells after the receptor stimulation. Although general cell stimulation methods before gene introduction are described below, stimulation methods according to the type of cell are well known in the art, and methods other than these can also be used. For the introduction of an exogenous receptor gene into cells, animal virus vectors such as retroviral vectors and lentiviral vectors can be preferably used, as explained in the method for preparing iLS cells in I-1. If desired, a CD3 gene may be introduced simultaneously with the exogenous receptor gene.

When introducing a TCR/CAR/CAAR gene into T cells, the T cells are subjected to TCR stimulation with an anti-CD3 antibody (an anti-CD28 antibody may be added together) and IL-2 to expand them, and then the TCR/CAR/CAAR gene is introduced by a spin infection method or the like using a viral vector.

When introducing a CAR/CAAR gene, or a TCR gene and a CD3 gene, into NK cells, the NK cells are subjected to receptor stimulation and expanded by co-culturing the NK cells with a K562 leukemia cell line expressing membrane-bound IL-21 (memIL-21) and 4-1BB ligand (41BBL) in the presence of appropriate cytokines (IL-2 alone, or IL-2, IL-15, and IL-21). Then, the gene(s) is/are introduced by a spin infection method or the like using a viral vector.

When introducing a CAR/CAAR gene, or a TCR gene and a CD3 gene, into macrophages, the macrophages are subjected to receptor stimulation with M-CSF or GM-CSF to expand them, and then the gene(s) is/are introduced by a spin infection method or the like using a viral vector.

When introducing a CAR/CAAR gene, or a TCR gene and a CD3 gene, into B cells, the cells are subjected to receptor stimulation with cytokines such as IL-7 and SCF, or a CD40 ligand, according to the differentiation stage, to expand them, and then the gene(s) is/are introduced by a spin infection method or the like using a viral vector.

After introducing the exogenous receptor gene, the receptor stimulation may be continued for a certain period (about several days to several weeks).

Preferred examples of an antigen recognized by the receptor when the exogenous receptor is an antigen-specific receptor, specific examples of a self-antigen used for an antigen-derived portion when the exogenous receptor is a CAAR, and methods for designing a CAR gene or a CAAR gene and methods for preparing a TCR gene are as described in I-2.

### II. Method of producing human T/NK progenitor cells

The present invention provides a novel production method of human T/NK progenitor cells that achieves improved expansion efficiency in the expansion of human T/NK progenitor cells compared to the method described in Non-Patent Document 2 applied to mouse cells, a method of producing T cells or NK cells comprising a step of inducing differentiation from the T/NK progenitor cells produced by the novel production method into T cells or NK cells, and a method of producing T cells or NK cells expressing an exogenous receptor combining the novel production method and the step of making the exogenous receptor to be expressed described above, and the like.

The method of producing human T/NK progenitor cells of the present invention (human pT/NK cell production method) comprises a step of culturing a cell population comprising human hematopoietic stem cells in the presence of a Notch ligand in a medium containing SCF, Flt3-L, and IL-7 at concentrations of 1 ng/mL or more and less than 10 ng/mL each (hereinafter, the medium used in this step is referred to as "human pT/NK cell induction medium"). Unlike the case of mice, in the human pT/NK cell induction medium, human pT/NK cells can be generated and expanded in large quantities by adopting the low concentrations described above as the concentrations of SCF, Flt3-L, and IL-7. Furthermore, when at least one of TPO and M-CSF is added to the human pT/NK cell induction medium at a predetermined concentration, the production amount of human pT/NK cells can be further increased several times.

As the human cell population comprising human hematopoietic stem cells used in the human pT/NK cell production method, those explained in I-1. can be used. The human cell population may be a cell population after the expansion culture described above. Culture in the presence of a Notch ligand is also as described above, and the Notch ligand and culture conditions explained in I-1. can be appropriately adopted. As the Notch ligand, a recombinant protein may be adopted, or feeder cells expressing a Notch ligand may be adopted.

The concentrations of SCF, Flt3-L, and IL-7 contained in the human pT/NK cell induction medium can be appropriately selected within the range of 1 ng/mL or more and less than 10 ng/mL. All three cytokines may be at the same concentration, or some or all of the three cytokines may be at different concentrations within the above range. Preferably, the concentrations are each appropriately selected from the range of 3 to 7 ng/mL, more preferably the concentrations are each appropriately selected from the range of 4 to 6 ng/mL, and even more preferably they are each about 5 ng/mL.

The concentration of TPO contained in the human pT/NK cell induction medium can be appropriately selected within the range of 1 to 100 ng/mL. The TPO concentration may be, for example, 1 to 80 ng/mL, 1 to 70 ng/mL, 1 to 60 ng/mL, 1 to 55 ng/mL, 1 to 50 ng/mL, 5 to 80 ng/mL, 5 to 70 ng/mL, 5 to 60 ng/mL, 5 to 55 ng/mL, 5 to 50 ng/mL, 10 to 80 ng/mL, 10 to 70 ng/mL, 10 to 60 ng/mL, 10 to 55 ng/mL, or 10 to 50 ng/mL.

The concentration of M-CSF contained in the human pT/NK cell induction medium can be appropriately selected within the range of 1 to 100 ng/mL. The M-CSF concentration may be, for example, 1 to 50 ng/mL, 1 to 40 ng/mL, 1 to 30 ng/mL, 5 to 50 ng/mL, 5 to 40 ng/mL, 5 to 30 ng/mL, 5 to 25 ng/mL, 7 to 50 ng/mL, 7 to 40 ng/mL, 7 to 30 ng/mL, or 7 to 25 ng/mL.

In the human pT/NK cell induction medium, TPO and M-CSF may be contained either alone or in combination. Examples of particularly preferred media include media containing SCF, Flt3-L, and IL-7 at concentrations of 4 to 6 ng/mL or 5 ng/mL each, and TPO at a concentration of 10 to 55 ng/mL or 10 to 50 ng/mL.

As the medium to be used in the method of the present invention, any media such as those used for ordinary cell culture or those formulated for culturing hematopoietic cells can be used without particular limitation. Representative examples of media used for ordinary cell culture include IMDM (Iscove's Modified Dulbecco's Medium), αMEM (alpha Modified Eagle's Minimum Essential Medium), DMEM (Dulbecco's Modified Eagle's Medium), Ham's F12 medium (Ham's Nutrient Mixture F12), McCoy's 5A medium, EMEM (Eagle's Minimum Essential Medium), and RPMI1640 medium. Representative examples of serum-free media suitable for the culture of hematopoietic cells include StemPro34 (manufactured by Invitrogen), Stemline (manufactured by Sigma-Aldrich), Stemline II (manufactured by Sigma-Aldrich), X-VIVO 10 (manufactured by Lonza), X-VIVO 15 (manufactured by Lonza), X-VIVO 20 (manufactured by Lonza), HPGM (manufactured by Lonza), StemSpan H3000 (manufactured by STEMCELL Technologies), StemSpanSFEM (manufactured by STEMCELL Technologies), and QBSF-60 (manufactured by Quality Biological). Depending on the purpose, the cells may be cultured under serum-free conditions, or conditions supplemented with serum such as FBS or a serum substitute such as Knockout Serum Replacement (manufactured by Gibco) may be adopted.

The culture period is not particularly limited, but is, for example, 7 to 56 days (preferably 28 to 42 days), and it is preferable to exchange the medium with a fresh medium at intervals of 1 to 7 days (preferably 3 to 4 days).

For other culture conditions, conditions employed in ordinary cell culture can be appropriately selected. The culture temperature is, for example, 35 to 39°C, and culture can be performed in the presence of 0 to 10% CO₂. Culture in the presence of a Notch ligand in the human pT/NK cell production method may be carried out by a method using feeder cells expressing a Notch ligand, or by another method using a culture vessel whose inner surface is coated with a Notch ligand protein or the like.

A method of producing human NK cells according to the present invention comprises a step of producing human T/NK progenitor cells by the human pT/NK cell production method, and a step of culturing the produced human T/NK progenitor cells in a medium comprising IL-7, Flt3-L, SCF and IL-15 in the absence of a Notch ligand to induce differentiation into human NK cells. As described in B-2 of the Examples below, in the induction of differentiation of human T/NK progenitor cells into human NK cells, approximately 12 to 30 times as many NK cells can be produced by culturing in a medium containing the above four cytokines in the absence of a Notch ligand without using feeder cells expressing a Notch ligand, as compared with a case of culturing in the presence of a Notch ligand.

IL-7, Flt3-L, SCF, and IL-15 can be used at concentrations of about 1 to 50 ng/ml each, for example, about 5 to 30 ng/ml each. For example, IL-15 and Flt3-L may be used at about 1 to 30 ng/ml or about 5 to 20 ng/ml each, and SCF and IL-15 may be used at about 5 to 50 ng/ml or about 10 to 40 ng/ml each. By performing the above-mentioned feeder-free culture for a period of about 2 to 4 weeks while appropriately exchanging the medium, the production amount of NK cells can be greatly improved as described above.

Human NK cells obtained by the human pT/NK cell production method described above are maintained in an immature state and exhibit a characteristic surface antigen profile. The present invention also provides cultured human NK cells characterized by such surface antigens.

The cultured human NK cell provided by the present invention is characterized by being more immature and having a lower degree of exhaustion than human umbilical cord blood NK cells isolated from human umbilical cord blood, due to lower expression levels of maturity indicators CD16 and KIR (killer cell immunoglobulin-like receptor) and exhaustion marker TIGIT (T cell immunoreceptor with Ig and ITIM domains; NCBI Gene ID: 201633, NCBI Reference Sequence: NM_173799.4) compared to those of said human umbilical cord blood NK cells. In this field, human umbilical cord blood NK cells are known to be more immature in maturity compared with other human NK cells such as human peripheral blood NK cells. However, as shown in Example B-11, human NK cells induced from human T/NK progenitor cells produced by the human pT/NK cell production method of the present invention are even more immature than human umbilical cord blood NK cells. In addition, the cultured human NK cell of the present invention is also characterized by having a lower expression of the exhaustion marker TIGIT, which is an indicator of the degree of exhaustion of NK cells, than human umbilical cord blood NK cells, and thus having a lower degree of exhaustion. As described above, the cultured human NK cell of the present invention is a cell having characteristics different from those of human NK cells naturally existing in the human body. The expression levels of CD16, KIR, and TIGIT in the cultured human NK cell of the present invention may be, independently, 1/2 or less, 1/3 or less, 1/4 or less, 1/5 or less, 1/6 or less, 1/7 or less, or 1/8 or less of the expression levels in human umbilical cord blood NK cells.

KIR includes KIR2DL2 (killer cell immunoglobulin like receptor, two Ig domains and long cytoplasmic tail 2; NCBI Gene ID: 3803, NCBI Reference Sequence: NM_014219.3) and KIR2DL3 (killer cell immunoglobulin like receptor, two Ig domains and long cytoplasmic tail 3; NCBI Gene ID: 3804, NCBI Reference Sequence: NM_015868.3). The expression of KIR in the cultured human NK cell of the present invention may be the expression of KIR2DL2, the expression of KIR2DL3, or both.

The cultured human NK cell of the present invention may further be characterized by higher expression of 1, 2, 3, 4, or 5 genes selected from CCR3, CCR6, CCR7, CCR8, and CCR9 compared with human umbilical cord blood NK cells and human peripheral blood NK cells. The expression of surface antigen markers CD3, CD4, CD8, CD56, and CD161 in the cultured human NK cell of the present invention is similar to that in other human NK cells, and the cell is CD3-negative, CD4-negative, CD8-negative, CD56-positive, and CD161-positive.

The expression of these surface antigen markers can be examined by well-known methods such as flow cytometry using an antibody against each surface antigen marker, and real-time PCR using a primer/probe for detecting the expression of each surface antigen marker.

### III. Production of immune cell therapy agent for tumor or autoimmune disease

The method of producing an immune cell therapy agent for a tumor or an autoimmune disease according to the present invention includes Production Method 1 for producing CD8-positive cytotoxic TCR-T, CAR-T, or CAAR-T cells as an immune cell therapy agent, and Production Method 2 for producing CAR-NK cells, CAAR-NK cells, CAR-macrophages, or CAAR-macrophages as an immune cell therapy agent. Production Method 1 and Production Method 2 each include two types: a method of producing each receptor-expressing cell by the method of Mode A described above, and a method of producing each receptor-expressing cell by the method of Mode B described above.

### Production Method 1A

Production Method 1A is a method comprising: producing CD8-positive cytotoxic TCR-T, CAR-T, or CAAR-T cells by the production method of the TCR/CAR/CAAR-expressing immune cell according to Mode A, in which an exogenous receptor gene is introduced into multipotent blood progenitor cells followed by inducing differentiation into immune cells; and preparing the produced cells as an immune cell therapy agent for a tumor or an autoimmune disease. This method comprises the following steps 1A-1 to 1A-5.
Step 1A-1 of preparing multipotent blood progenitor cells from a cell population comprising hematopoietic stem cells.
Step 1A-2 of introducing a gene encoding said exogenous receptor into the multipotent blood progenitor cells to prepare multipotent blood progenitor cells expressing an exogenous receptor, wherein said exogenous receptor is a CAAR, or a TCR or CAR that specifically recognizes a tumor antigen.
Step 1A-3 of inducing differentiation of the multipotent blood progenitor cells expressing said exogenous receptor into T cells to prepare T cells expressing said exogenous receptor (TCR-T, CAR-T, or CAAR-T cells).
Step 1A-4 of subjecting the TCR-T, CAR-T, or CAAR-T cells to TCR stimulation to induce differentiation into CD8-positive cytotoxic TCR-T, CAR-T, or CAAR-T cells.
Step 1A-5 of preparing the CD8-positive cytotoxic TCR-T or CAR-T cells as an immune cell therapy agent for a tumor expressing said tumor antigen, or preparing the CD8-positive cytotoxic CAAR-T cells as an immune cell therapy agent for an autoimmune disease.

### Production Method 1B

Production Method 1B is a method comprising: producing CD8-positive cytotoxic TCR-T, CAR-T, or CAAR-T cells by the production method of the TCR/CAR/CAAR-expressing immune cell according to Mode B, in which multipotent blood progenitor cells are induced to differentiate into immune cells followed by introduction of an exogenous receptor gene; and preparing the produced cells as an immune cell therapy agent for a tumor or an autoimmune disease. This method comprises the following steps 1B-1 to 1B-4.
Step 1B-1 of preparing multipotent blood progenitor cells from a cell population comprising hematopoietic stem cells.
Step 1B-2 of inducing differentiation of the multipotent blood progenitor cells into T cells.
Step 1B-3 of subjecting the T cells to TCR stimulation and then introducing thereinto a gene encoding an exogenous receptor, wherein the exogenous receptor is a CAAR, or a TCR or CAR that specifically recognizes a tumor antigen, and thereby preparing CD8-positive cytotoxic TCR-T, CAR-T, or CAAR-T cells.
Step 1B-4 of preparing the CD8-positive cytotoxic TCR-T or CAR-T cells as an immune cell therapy agent for a tumor expressing said tumor antigen, or preparing the CD8-positive cytotoxic CAAR-T cells as an immune cell therapy agent for an autoimmune disease.

### Production Method 2A

Production Method 2A is a method comprising: producing CAR-NK cells, CAAR-NK cells, CAR-macrophages or CAAR-macrophages by the production method of the TCR/CAR/CAAR-expressing immune cell according to Mode A, in which an exogenous receptor gene is introduced into multipotent blood progenitor cells followed by inducing differentiation into immune cells; and preparing the produced cells as an immune cell therapy agent for a tumor or an autoimmune disease. This method comprises the following steps 2A-1 to 2A-4.
Step 2A-1 of preparing multipotent blood progenitor cells from a cell population comprising hematopoietic stem cells.
Step 2A-2 of introducing a gene encoding a chimeric receptor into the multipotent blood progenitor cells, wherein the chimeric receptor is a CAAR, or a CAR that specifically recognizes a tumor antigen, and thereby preparing multipotent blood progenitor cells expressing said chimeric receptor.
Step 2A-3 of inducing differentiation of the multipotent blood progenitor cells expressing said chimeric receptor into NK cells or macrophages to prepare NK cells (CAR-NK or CAAR-NK cells) or macrophages (CAR-macrophages or CAAR-macrophages), each expressing said chimeric receptor.
Step 2A-4 of preparing the CAR-NK cells or CAR-macrophages as an immune cell therapy agent for a tumor expressing said tumor antigen, or preparing the CAAR-NK cells or CAAR-macrophages as an immune cell therapy agent for an autoimmune disease.

Since CAR/CAAR-NK cells and CAR/CAAR-macrophages have sufficient proliferation ability even before receptor stimulation, they can be prepared as an immune cell therapy agent without receptor stimulation and used for administration to a patient. To further activate them and increase proliferation ability, a step of subjecting the prepared CAR/CAAR-NK cells or CAR/CAAR-macrophages to receptor stimulation may be performed after step 2A-3. In this case, in step 2A-4, the CAR/CAAR-NK cells or CAR/CAAR-macrophages after receptor stimulation are prepared as an immune cell therapy agent.

### Production Method 2B

Production Method 2B is a method comprising: producing CAR-NK cells, CAAR-NK cells, CAR-macrophages or CAAR-macrophages by the production method of the TCR/CAR/CAAR-expressing immune cell according to Mode B, in which multipotent blood progenitor cells are induced to differentiate into immune cells followed by introduction of an exogenous receptor gene; and preparing the produced cells as an immune cell therapy agent for a tumor or an autoimmune disease. This method comprises the following steps 2B-1 to 2B-4.
Step 2B-1 of preparing multipotent blood progenitor cells from a cell population comprising hematopoietic stem cells.
Step 2B-2 of inducing differentiation of the multipotent blood progenitor cells into NK cells or macrophages.
Step 2B-3 of subjecting the NK cells or the macrophages to receptor stimulation and then introducing thereinto a gene encoding a chimeric receptor, wherein the chimeric receptor is a CAAR, or a CAR that specifically recognizes a tumor antigen, and thereby preparing NK cells (CAR-NK or CAAR-NK cells) or macrophages (CAR-macrophages or CAAR-macrophages), each expressing said chimeric receptor.
Step 2B-4 of preparing the CAR-NK cells or CAR-macrophages as an immune cell therapy agent for a tumor expressing said tumor antigen, or preparing the CAAR-NK cells or CAAR-macrophages as an immune cell therapy agent for an autoimmune disease.

In Production Method 2B, since cells are subjected to receptor stimulation at the time of introduction of the CAR gene, the CAR/CAAR-NK cells or CAR/CAAR-macrophages obtained in step 2B-3 already have high proliferation ability, and can be preferably prepared as an immune cell therapy agent without further receptor stimulation.

Steps 1A-1 to 1A-4 of Production Method 1A, and steps 2A-1 to 2A-3 and the step of subjecting CAR/CAAR-NK cells or CAR/CAAR-macrophages to receptor stimulation of Production Method 2A, can be performed as described in I-1. to I-3.

Steps 1B-1 to 1B-3 of Production Method 1B and steps 2B-1 to 2B-3 of Production Method 2B can be performed as described in I-1., I-4. and I-5.

Step 1A-5, step 1B-4, step 2A-4, and step 2B-4, which are the final steps of each production method, are steps for preparing (formulating) cells expressing an exogenous receptor or a chimeric receptor as an immune cell therapy agent for a tumor expressing a tumor antigen that is specifically recognized by the receptor (when the receptor is a TCR or a CAR) or as an immune cell therapy agent for an autoimmune disease (when the receptor is a CAAR), respectively. Receptor-expressing cells are generally administered to a patient with a tumor or an autoimmune disease by intravenous administration, intraperitoneal administration, or intratumoral administration (in the case of a tumor patient), and thus can be formulated as an intravenous infusion, an infusion, an injection, or the like containing the cells in a vehicle suitable for these administration routes. As the vehicle, for example, an aqueous vehicle (e.g., high-purity sterile water) containing about 2.5 to 5% human serum albumin, about 0.2 to 0.5% sodium chloride, and about 3 to 8% dimethyl sulfoxide as a cryoprotectant can be used. The number of receptor-expressing cells in one dose of the formulation (e.g., one bag of intravenous infusion) can be set as appropriate depending on the type of cell, the type of tumor, etc., and can be, for example, about 1 x 10⁵ to 1 x 10⁹ as the number of viable cells.

By the production method of the present invention described above, an immune cell therapy agent for a tumor containing TCR-T cells, an immune cell therapy agent for a tumor containing CAR-T cells, an immune cell therapy agent for a tumor containing CAR-NK cells, an immune cell therapy agent for a tumor containing CAR-macrophages, an immune cell therapy agent for an autoimmune disease containing CAAR-NK cells, and an immune cell therapy agent for an autoimmune disease containing CAAR-macrophages can be produced. These immune cell therapy agents can each be used alone for the treatment of a tumor or an autoimmune disease. Or, an immune cell therapy agent for a tumor containing CAR-macrophages can be administered to a tumor patient in combination with one or more immune cell therapy agents containing TCR-T cells, CAR-T cells or CAR-NK cells. When multiple immune cell therapy agents are combined, the exogenous receptors (in this case, the exogenous receptors are antigen-specific receptors) expressed by the immune cells of each immune cell therapy agent may all be receptors that recognize the same tumor antigen, or some or all of the receptors may be those that recognize different tumor antigens. The term "administered in combination" means that multiple agents are administered to a patient simultaneously, sequentially, or separately. Sequential administration means that the administration of the next agent is continued immediately after the completion of the administration of one agent. Separate administration means that multiple agents are administered at intervals, for example, at intervals of about several hours or more in the same day, or on different days during one cycle of the treatment period. When multiple immune cell therapy agents are administered in combination, they are preferably administered sequentially or separately to the patient.

According to the present invention, an immune cell therapy agent effective against various tumors expressing a tumor antigen, as well as an immune cell therapy agent effective against an autoimmune disease for which a self-antigen has been identified, can be produced. Examples of tumors expressing a tumor antigen are as described above, and an immune cell therapy agent can be produced for various solid cancers including neuroblastoma, melanoma, esophageal cancer, lung cancer such as non-small cell lung cancer, head and neck cancer, bladder cancer, colorectal cancer, synovial sarcoma, breast cancer, ovarian cancer and pancreatic cancer, as well as various blood cancers including leukemia. Examples of autoimmune diseases and self-antigens are also as described above, and an immune cell therapy agent can be produced for various autoimmune diseases including pemphigus vulgaris and myasthenia gravis. Furthermore, as described above, according to the present invention, an immune cell therapy agent effective against an infectious disease can also be produced.

Patients to be treated with the immune cell therapy agent are mainly humans, but various animals suffering from a tumor, including other mammals, can be targets.

Regarding the treatment of a tumor or an autoimmune disease using these immune cell therapy agents (various TCR/CAR/CAAR-expressing immune cells or multipotent blood progenitor cells), it will be further explained in "III.Treatment of tumor or autoimmune disease" below.

### IV. Treatment of tumor or autoimmune disease

The treatment method for a tumor or an autoimmune disease according to the present invention is a method of treating a tumor or an autoimmune disease using the immune cell therapy agent that can be produced as described above, and includes the following modes.

### Treatment Method 1a

Treatment Method 1a is a method of treating a tumor or an autoimmune disease, comprising: producing CD8-positive cytotoxic TCR-T, CAR-T, or CAAR-T cells by the production method of the TCR/CAR/CAAR-expressing immune cell according to Mode A, in which an exogenous receptor gene is introduced into multipotent blood progenitor cells followed by inducing differentiation into immune cells; and administering the produced cells to a patient. This method comprises the following steps 1a-1 to 1a-5.
Step 1a-1 of preparing multipotent blood progenitor cells from a cell population comprising hematopoietic stem cells.
Step 1a-2 of introducing a gene encoding an exogenous receptor into the multipotent blood progenitor cells to prepare multipotent blood progenitor cells expressing the exogenous receptor, wherein the exogenous receptor is a CAAR, or a TCR or CAR that specifically recognizes a tumor antigen.
Step 1a-3 of inducing differentiation of the multipotent blood progenitor cells expressing said exogenous receptor into T cells to prepare TCR-T, CAR-T, or CAAR-T cells that are T cells expressing said exogenous receptor.
Step 1a-4 of subjecting the TCR-T, CAR-T, or CAAR-T cells to TCR stimulation to induce differentiation into CD8-positive cytotoxic TCR-T, CAR-T, or CAAR-T cells.
Step 1a-5 of administering the CD8-positive cytotoxic TCR-T or CAR-T cells to a patient having a tumor expressing said tumor antigen, or administering the CD8-positive cytotoxic CAAR-T cells to a patient with an autoimmune disease.

### Treatment Method 1b

Treatment Method 1b is a method of treating a tumor or an autoimmune disease, comprising: producing CD8-positive cytotoxic TCR-T, CAR-T, or CAAR-T cells by the production method of the TCR/CAR/CAAR-expressing immune cell according to Mode B, in which multipotent blood progenitor cells are induced to differentiate into immune cells followed by introduction of an exogenous receptor; and administering the produced cells to a patient. This method comprises the following steps 1b-1 to 1b-4.
Step 1b-1 of preparing multipotent blood progenitor cells from a cell population comprising hematopoietic stem cells.
Step 1b-2 of inducing differentiation of the multipotent blood progenitor cells into T cells.
Step 1b-3 of subjecting the T cells to TCR stimulation and then introducing thereinto a gene encoding an exogenous receptor, wherein the exogenous receptor is a CAAR, or a TCR or CAR that specifically recognizes a tumor antigen, and thereby preparing CD8-positive cytotoxic TCR-T, CAR-T, or CAAR-T cells.
Step 1b-4 of administering the CD8-positive cytotoxic TCR-T or CAR-T cells to a patient having a tumor expressing said tumor antigen, or administering the CD8-positive cytotoxic CAAR-T cells to a patient with an autoimmune disease.

### Treatment Method 2a

Treatment Method 2a is a method of treating a tumor or an autoimmune disease, comprising: producing CAR-NK cells, CAAR-NK cells, CAR-macrophages, or CAAR-macrophages by the production method of the TCR/CAR/CAAR-expressing immune cell according to Mode A, in which an exogenous receptor gene is introduced into multipotent blood progenitor cells followed by inducing differentiation into immune cells; and administering the produced cells to a patient. This method comprises the following steps 2a-1 to 2a-4.
Step 2a-1 of preparing multipotent blood progenitor cells from a cell population comprising hematopoietic stem cells.
Step 2a-2 of introducing a gene encoding a chimeric receptor into the multipotent blood progenitor cells to prepare multipotent blood progenitor cells expressing the chimeric receptor, wherein the chimeric receptor is a CAAR, or a CAR that specifically recognizes a tumor antigen.
Step 2a-3 of inducing differentiation of the multipotent blood progenitor cells expressing said chimeric receptor into NK cells or macrophages to prepare chimeric receptor-expressing immune cells that are CAR-NK cells, CAAR-NK cells, CAR-macrophages or CAAR-macrophages.
Step 2a-4 of administering the CAR-NK cells or CAR-macrophages to a patient having a tumor expressing said tumor antigen, or administering the CAAR-NK cells or CAAR-macrophages to a patient with an autoimmune disease.

As explained in Production Method 2A in III., since CAR-NK cells, CAAR-NK cells, CAR-macrophages, and CAAR-macrophages have sufficient proliferation ability even before receptor stimulation, these chimeric receptor-expressing immune cells can be administered to a patient without receptor stimulation. Or, after step 2a-3, the prepared chimeric receptor-expressing immune cells may be subjected to receptor stimulation to further increase proliferation ability, and then administered to a patient.

### Treatment Method 2b

Treatment Method 2b is a method of treating a tumor or an autoimmune disease, comprising: producing CAR-NK cells, CAAR-NK cells, CAR-macrophages, or CAAR-macrophages by the production method of the TCR/CAR/CAAR-expressing immune cell according to Mode B, in which multipotent blood progenitor cells are induced to differentiate into immune cells followed by introduction of an exogenous receptor gene; and administering the produced cells to a patient. This method comprises the following steps 2b-1 to 2b-4.
Step 2b-1 of preparing multipotent blood progenitor cells from a cell population comprising hematopoietic stem cells.
Step 2b-2 of inducing differentiation of the multipotent blood progenitor cells into NK cells or macrophages.
Step 2b-3 of subjecting the NK cells or macrophages to receptor stimulation and then introducing thereinto a gene encoding a chimeric receptor, wherein the chimeric receptor is a CAAR, or a CAR that specifically recognizes a tumor antigen, and thereby preparing CAR-NK cells, CAAR-NK, CAR-macrophages or CAAR-macrophages.
Step 2b-4 of administering the CAR-NK cells or CAR-macrophages to a patient having a tumor expressing said tumor antigen, or administering the CAAR-NK cells or CAAR-macrophages to a patient with an autoimmune disease.

### Treatment Method 3

Treatment Method 3 is a method of treating a tumor by administering CAR-macrophages and CAR-T cells in combination to a patient, and comprises the following steps 3-1 to 3-3.
Step 3-1 of preparing CAR-macrophages by the following (1) or (2):
   (1) preparing multipotent blood progenitor cells from a cell population comprising hematopoietic stem cells, introducing a gene encoding a CAR that specifically recognizes a tumor antigen into the multipotent blood progenitor cells to thereby prepare multipotent blood progenitor cells expressing said CAR, and then inducing differentiation of the CAR-expressing multipotent blood progenitor cells into macrophages;
   (2) preparing induced leukocyte stem cells or T/NK progenitor cells from a cell population comprising hematopoietic stem cells, inducing differentiation thereof into macrophages, subjecting the macrophages to receptor stimulation, and then introducing thereinto a gene encoding said CAR.
Step 3-2 of preparing CAR-T cells by the following (3) or (4):
   (3) preparing multipotent blood progenitor cells from a cell population comprising hematopoietic stem cells, introducing a gene encoding said CAR into the multipotent blood progenitor cells to thereby prepare multipotent blood progenitor cells expressing said CAR, inducing differentiation of the CAR-expressing multipotent blood progenitor cells into T cells, and then subjecting the T cells to TCR stimulation to thereby induce differentiation into CD8-positive cytotoxic TCR-T or CAR-T cells;
   (4) preparing induced leukocyte stem cells or T/NK progenitor cells from a cell population comprising hematopoietic stem cells, inducing differentiation thereof into T cells, subjecting the T cells to TCR stimulation, and then introducing thereinto a gene encoding said CAR.
Step 3-3 of administering the CAR-macrophages and the CAR-T cells in combination to a patient having a tumor expressing said tumor antigen.

### Treatment Method 4

Treatment Method 4 is a method of treating a tumor by administering CAR-macrophages and CAR-NK cells in combination to a patient, and comprises the following steps 4-1 to 4-3.
Step 4-1 of preparing CAR-macrophages by the following step (1) or (2):
   (1) preparing multipotent blood progenitor cells from a cell population comprising hematopoietic stem cells, introducing a gene encoding a CAR that specifically recognizes a tumor antigen into the multipotent blood progenitor cells to thereby prepare multipotent blood progenitor cells expressing said CAR, and then inducing differentiation of the CAR-expressing multipotent blood progenitor cells into macrophages;
   (2) preparing induced leukocyte stem cells or T/NK progenitor cells from a cell population comprising hematopoietic stem cells, inducing differentiation thereof into macrophages, subjecting the macrophages to receptor stimulation, and then introducing thereinto a gene encoding said CAR.
Step 4-2 of preparing CAR-NK cells by the following step (5) or (6):
   (5) preparing multipotent blood progenitor cells from a cell population comprising hematopoietic stem cells, introducing a gene encoding said CAR into the multipotent blood progenitor cells to thereby prepare multipotent blood progenitor cells expressing the CAR, and then inducing differentiation of the CAR-expressing multipotent blood progenitor cells into NK cells;
   (6) preparing induced leukocyte stem cells or T/NK progenitor cells from a cell population comprising hematopoietic stem cells, inducing differentiation thereof into NK cells, subjecting the NK cells to receptor stimulation, and then introducing thereinto a gene encoding said CAR.
Step 4-3 of administering the CAR-macrophages and the CAR-NK cells in combination to a patient having a tumor expressing said tumor antigen.

As explained in Production Method 2A in III., since CAR-NK cells and CAR-macrophages have sufficient proliferation ability even before receptor stimulation, receptor stimulation is not essential even in step 3-1(1), step 4-1(1), and step 4-2(5). Or, in step 3-1(1), step 4-1(1), and step 4-2(5), the prepared CAR-NK cells and CAR-macrophages may be subjected to receptor stimulation to further increase proliferation ability, and then administered to a patient.

Steps 1a-1 to 1a-4 of Treatment Method 1a, steps 2a-1 to 2a-3 of Treatment Method 2a, steps 3-1(1) and 3-2(3) of Treatment Method 3, steps 4-1(1) and 4-2(5) of Treatment Method 4, and the step of subjecting CAR-NK cells, CAAR-NK cells, CAR-macrophages or CAAR-macrophages to receptor stimulation optionally performed in Treatment Method 2a, Treatment Method 3 and Treatment Method 4 can be performed as described in I-1. to I-3.

Steps 1b-1 to 1b-3 of Treatment Method 1b, steps 2b-1 to 2b-3 of Treatment Method 2b, steps 3-1(2) and 3-2(4) of Treatment Method 3, and steps 4-1(2) and 4-2(6) of Treatment Method 4 can be performed as described in I-1., I-4., and I-5.

In the treatment methods of each of the above modes, the multipotent blood progenitor cells, induced leukocyte stem cells, or T/NK progenitor cells may be prepared from umbilical cord blood (usually not derived from the patient themselves), or a cell population comprising hematopoietic stem cells may be obtained from bone marrow fluid or peripheral blood collected from a patient to prepare multipotent blood progenitor cells, induced leukocyte stem cells, or T/NK progenitor cells derived from the patient's own cells.

In step 1a-5, step 1b-4, step 2a-4, step 2b-4, step 3-3, and step 4-3, the immune cells expressing an exogenous receptor are prepared in an appropriate dosage form such as an intravenous infusion as explained in III., and administered to a tumor patient or an autoimmune disease patient. A tumor patient to whom the immune cells are administered is a patient having a tumor expressing a tumor antigen recognized by the exogenous receptor (in this case, the receptor is an antigen-specific receptor) that has been made to be expressed in the immune cells. In addition, an autoimmune disease patient to whom the immune cells are administered is a patient having an autoimmune disease caused by a self-antigen employed in the antigen-derived portion of the CAAR that has been made to be expressed in the immune cells, or caused by pathogenic B cells that recognize the self-antigen. Administration to a patient may be performed once, or may be performed twice or more at appropriate intervals. In step 3-3 and step 4-3, the CAR-macrophages and CAR-T/CAR-NK cells may each be administered once, or may each be administered twice or more at appropriate intervals. The meaning of the term "administered in combination" is as explained in III. The dose of each immune cells may vary depending on the type of cell to be administered, the type of tumor, the body weight of the patient, and the like, and about 1 x 10⁵ to 1 x 10⁹ cells as the number of viable cells may be administered in one administration.

In conventional TCR-T/CAR-T/CAR-NK therapy, TCR-T/CAR-T/CAR-NK cells are administered after reducing lymphocytes in a patient's body by anticancer drug treatment or the like. In the method of treating a tumor according to the present invention, a pre-treatment for reducing lymphocytes in a patient's body may also be performed prior to administration of immune cells expressing a tumor antigen-specific TCR or CAR.

A tumor to be treated is a tumor expressing a tumor antigen recognized by an exogenous receptor (in this case, an antigen-specific receptor). In addition, an autoimmune disease to be treated is an autoimmune disease caused by a self-antigen employed in the antigen-derived portion of a CAAR, or by pathogenic B cells recognizing the self-antigen. Specific examples of tumors and autoimmune diseases are as listed in I-2. along with specific examples of tumor antigens and self-antigens.

### EXAMPLES

Hereinafter, the present invention will be described more concretely based on Examples. However, the present invention is not limited to the following Examples.

### A. Production of immune cells expressing TCR or CAR

### A-1. Experimental materials

### Medium

The media used were Roswell Park Memorial Institute (RPMI)-1640 (Sigma-Aldrich), Isocove's Modified Dulbecco's Medium (IMDM; Sigma-Aldrich), Minimum Essential Medium alpha (MEMα; Invitrogen), and Stemline II (Sigma-Aldrich). In all media, 100 µg/mL penicillin and 100 U/mL streptomycin were added. In addition, 10% fetal bovine serum (FBS; Gibco) was added to RPMI-1640 and IMDM, and 20% FBS was added to MEMα. A medium obtained by adding 1% MEM Non-Essential Amino Acid Solution (Gibco) and 1 mM Sodium Pyruvate (Gibco) to RPMI-1640 was used as RPMI complete (RPMIcomp).

### Cytokines

Recombinant human (rh) interleukin-2 (IL-2), IL-7, IL-15, FMS-related tyrosine kinase 3 ligand (Flt3-L), Stem cell factor (SCF), thrombopoietin (TPO), and Granulocyte-colony stimulating factor (G-CSF), and recombinant mouse (rm) IL-3, IL-6, IL-7, IL-15, SCF, Flt3-L, TPO, and Macrophage-stimulating factor (M-CSF) were purchased from R&D Systems.

### Preparation of retrovirus

As the ID3-ERT2 retroviral vector, one previously reported (Non-Patent Document 3) was used. This retroviral vector is a vector constructed by cloning an ID3-ERT2 gene, in which a mutant human estrogen receptor ligand-binding domain (ERT2) sequence is fused to the full-length human ID3 gene cDNA, into a known pCSRet retroviral vector. In the base sequence of the ID3-ERT2 gene shown in SEQ ID NO: 1, positions 1 to 357 are the human ID3 gene cDNA, and positions 370 to 1311 are the ERT2 mutant ligand-binding domain sequence.

A plasmid (murine TCR OTI-2A.pMIG II) encoding an OTI-TCR gene (a gene for a T-cell receptor that specifically reacts with an OVA antigen, top of FIG. 2, SEQ ID NO: 17) was purchased from addgene. In the base sequence of the OTI-TCR gene shown in SEQ ID NO: 17, positions 1 to 828 (residues 1 to 276 in the amino acid sequence of SEQ ID NO: 18) are the OTI TCR α chain sequence, positions 829 to 882 (residues 277 to 294) are the T2A self-cleaving peptide sequence, and positions 883 to 1821 (residues 295 to 607) are the OTI TCR β chain sequence.

A pMIGII-hCD19-m28z-CAR plasmid carrying an hCD19-m28z-CAR gene was constructed by fusing a 3xFlag gene, a human CD19-specific FMC63 antibody single-chain variable region fragment gene, a mouse CD28 cell membrane domain and intracellular signaling domain, and a mouse CD3ε intracellular signaling domaingene downstream of a mouse IgG2aκ signal peptide gene (FIG. 10B). In the base sequence of the hCD19-m28z-CAR gene shown in SEQ ID NO: 3, positions 1 to 60 are the mouse IgG2aκ signal peptide sequence, positions 61 to 126 are the 3xFlag gene, positions 127 to 870 are the human CD19-specific FMC63 antibody single-chain variable region fragment gene, positions 871 to 312 are the mouse CD28 cell membrane domain and intracellular signaling domain gene, and positions 1183 to 1521 are the mouse CD3ε intracellular signaling domain gene sequence.

Retroviruses containing these genes were produced by introducing the target plasmid and a VSV-G plasmid into Plat E cells (Cell Biolabs Inc.) using FuGENE 6 Transfection Reagent (Roche).

### Preparation of lentiviruses

As the lentiviral vector, CSII vector was used. Lentiviruses expressing a target gene were produced by introducing a plasmid encoding the target gene such as a CAR gene, a Gag/Pol plasmid, a Rev plasmid, and a VSV-G or GALV envelope plasmid into 293T cells using FuGENE 6 Transfection Reagent (Roche). As the GALV envelope plasmid, commercially available phCMV-GALV-MTR containing a synthetic gene GALV-MTR (a gene encoding an envelope in which the corresponding regions of the GALV envelope were replaced with a membrane domain and a cytoplasmic domain derived from Mouse Leukemia virus (MuLV), SEQ ID NO: 15) was used.

### A-2. Experiments and Results

### A-2-1. Production of iLS cells

Bone marrow fluid was collected from C57BL/6 mice, and LSK cells negative for Lineage (CD90, B220, Gr-1, CD11b, Ter119) and positive for c-Kit and Sca-I were isolated by a cell sorter (FACS Aria III). LSK cells were seeded on OP9 feeder cells and cultured in RPMIcomp medium supplemented with 30 ng/mL rmIL-7, 30 ng/mL rmFlt3-L, 30 ng/mL rmSCF, and 30 ng/mL rmTPO in an environment of 37°C and 5% CO₂. After 4-6 days, cells were collected and transduced with the ID3-ERT2 gene using a retrovirus by a spin infection method. To enable identification and isolation of ID3-ERT2 gene-transduced cells, human CD25 was utilized as a marker for gene transduction, and the human CD25 gene was linked to the ID3-ERT2 gene and transduced into the cells. ID3-ERT2 gene-transduced cells positive for human CD25 were isolated by a cell sorter. Sorted iLS cells were cultured on OP9 cells in an iLS cell medium (IMDM supplemented with 10 ng/mL rmIL-7, 10 ng/mL rmFlt3-L, 10 ng/mL rmSCF, and 40 nM 4-hydroxytamoxifen (4-OHT)). iLS cells were expanded by subculturing on fresh OP9 cells every 3-4 days. About one month later, iLS cells proliferating stably were confirmed by FCM analysis and used in subsequent experiments (FIG. 1).

### A-2-2. Gene transduction of cancer-specific receptors into iLS cells

To produce iLS cells stably expressing a cancer-specific receptor, iLS cells were transduced with an OTI-TCR gene or an hCD19-m28z-CAR gene using a retrovirus by a spin infection method. After gene transduction, iLS cells positive for GFP, which is a marker protein, were isolated by a cell sorter. Isolated OTI-TCR gene-transduced iLS (OTI TCR-iLS) cells and hCD19-m28z-CAR gene-transduced iLS (CD19-CAR iLS) cells were cultured under iLS cell culture conditions and maintained and expanded (FIG. 2, FIG. 10).

### A-2-3. T-cell induction from cancer-specific receptor gene-transduced iLS cells

OTI TCR-iLS cells or CD19-CAR iLS cells were seeded on OP9/Delta-like 1 (OP9/DLL1) cells and cultured in MEMα supplemented with 5 ng/mL rmIL-7, 5 ng/mL rmFlt3-L, and 40 nM 4-OHT. Every 3-4 days, half of the medium was exchanged with the above medium excluding 4-OHT, and one week later, the iLS cells were seeded on fresh OP9/DLL1 cells. After culturing for 2 weeks, the cells were cultured in MEMα containing 1 ng/mL rmIL-7 and 1 ng/mL rmFlt3-L. Cells were collected 3 weeks after the start of induction, and CD4/CD8 double-positive (DP) cells were isolated by CD8a (Ly-2) MicroBeads (Miltenyi Biotech) (FIGS. 3, 4, and 6).

To subject DP cells generated from OTI TCR-iLS cells to TCR stimulation, spleen cells were collected from C57BL/6 mice, treated with 10 µg/mL mitomycin C (MMC) for 2.5 hours, and used as antigen-presenting cells. DP cells derived from OTI TCR-iLS cells and MMC-treated antigen-presenting cells were mixed at a ratio of 2:1, and TCR stimulation was performed by co-culturing in RPMIcomp medium containing 5 ng/mL rmIL-7, 5 ng/mL rmIL-15, 50 IU/mL rhIL-2, and 10 µg/mL OVA peptide (OVA₂₅₇₋₂₆₄), to thereby generate CD8 T cells (FIG. 5).

On the other hand, DP cells generated from CD19-CAR iLS cells were subjected to TCR stimulation by culturing on plates coated with anti-CD3 and anti-CD28 antibodies in RPMIcomp medium supplemented with 5 ng/mL rmIL-7, 5 ng/mL rmIL-15, and 50 IU/mL rhIL-2. Cells were collected one week after the TCR stimulation and further cultured for 4 days in RPMIcomp medium supplemented with 5 ng/mL rmIL-7, 5 ng/mL rmIL-15, and 50 IU/mL rhIL-2 in the absence of TCR stimulation. The cells were analyzed by FCM analysis, and it was confirmed that CD8-positive CAR-T cells were generated (FIG. 6).

### A-2-4. Evaluation of antigen-specific cytokine release of CD8-positive T cells derived from OTI TCR-iLS cells

OVA antigen-presenting cells were obtained by culturing spleen cells collected from C57BL/6 mice with 1 µg/mL OVA₂₅₇₋₂₆₄ for 1 hour. CD8-positive T cells derived from OTI TCR-iLS cells were mixed with a 10-fold amount of OVA antigen-presenting cells, or treated with 10 ng/mL PMA and 1 µg/mL ionomycin, and cultured in RPMIcomp medium supplemented with a 1/1000 amount of PE-Cy7-conjugated CD107a antibody and 1x Protein transport inhibitor (eBioscience). After 6 hours, intracellular staining was performed and expression levels of cytokines were evaluated by FCM analysis. As a result, iLS cell-derived OTI TCR-T cells were activated by stimulation, and expression of CD107a, IFNγ, and TNFa was observed (FIG. 7).

### A-2-5. Cytotoxic activity of CD8-positive T cells derived from OTI TCR-iLS cells

B16 cells stably expressing OVA antigen (B16-OVA) or B16 cells expressing only neomycin as a selection marker (B16-Neo), which were treated overnight with 500 ng/mL IFNγ, were stained with Cell Tracer Far-Red (Invitrogen). Thereafter, the cells were mixed with CD8-positive T cells derived from OTI TCR-iLS cells or CD8 T cells from the spleen of OTI Tg mice (transgenic mice expressing the OTI-TCR gene) at different ratios, and co-cultured for 18 hours in RPMIcomp medium supplemented with 50 IU/mL rhIL-2. After co-culturing, dead cells were stained with Live/Dead Near-IR (Invitrogen), and the ratio of dead cells was measured by FCM analysis. As a result, iLS cell-derived OTI TCR-T cells showed high cytotoxic activity similar to OTI Tg mouse spleen-derived T cells (FIG. 8).

### A-2-6. In vivo anti-tumor activity of CD8-positive T cells derived from OTI TCR-iLS cells

B16-OVA tumor-bearing mice were produced by subcutaneously transplanting 1x10⁵ B16-OVA cells into C57BL/6 mice. Five days after transplantation, the tumor-bearing mice were irradiated with X-rays at 4 Gy, and then 3x10⁶ OTI TCR T-iLS cells were administered via the tail vein. For 3 days from the T-cell transplantation day, 50,000 IU rhIL-2 was intraperitoneally administered once every day. Tumor diameters were measured over time to evaluate the anti-tumor activity of the TCR-T cells. Tumor volume was calculated as (short side)² x (long side) x 0.5. As a result, iLS-derived OTI TCR-T cells showed anti-tumor activity equivalent to OTI Tg mouse-derived T cells (FIG. 9).

### A-2-7. Induction of differentiation from CD19-CAR iLS cells into NK cells

CD19-CAR iLS cells were seeded on OP9 cells and cultured for 10 days in RPMIcomp medium supplemented with 10 ng/mL rmIL-15 and 100 IU/mL rhIL-2. As a result, CAR-NK cells were generated at a high ratio (FIG. 10).

### A-2-8. Cytotoxic activity of iLS cell-derived CD19-CAR-NK cells

CD19-CAR-NK cells and a B16 melanoma cell line expressing human CD19 were mixed at various cell ratios and co-cultured for 4 hours. As a result, it was revealed that they showed higher cytotoxic activity compared with a B16 melanoma cell line not expressing human CD19 (FIG. 11).

### A-2-9. Induction of differentiation from CD19-CAR iLS cells into macrophages

CD19-CAR iLS cells were seeded on OP9 cells and cultured in IMDM supplemented with 10 ng/mL rmSCF, 5 ng/mL rmM-CSF, 5 ng/mL rmIL-3, and 5 ng/mL rmIL-6. Four days later, half of the medium was exchanged. After culturing for one week from the start of induction, FCM analysis was performed, and the generation of CD11b-positive, F4/80-positive, MHC class II (I-A)-positive macrophages was confirmed (FIG. 12). Thus, it was revealed that CAR iLS cells have the ability to generate CAR-macrophages.

### A-2-10. Production of pT/NK from human umbilical cord blood CD34-positive cells

hCB-CD34-positive cells were obtained from RIKEN BioResource Research Center. hCB-CD34-positive cells were expanded by culturing for one week in Stemline II medium supplemented with 100 ng/mL rhSCF, 100 ng/mL rhTPO, and 100 ng/mL rhG-CSF. These cells were seeded on OP9/DLL1 cells and cultured in MEMα supplemented with 5 ng/mL rhIL-7, 5 ng/mL rhFlt3-L, and 5 ng/mL rhSCF. As a result, pT/NK cells (CD5⁺CD7⁺ cells) were generated on day 17 of culture (FIG. 13 bottom left).

### A-2-11. Induction of differentiation from pT/NK cells into NK cells

pT/NK cells were seeded on OP9/DLL1 cells and cultured in MEMα supplemented with 20 ng/mL rhIL-7, 10 ng/mL rhFlt3-L, 20 ng/mL rhSCF, and 10 ng/mL rhIL-15, thereby inducing differentiation into NK cells. The efficiency of differentiation into NK cells was evaluated by FCM analysis. For comparison, hCB-HSPCs after expansion in Stemline II medium were seeded on OP9/DLL1 cells and induced into NK cells by culturing with the addition of 20 ng/mL rhIL-7, 10 ng/mL rhFlt3-L, 20 ng/mL rhSCF, and 10 ng/mL rhIL-15. As a result, it was revealed that pT/NK cells generated 17-fold more NK cells than hCB-HSPCs (FIG. 13 bottom right).

### A-2-12. Cytokine release assay of pT/NK cell-derived NK cells

To evaluate the cytokine production ability of NK cells derived from pT/NK cells, the NK cells were mixed with a leukemia cell line K562 cell line (K562 cells), which are target cells, or treated with 10 ng/mL PMA and 1 µg/mL ionomycin, and cultured in MEMα medium containing a 1/500 amount of APC-conjugated CD107a antibody and 1x Protein transport inhibitor. After 4 hours, intracellular staining was performed and FCM analysis of cytokines was conducted, revealing that the NK cells were activated by the target cells (FIG. 14).

### A-2-13. Cytotoxic activity of pT/NK cell-derived NK cells

pT/NK cell-derived NK cells or hCB-HSPC-derived NK cells were mixed with K562 cells at various cell ratios and co-cultured for 4 hours. As a result, it was revealed that the pT/NK cell-derived NK cells showed high cytotoxic activity comparable to the hCB-HSPC-derived NK cells (FIG. 15).

### A-2-14. Gene transduction into pT/NK cell-derived NK cells

CAR-NK cells were produced by transducing NK cells derived from pT/NK cells with a CAR gene. As the CAR gene, an anti-CD19 CAR gene (SEQ ID NOs: 11 and 12) that specifically recognizes human CD19 and an anti-GD2 CAR gene (SEQ ID NOs: 13 and 14) encoding a CAR that specifically recognizes the neuroblastoma antigen GD2, both constructed using human proteins for the intracellular domains, were used. In the anti-CD19 CAR gene sequence of SEQ ID NO: 11, positions 1-63 (1-21 AA of SEQ ID NO: 12) are a CD8α signal peptide sequence, positions 64-129 (22-43 AA) are a 3xFlag gene, positions 130-864 (44-288 AA) are an anti-CD19 scFv (antibody Clone FMC63) gene, positions 874-1014 (292-338 AA) are a human CD8α hinge region gene, positions 1024-1119 (342-373 AA) are a human CD28 transmembrane domain gene, positions 1120-1245 (374-415 AA) are a human 4-1BB intracellular signaling domain gene, and positions 1252-1593 (418-531 AA) are a human CD3ζ intracellular signaling domain gene. In the anti-GD2 CAR gene sequence of SEQ ID NO: 13, positions 130-867 (44-289 AA of SEQ ID NO: 14) are an anti-GD2 scFv (antibody Clone 14g2a) gene, and the sequences upstream and downstream thereof are identical to those in SEQ ID NO: 11 (positions 1-63 (1-21 AA) are a CD8α signal peptide sequence, positions 64-129 (22-43 AA) are a 3xFlag sequence, positions 877-1017 (293-339 AA) are a human CD8α hinge region, positions 1027-1122 (343-374 AA) are a human CD28 transmembrane domain, positions 1123-1248 (375-416 AA) are a human 4-1BB intracellular signaling domain, and positions 1255-1596 (419-532 AA) are a human CD3ζ intracellular signaling domain).

K562 (K562/memIL-21/41BBL) cells stably expressing membrane-bound IL-21 (memIL-21) and 4-1BB ligand (41BBL) were treated with 10 µg/mL mitomycin C for 2.5 hours. Mitomycin C-treated K562/memIL-21/41BBL cells and pT/NK cell-derived NK cells are mixed at a ratio of 2:1 and cultured in MEMα medium supplemented with 200 IU/mL rhIL-2 to expand NK cells. After culturing for one week, the NK cells were cultured for 2 days in MEMα medium containing 10 ng/mL rhIL-15, 10 ng/mL rhIL-21, and 100 IU/mL rhIL-2, and the cells were transduced with the anti-CD19 or anti-GD2 CAR gene using a lentivirus by a spin infection method. CAR-NK cells positive for the marker protein Venus were isolated by a cell sorter, and then co-cultured with K562/memIL-21/41BBL cells to further expand them. After expansion, it was confirmed by performing FCM analysis for the expression of Venus that the expression of each transgene was maintained (FIG. 16A).

### A-2-15. Cytotoxic activity of pT/NK cell-derived anti-CD19 CAR-NK cells

First, NALM6 cells, which are a B-cell leukemia line and express CD19 antigen, were stained with Cell Tracer Far-Red (Invitrogen). Next, pT/NK cell-derived anti-CD19 CAR-NK cells were mixed with NALM6 cells at a ratio of 1:1 and co-cultured for 4 hours. After co-culturing, dead cells were stained with Live/Dead Near-IR (Invitrogen), and the ratio of dead cells was measured using FCM analysis, revealing that the CAR-NK cells showed cytotoxic activity in an antigen-specific manner (FIG. 16B).

### A-2-16. CAR gene transduction into pT/NK cells

FIG. 17 shows the results of comparing gene transduction efficiency into pT/NK cells using lentiviruses with a VSV-G or GALV envelope (a gene encoding only Venus was used). The transduction efficiency was increased by using the GALV envelope.

A lentivirus encoding a CAR gene produced using the GALV envelope (SEQ ID NO: 16) was used for gene transduction into the generated pT/NK cells. The lentivirus was seeded on a plate coated with RetroNectin and centrifuged at 2000 xg for 1.5 hours to bind the lentivirus to the RetroNectin, and then pT/NK cells were seeded. After centrifugation at 800 xg for 10 minutes, the cells were cultured in an incubator. Cells transduced with the CAR gene were sorted by a cell sorter to obtain CAR gene-transduced pT/NK cells (CAR-pT/NK cells) (FIG. 18A). The obtained CAR-pT/NK cells were cultured under NK cell induction conditions to thereby obtain pT/NK cell-derived NK cells stably expressing the CAR gene (FIG. 18B).

### B. Study of human pT/NK cell expansion method and method for inducing differentiation from human pT/NK cells into NK cells

### B-1. Study of cytokine concentrations for human pT/NK cell induction

Optimal cytokine concentrations for generating and expanding pT/NK cells from hCB-CD34 positive cells were studied. HSPCs (a cell population obtained by expanding CD34 positive cells) derived from hCB-CD34 positive cells obtained from RIKEN BioResource Research Center were cultured on OP9/DLL1 feeder cells (in the presence of Notch signaling) for 6 weeks with the addition of recombinant human (rh) IL-7, rhFlt3-L, and rhSCF at the cytokine concentrations shown in Table 1. After culture, the number of produced pT/NK cells was evaluated by FCM analysis.

From the studies so far, it has been confirmed that the expansion efficiency of mouse pT/NK cells is significantly increased by increasing the cytokine concentration to about 25 ng/ml to 50 ng/ml, rather than setting it to 10 ng/ml as reported in Non-Patent Document 2. FIG. 19 shows the results of counting the number of pT/NK cells after culturing mouse HSPCs (Lineage-Sca1-ckit+: LSK) on OP9/DLL1 feeder cells for 2 weeks with the addition of SCF, IL-7, and Flt3-L to the medium at concentrations of 10 ng/ml or 30 ng/ml each. Even at 10 ng/ml, a large amount of pT/NK cells, about 10,000 cells per HSPC, are generated. However, when the concentration of each cytokine is increased to 30 ng/ml, the number of pT/NK cells generated from one HSPC is about 60,000, showing even more significant expansion.

On the other hand, in the case of human pT/NK cells, unlike mouse pT/NK cells, as shown in FIG. 20, human pT/NK cells were not expanded when high concentrations (≥ 25 ng/ml) of cytokines were added. It was revealed that human pT/NK cells can be most efficiently expanded when rhIL-7, rhFlt3-L, and rhSCF are each added at 5 ng/ml. In the above A-2-10, each cytokine was used at 5 ng/ml based on this study result.

**[Table 1]**

| Condition | IL-7 (ng/mL) | Fit3-L (ng/mL) | SCF (ng/mL) |
|---|---|---|---|
| 1 | 50 | 50 | 50 |
| 2 | 25 | 25 | 25 |
| 3 | 10 | 10 | 10 |
| 4 | 5 | 5 | 5 |
| 5 | 50 | 5 | 5 |
| 6 | 25 | 5 | 5 |
| 7 | 10 | 5 | 5 |
| 8 | 5 | 50 | 5 |
| 9 | 5 | 25 | 5 |
| 10 | 5 | 10 | 5 |
| 11 | 5 | 5 | 50 |
| 12 | 5 | 5 | 25 |
| 13 | 5 | 5 | 10 |

### B-2. Large-scale production of NK cells using human pT/NK cell expansion method

The number of NK cells generated using pT/NK cells was evaluated. The culture protocol is shown in FIG. 21A. First, in order to expand hCB-HSPCs, CD34 positive cells were cultured for 1 week using Stemline II medium supplemented with 100 ng/ml rhTPO, 100 ng/ml rhSCF, and 100 ng/ml rhG-CSF. The expanded HSPCs were cultured on OP9/DLL1 cells in MEMα medium containing 5 ng/ml rhIL-7, 5 ng/ml rhFlt3-L, and 5 ng/ml rhSCF, to thereby generate and expand pT/NK cells. After 4 weeks of culture, the pT/NK cells were cultured in MEMα medium containing 10 ng/ml rhIL-15, 10 ng/ml rhFlt3-L, 20 ng/ml rhIL-7, and 20 ng/ml rhSCF in the absence of Notch signaling, to thereby induce differentiation of the pT/NK cells into NK cells. As a comparative control, NK cells were directly induced to differentiate using the culture method (culturing HSPCs in MEMα medium containing 10 ng/ml rhIL-15, 10 ng/ml rhFlt3-L, 20 ng/ml IL-7, and 20 ng/ml rhSCF) of a previous report (Li et al., Cell Stem Cell, Vol. 23, Issue 2, pp. 181-192.e5, August 02, 2018). FIG. 21B shows the proliferation rate of cells from CB-CD34 positive cells. FIG. 21C shows the results of FCM analysis after NK cell generation. FIG. 21D shows the number of produced NK cells when NK cells were directly induced from HSPCs (conventional method) and when the pT/NK cell expansion method was used. From these results, it was revealed that CD56-positive NK cells can be produced in large quantities from pT/NK cells using the present method. Compared with the conventional method, the present method produced 200- to 500-fold more NK cells. Furthermore, according to the method described in A-2-11 above, in which Notch ligand co-culture was performed using feeder cells expressing a Notch ligand when inducing differentiation of pT/NK cells into NK cells, the production amount of NK cells from pT/NK cells was 17-fold higher than that of the conventional method. Comparing the present method, in which culture is performed in the absence of Notch signaling during NK cell differentiation induction, with the method described in A-2-11, the production amount of NK cells by the present method is about 12- to 30-fold higher than that of the method described in A-2-11. Thus, according to the present method, the production amount of NK cells can be further significantly improved.

### B-3. Proliferation ability of human pT/NK cell-derived NK cells

The proliferation ability of pT/NK cell-derived NK cells was evaluated. As comparative controls, NK cells collected from CB (human umbilical cord blood) or PB (human peripheral blood) (CB-NK and PB-NK, respectively) were used. FIG. 22A shows the cell proliferation rate after culturing for 2 weeks in MEMα medium supplemented with 10 ng/ml rhIL-15. FIG. 22B shows the number of cells when NK cells were expanded by mixing with MMC (mitomycin)-treated aAPCs (artificial antigen presenting cells; cells expressing a ligand that stimulates NK cells) at an E:T ratio of 1:2 and culturing in MEMα medium containing 200 IU/ml rhIL-2. Fresh aAPCs were added 1 week after the start of culture, and culture was performed for a total of 2 weeks. As a result, it was revealed that pT/NK-derived NK cells have proliferation ability comparable to that of CB-NK cells and PB-NK cells.

### B-4. Expression of surface antigen markers in human pT/NK cell-derived NK cells (FIG. 23)

The expression levels of NK cell-related receptors in pT/NK-derived NK cells were evaluated by FCM analysis. In pT/NK-derived NK cells, the expression levels of NKp44, NKp46, and NKG2D were comparable to those in CB-NK cells and PB-NK cells. On the other hand, it was revealed that the expression levels of CD16 and KIR2DL2/L3 were lower than those in PB-NK cells, and the expression level of NKG2A was lower than that in CB-NK cells. From these results, pT/NK-derived NK cells were considered to be in an early stage of maturation. In addition, the expression of TIGIT in pT/NK-derived NK cells was markedly lower than that in CB-NK cells and PB-NK cells, suggesting that the pT/NK-derived NK cells have a lower degree of exhaustion.

### B-5. Cytotoxic activity of human pT/NK cell-derived NK cells

The cytotoxic activity of pT/NK cell-derived NK cells against K562 cells was compared with that of PB- or CB-derived NK cells. After mixing NK cells and K562 cells at various E:T ratios, co-culture was performed for 4 hours. After culture, the dead cell ratio of K562 cells was evaluated by FCM analysis (FIG. 24A). In addition, to evaluate the expression level of effector molecules of activated NK cells, NK cells and K562 cells were mixed at an E:T ratio of 1:2 and cultured in MEMα medium containing a 1/500 amount of APC-conjugated CD107a antibody and 1x Protein transport inhibitor. After 4 hours, intracellular staining was performed, and the expression level of cytokines was analyzed by FCM (FIG. 24B). pT/NK-derived NK cells showed high cytotoxic activity comparable to that of CB-NK cells and PB-NK cells. It was also shown that the expression level of effector molecules by K562 cell stimulation was comparable to that of CB-NK cells and PB-NK cells (FIG. 24C).

### B-6. Functions of cryopreserved human pT/NK cell-derived NK cells

The effect of cryopreservation on pT/NK-derived NK cells was evaluated. The number of pT/NK cell-derived NK cells after freeze-thawing was counted, and the recovery rate (number of cells after freezing / number of cells before freezing x 100) was calculated. As a result, 60-100% of the NK cells before freezing were obtained even after freeze-thawing (FIG. 25A). Next, the cell proliferation ability was evaluated by culturing the freeze-thawed pT/NK cell-derived NK cells in a medium supplemented with aAPCs and 200 IU/ml rhIL-2. Since the cells were expanded approximately 200- to 650-fold by co-culturing for 2 weeks, it was confirmed that the proliferation ability of the NK cells was not lost by cryopreservation (FIG. 25B). Subsequently, after co-culturing pT/NK cell-derived NK cells in a medium supplemented with aAPCs and 200 IU/ml rhIL-2, the cytotoxic activity against K562 cells was evaluated. After culturing NK cells and K562 cells at various E:T ratios for 4 hours, the dead cell ratio of K562 cells was measured by FCM analysis. As a result, it was shown that the frozen pT/NK cell-derived NK cells maintained high cytotoxic activity similar to that before freezing (FIG. 25C).

### B-7. Search for factors promoting expansion of human pT/NK cells

In order to search for factors that promote differentiation and expansion of hCB-HSPCs into pT/NK cells, 10 ng/ml of each cytokine or 0.1 mM nicotinamide was added to a system in which hCB-HSPCs were cultured on OP9/DLL1 cells in the presence of 5 ng/ml rhIL-7, 5 ng/ml rhFlt3-L, and 5 ng/ml rhSCF, and the expansion rate of pT/NK cells was calculated. As a result, it was revealed that M-CSF, TPO, IL-6, G-CSF, and nicotinamide promote the differentiation and expansion of pT/NK cells (FIG. 26).

### B-8. Expansion of human pT/NK cells by TPO and M-CSF

The concentration at which TPO or M-CSF promotes the generation of pT/NK cells was studied. 0-50 ng/ml TPO or M-CSF was added to a system in which hCB-HSPCs were cultured on OP9/DLL1 cells in the presence of 5 ng/ml rhIL-7, 5 ng/ml rhFlt3-L, and 5 ng/ml rhSCF, and the proliferation rate of pT/NK cells was examined. As a result, it was revealed that addition of 10-50 ng/ml TPO or 10-25 ng/ml M-CSF promotes pT/NK differentiation and expansion (FIG. 27).

### B-9. NK cell production ability of human pT/NK cells expanded by TPO or M-CSF

The differentiation ability of pT/NK cells expanded by adding TPO or M-CSF into NK cells was evaluated. That is, hCB-HSPCs were cultured on OP9/DLL1 cells for 4 weeks in a medium supplemented with 25 ng/ml TPO or M-CSF in addition to 5 ng/ml rhIL-7, 5 ng/ml rhFlt3-L, and 5 ng/ml rhSCF, to thereby expand pT/NK cells. Next, the pT/NK cells were cultured in MEMα medium containing 10 ng/ml rhIL-15, 10 ng/ml rhFlt3-L, 20 ng/ml rhIL-7, and 20 ng/ml rhSCF to thereby induce differentiation into NK cells. As a result, 4.3-fold more NK cells were obtained with TPO and 1.6-fold more with M-CSF, compared with a case where each cytokine was not added (FIG. 28).

### B-10. Induction from bone marrow HSPCs to pT/NK cells and NK cells by TPO

To clarify whether TPO is also effective for inducing pT/NK from bone marrow-derived HSPCs, human bone marrow-derived CD34 positive cells were cultured for 1 week using Stemline II medium supplemented with 100 ng/ml rhTPO, 100 ng/ml rhSCF, and 100 ng/ml rhG-CSF. Thereafter, the cells were cultured on OP9/DLL1 cells in MEMα medium containing 5 ng/ml rhIL-7, 5 ng/ml rhFlt3-L, 5 ng/ml rhSCF, and 25 ng/ml TPO for 8 weeks to thereby generate and expand pT/NK cells. As a result, it was revealed that the bone marrow-derived pT/NK cells were expanded several-fold by adding TPO (FIG. 29 bottom left). In addition, when NK cells were induced from the pT/NK cells at the 5th week of culture, approximately 100-fold as many NK cells were obtained as compared with a case where TPO was not added (FIG. 29 bottom right).

### B-11. Expression of surface antigen markers of pT/NK cell-derived NK cells induced from human HSPCs

The expression levels of NK cell-related receptors in pT/NK cell-derived NK cells (hBM-pT/NK-derived NK cells) induced from human bone marrow HSPCs were evaluated by FCM analysis. As a result, although expression of NKp44, NKp46, and NKG2D was observed as in hCB blood-derived NK cells, expression of CD16 and KIR (KIR2DL2/L3) was low, suggesting that they are NK cells in an early stage of maturation (FIG. 30A).

FIG. 30B shows the results of comparing the expression of activated receptors, inhibitory receptors, and exhaustion markers in human T/NK progenitor cell-derived NK cells (pT/NK-NK cells) induced from human umbilical cord blood HSPCs, human CB-NK cells, and human PB-NK cells. While CB-NK cells are known to be immature NK cells with a low degree of maturity, the human pT/NK-NK cells were characterized by being even more immature than the CB-NK cells. In addition, the human pT/NK-NK cells were also characterized by having lower expression of the exhaustion marker TIGIT and a lower degree of exhaustion than the CB-NK cells.

FIG. 30C is RNA-seq data comparing gene expression in human pT/NK-NK cells induced from human umbilical cord blood HSPCs, human CB-NK cells, and human PB-NK cells. In human pT/NK-NK cells (NK.pTNK_1 to 3), CCR3, CCR6, CCR7, CCR8, and CCR9 were expressed at higher levels compared with human CB-NK cells (NK.CB_1 to 3) and human PB-NK cells (NK.PB_1 to 3).

### B-12. Cytotoxic activity of NK cells differentiated from bone marrow HSPCs

The cytotoxic activity of hBM-pT/NK cell-derived NK cells against K562 cells was evaluated. After mixing NK cells and K562 cells at various E:T ratios, co-culture was performed for 4 hours. The dead cell ratio of K562 cells after culture was analyzed by FCM. As a result, the hBM-pT/NK cell-derived NK cells showed high anti-cancer activity against K562 cells (FIG. 31).

### B-13. Identification of specific markers for pT/NK cells

Expression of CD122 (IL-2 receptor β chain) in pT/NK cells was evaluated by FCM analysis (FIG. 32A). pT/NK cells contain three fractions: ProT1 (CD7⁺CD5⁻CD1a⁻) cells, ProT2 (CD7⁺CD5⁺CD1a⁻) cells, and PreT (CD7⁺CD5⁺CD1a⁺) cells. CD122 expression was not observed in CD34⁺CD7⁻ cells, which are hematopoietic progenitor cells before receiving Notch signaling; however, it was highly expressed in ProT1 (CD7⁺CD5⁻CD1a⁻) cells and ProT2 (CD7⁺CD5⁺CD1a⁻) cells, and its expression decreased in PreT (CD7⁺CD5⁺CD1a⁺) cells. CD122 is used as a marker for NK progenitor cells in mice (Non-Patent Document 4), but there is no report in humans. That is, CD122 was considered to be a novel marker molecule for human T/NK progenitor cells. To evaluate the proliferation ability of these CD122-positive cells, a CFSE assay was performed (FIG. 32B). After staining in 0.1% BSA/PBS supplemented with 10 µg/ml CFSE, CD122-positive cells were isolated by a cell sorter and cultured on OP9/DLL1 cells in the presence of 5 ng/ml rhIL-7, 5 ng/ml rhFlt3-L, and 5 ng/ml rhSCF for 1 week. Thereafter, the fluorescence intensity of CFSE was analyzed by FCM. As a result, it was shown that the CD122-positive cells have high proliferation ability. Next, the differentiation ability of the CD122-positive cells into T cells and NK cells was evaluated (FIG. 32C). hCB-derived HSPCs were cultured on OP9/DLL1 cells for 2 weeks, and then Lineage marker-negative CD122-positive cells were isolated by a cell sorter, followed by culturing the cells under T-cell and NK-cell induction conditions. Since the CD122-positive cells generated both CD4/CD8 double-positive (DP) cells and CD56-positive NK cells, they were suggested to be pT/NK cells.

### C. Production and functional evaluation of human pT/NK cell-derived CAR-NK cells

### C-1. CAR retroviral vector

An anti-CD19 CAR gene or an anti-HER2 CAR gene was loaded into a pMYs-IG retroviral vector, and pMYs-CD19 CAR-BBz-IG (middle row of FIG. 33) and pMYs-HER2 CAR-BBz-IG (bottom row of FIG. 33) vectors were produced.

### C-2. CAR-NK cells generated from pT/NK cell-derived NK cells

pT/NK cell-derived NK cells were transduced with the CAR gene by a spin infection method using a retrovirus. After isolating CAR-NK cells positive for GFP, which is a marker protein, by a cell sorter, they were further expanded by co-culturing with aAPCs. After culture, the expression of GFP was analyzed by FCM, and it was confirmed that the transgene was stably expressed (FIG. 34A). Next, the antigen-specific cytotoxic activity of the anti-CD19 CAR-NK cells or anti-HER2 CAR-NK cells was evaluated. The pT/NK cell-derived anti-CD19 CAR-NK cells were mixed with a CD19 antigen-positive B-cell leukemia cell line NALM6 (NALM6 cells), and the anti-HER2 CAR-NK cells were mixed with a HER2 antigen-positive breast cancer cell line MDA-MB-453 (MDA-MB-453 cells), at various E:T ratios. After co-culturing for 4 hours, the dead cell ratio of the target cells was measured by FCM analysis. As a result, both the anti-CD19 CAR-NK cells and the anti-HER2 CAR-NK cells showed higher cytotoxic activity against the target cells compared with Mock NK cells (FIG. 34B).

### C-3. Transduction of pT/NK cells with CAR gene

pT/NK cells were transduced with the anti-CD19 CAR gene or anti-HER2 CAR gene, and cells expressing GFP, which is a marker protein, were isolated by a cell sorter. Thereafter, the cells were expanded by culturing them on OP9/DLL1 cells for 2 weeks in the presence of 5 ng/ml rhIL-7, 5 ng/ml rhFlt3-L, and 5 ng/ml rhSCF. FCM analysis of GFP expression in the cultured pT/NK cells showed that the pT/NK cells stably expressed the transgene (FIG. 35).

### C-4. Induction of differentiation from CAR-pT/NK cells into CAR-NK cells

Production of CAR-NK cells from pT/NK cells transduced with a CAR gene (CAR-pT/NK cells) was attempted. FIG. 36A shows a scheme for producing CAR-NK cells from CAR-pT/NK cells. CAR-pT/NK cells were expanded by culturing them on OP9/DLL1 cells in the presence of 5 ng/ml rhIL-7, 5 ng/ml rhFlt3-L, and 5 ng/ml rhSCF. Thereafter, the cells were cultured in MEMα medium containing 10 ng/ml rhIL-15, 10 ng/ml rhFlt3-L, 20 ng/ml IL-7, and 20 ng/ml rhSCF to thereby induce them into NK cells. The obtained CAR-NK cells were expanded by culturing them in a medium containing aAPCs and 200 IU/ml rhIL-2 for 2 weeks. FIG. 36B shows the results of FCM analysis of the CAR-pT/NK cell-derived CAR-NK cells on Day 42 of culture, and it can be confirmed that NK cells stably expressing the transgene were produced. It was also revealed that approximately 1 x 10⁴ CAR-NK cells were produced per CAR-pT/NK cell (FIG. 36C).

### C-5. Cytotoxic activity of CAR-pT/NK cell-derived CAR-NK cells

The cytotoxic activity of CAR-pT/NK cell-derived CAR-NK cells against target cells was evaluated. Anti-CD19 CAR-NK cells and NALM6 cells, or anti-HER2 CAR-NK cells and MDA-MB-453 cells, were respectively co-cultured for 4 hours and then analyzed by FCM. As a result, both cells were shown to have antigen-specific cytotoxic activity (FIG. 37).

### C-6. Expression of cell surface markers of CAR-pT/NK cell-derived CAR-NK cells

The expression levels of representative NK cell-related receptors in pT/NK-derived anti-HER2 CAR-NK cells, CB-derived anti-HER2 CAR-NK cells, and PB-derived anti-HER2 CAR-NK cells were analyzed by FCM. The results are shown in FIG. 38. The pT/NK cell-derived anti-HER2 CAR-NK cells showed an NK cell phenotype of a more early stage of maturation compared with the CB-derived anti-HER2 CAR-NK cells and the PB-derived anti-HER2 CAR-NK cells. In addition, the pT/NK cell-derived anti-HER2 CAR-NK cells had a lower expression level of TIGIT compared with the CB-derived anti-HER2 CAR-NK cells and the PB-derived anti-HER2 CAR-NK cells.

### C-7. Cytotoxic activity of CAR-pT/NK cell-derived CAR-NK cells

After co-culturing pT/NK-, CB-NK-, and PB-NK-derived anti-HER2 CAR-NK cells each with MDA-MB-453 for 4 hours, the dead cell ratio of MDA-MB-453 cells was measured by FCM analysis. As a result, the pT/NK cell-derived anti-HER2 CAR-NK cells showed superior anti-cancer activity to the CB-derived anti-HER2 CAR-NK cells and the PB-derived anti-HER2 CAR-NK cells (FIG. 39).

### C-8. In vivo anti-tumor activity of CAR-pT/NK cell-derived CAR-NK cells

The *in vivo* anti-tumor activity of pT/NK cell-derived anti-HER2 CAR-NK cells was evaluated. First, 1 x 10⁶ MDA-MB-453 cells stably expressing luciferase were subcutaneously transplanted into NOD. Cg-Prkdc^{scid}/IL2rg^{tmlSug}/ShiJic (NOG) mice to thereby produce tumor-bearing mice. At 14, 17, 21, and 24 days after the MDA-MB-453 cell transplantation, 5 x 10⁶ CAR-NK cells were administered via the tail vein. In addition, 50,000 IU rhIL-2 was intraperitoneally administered every 2-3 days for a total of 13 times. Tumor growth was measured by bioluminescence imaging. As a result, while tumor growth suppression was not observed in the groups administered with CB-derived anti-HER2 CAR-NK cells and pT/NK-derived Mock NK cells, tumor growth was markedly suppressed in the group administered with pT/NK-derived anti-HER2 CAR-NK cells (FIG. 40B, top row of FIG. 40C). In addition, no body weight loss was confirmed in any of the NK cell administration groups (bottom row of FIG. 40C), and no serious side effects were observed.

## Claims

1. A method of producing an immune cell expressing an exogenous receptor that is a T-cell receptor (TCR), a chimeric antigen receptor (CAR), or a chimeric autoantigen receptor (CAAR), the method comprising the steps of:
preparing multipotent blood progenitor cells from a cell population comprising hematopoietic stem cells;
preparing multipotent blood progenitor cells expressing said exogenous receptor by introducing a gene encoding said exogenous receptor into the multipotent blood progenitor cells; and
inducing differentiation of the multipotent blood progenitor cells expressing said exogenous receptor into immune cells.

2. A method of producing an immune cell expressing an exogenous receptor that is a TCR, a CAR, or a CAAR, the method comprising the steps of:
preparing multipotent blood progenitor cells from a cell population comprising hematopoietic stem cells;
inducing differentiation of the multipotent blood progenitor cells into immune cells; and
introducing a gene encoding said exogenous receptor into the immune cells,
wherein the multipotent blood progenitor cell is an induced leukocyte stem cell or a T/NK progenitor cell.

3. The method according to claim 1, wherein the multipotent blood progenitor cell is an induced leukocyte stem cell or a T/NK progenitor cell.

4. The method according to claim 1 or 2, wherein the multipotent blood progenitor cell is an induced leukocyte stem cell, and in the step of preparing multipotent blood progenitor cells, induced leukocyte stem cells are prepared by culturing a cell population comprising hematopoietic stem cells under conditions that induce differentiation into B cells while inhibiting a function of transcription factor E2A in the cells.

5. The method according to claim 4, wherein the inhibition of the function of E2A is performed by forced expression of a differentiation inhibitory factor Id in the hematopoietic stem cells.

6. The method according to claim 1 or 2, wherein the multipotent blood progenitor cell is a T/NK progenitor cell, and in the step of preparing multipotent blood progenitor cells, T/NK progenitor cells are prepared by culturing a cell population comprising hematopoietic stem cells in the presence of a Notch ligand.

7. The method according to claim 6, wherein the cell population comprising hematopoietic stem cells is cultured in a medium comprising SCF, TPO, and G-CSF at concentrations of 100 ng/mL or more each in the absence of a Notch ligand, and then cultured in the presence of a Notch ligand.

8. The method according to any one of claims 1 to 7, wherein the immune cell is a T cell, an NK cell, a macrophage, or a B cell.

9. The method according to claim 8, wherein the exogenous receptor is a TCR and the immune cell is a T cell.

10. The method according to claim 8, wherein the exogenous receptor is a CAR or a CAAR, and the immune cell is a T cell, an NK cell, or a macrophage.

11. The method according to any one of claims 1 to 10, wherein the exogenous receptor is an antigen-specific receptor and the antigen is a tumor antigen.

12. A method of producing an immune cell therapy agent for a tumor or an autoimmune disease, the method comprising the steps of:
preparing multipotent blood progenitor cells from a cell population comprising hematopoietic stem cells;
introducing a gene encoding an exogenous receptor into the multipotent blood progenitor cells to prepare multipotent blood progenitor cells expressing the exogenous receptor, wherein the exogenous receptor is a CAAR, or a TCR or CAR that specifically recognizes a tumor antigen;
inducing differentiation of the multipotent blood progenitor cells expressing said exogenous receptor into T cells to prepare T cells expressing said exogenous receptor (TCR-T, CAR-T, or CAAR-T cells);
subjecting the TCR-T, CAR-T, or CAAR-T cells to TCR stimulation to induce differentiation into CD8-positive cytotoxic TCR-T, CAR-T, or CAAR-T cells; and
preparing the CD8-positive cytotoxic TCR-T or CAR-T cells as an immune cell therapy agent for a tumor expressing said tumor antigen, or preparing the CD8-positive cytotoxic CAAR-T cells as an immune cell therapy agent for an autoimmune disease.

13. A method of producing an immune cell therapy agent for a tumor or an autoimmune disease, the method comprising the steps of:
preparing multipotent blood progenitor cells from a cell population comprising hematopoietic stem cells;
inducing differentiation of the multipotent blood progenitor cells into T cells;
subjecting the T cells to TCR stimulation and then introducing thereinto a gene encoding an exogenous receptor, wherein the exogenous receptor is a CAAR, or a TCR or CAR that specifically recognizes a tumor antigen, and thereby preparing CD8-positive cytotoxic TCR-T, CAR-T, or CAAR-T cells; and
preparing the CD8-positive cytotoxic TCR-T or CAR-T cells as an immune cell therapy agent for a tumor expressing said tumor antigen, or preparing the CD8-positive cytotoxic CAAR-T cells as an immune cell therapy agent for an autoimmune disease,
wherein the multipotent blood progenitor cell is an induced leukocyte stem cell or a T/NK progenitor cell.

14. A method of producing an immune cell therapy agent for a tumor or an autoimmune disease, the method comprising the steps of:
preparing multipotent blood progenitor cells from a cell population comprising hematopoietic stem cells;
introducing a gene encoding a chimeric receptor into the multipotent blood progenitor cells, wherein the chimeric receptor is a CAAR, or a CAR that specifically recognizes a tumor antigen, and thereby preparing multipotent blood progenitor cells expressing said chimeric receptor;
inducing differentiation of the multipotent blood progenitor cells expressing said chimeric receptor into NK cells or macrophages to prepare NK cells (CAR-NK or CAAR-NK cells) or macrophages (CAR-macrophages or CAAR-macrophages), each expressing said chimeric receptor; and
preparing the CAR-NK cells or CAR-macrophages as an immune cell therapy agent for a tumor expressing said tumor antigen, or preparing the CAAR-NK cells or CAAR-macrophages as an immune cell therapy agent for an autoimmune disease.

15. The method according to claim 14, further comprising a step of subjecting the prepared NK cells or macrophages expressing said chimeric receptor to receptor stimulation, wherein the chimeric receptor-expressing NK cells or macrophages after the receptor stimulation are prepared as the immune cell therapy agent.

16. A method of producing an immune cell therapy agent for a tumor or an autoimmune disease, the method comprising the steps of:
preparing multipotent blood progenitor cells from a cell population comprising hematopoietic stem cells;
inducing differentiation of the multipotent blood progenitor cells into NK cells or macrophages;
subjecting the NK cells or the macrophages to receptor stimulation and then introducing thereinto a gene encoding a chimeric receptor, wherein the chimeric receptor is a CAAR, or a CAR that specifically recognizes a tumor antigen, and thereby preparing NK cells (CAR-NK or CAAR-NK cells) or macrophages (CAR-macrophages or CAAR-macrophages), each expressing said chimeric receptor; and
preparing the CAR-NK cells or CAR-macrophages as an immune cell therapy agent for a tumor expressing said tumor antigen, or preparing the CAAR-NK cells or CAAR-macrophages as an immune cell therapy agent for an autoimmune disease,
wherein the multipotent blood progenitor cell is an induced leukocyte stem cell or a T/NK progenitor cell.

17. A method of treating a tumor or an autoimmune disease in a patient, comprising the steps of:
preparing multipotent blood progenitor cells from a cell population comprising hematopoietic stem cells;
introducing a gene encoding an exogenous receptor into the multipotent blood progenitor cells to prepare multipotent blood progenitor cells expressing the exogenous receptor, wherein the exogenous receptor is a CAAR, or a TCR or CAR that specifically recognizes a tumor antigen;
inducing differentiation of the multipotent blood progenitor cells expressing said exogenous receptor into T cells to prepare TCR-T, CAR-T, or CAAR-T cells that are T cells expressing said exogenous receptor;
subjecting the TCR-T, CAR-T, or CAAR-T cells to TCR stimulation to induce differentiation into CD8-positive cytotoxic TCR-T, CAR-T, or CAAR-T cells; and
administering the CD8-positive cytotoxic TCR-T or CAR-T cells to a patient having a tumor expressing said tumor antigen, or administering the CD8-positive cytotoxic CAAR-T cells to a patient with an autoimmune disease.

18. A method of treating a tumor or an autoimmune disease in a patient, comprising the steps of:
preparing multipotent blood progenitor cells from a cell population comprising hematopoietic stem cells;
inducing differentiation of the multipotent blood progenitor cells into T cells;
subjecting the T cells to TCR stimulation and then introducing thereinto a gene encoding an exogenous receptor, wherein the exogenous receptor is a CAAR, or a TCR or CAR that specifically recognizes a tumor antigen, and thereby preparing CD8-positive cytotoxic TCR-T, CAR-T, or CAAR-T cells; and
administering the CD8-positive cytotoxic TCR-T or CAR-T cells to a patient having a tumor expressing said tumor antigen, or administering the CD8-positive cytotoxic CAAR-T cells to a patient with an autoimmune disease,
wherein the multipotent blood progenitor cell is an induced leukocyte stem cell or a T/NK progenitor cell.

19. A method of treating a tumor or an autoimmune disease in a patient, comprising the steps of:
preparing multipotent blood progenitor cells from a cell population comprising hematopoietic stem cells;
introducing a gene encoding a chimeric receptor into the multipotent blood progenitor cells to prepare multipotent blood progenitor cells expressing the chimeric receptor, wherein the chimeric receptor is a CAAR, or a CAR that specifically recognizes a tumor antigen;
inducing differentiation of the multipotent blood progenitor cells expressing said chimeric receptor into NK cells or macrophages to prepare chimeric receptor-expressing immune cells that are CAR-NK cells, CAAR-NK cells, CAR-macrophages or CAAR-macrophages; and
administering the CAR-NK cells or CAR-macrophages to a patient having a tumor expressing said tumor antigen, or administering the CAAR-NK cells or CAAR-macrophages to a patient with an autoimmune disease.

20. The method according to claim 19, further comprising a step of subjecting the prepared chimeric receptor-expressing immune cells to receptor stimulation, wherein the chimeric receptor-expressing immune cells after the receptor stimulation are administered to the patient.

21. A method of treating a tumor or an autoimmune disease in a patient, comprising the steps of:
preparing multipotent blood progenitor cells from a cell population comprising hematopoietic stem cells;
inducing differentiation of the multipotent blood progenitor cells into NK cells or macrophages;
subjecting the NK cells or macrophages to receptor stimulation and then introducing thereinto a gene encoding a chimeric receptor, wherein the chimeric receptor is a CAAR, or a CAR that specifically recognizes a tumor antigen, and thereby preparing CAR-NK cells, CAAR-NK, CAR-macrophages or CAAR-macrophages; and
administering the CAR-NK cells or CAR-macrophages to a patient having a tumor expressing said tumor antigen, or administering the CAAR-NK cells or CAAR-macrophages to a patient with an autoimmune disease,
wherein the multipotent blood progenitor cell is an induced leukocyte stem cell or a T/NK progenitor cell.

22. A method of treating a tumor in a patient, comprising the steps of:
preparing CAR-macrophages by the following (1) or (2):
(1) preparing multipotent blood progenitor cells from a cell population comprising hematopoietic stem cells, introducing a gene encoding a CAR that specifically recognizes a tumor antigen into the multipotent blood progenitor cells to thereby prepare multipotent blood progenitor cells expressing said CAR, and then inducing differentiation of the CAR-expressing multipotent blood progenitor cells into macrophages;
(2) preparing induced leukocyte stem cells or T/NK progenitor cells from a cell population comprising hematopoietic stem cells, inducing differentiation thereof into macrophages, subjecting the macrophages to receptor stimulation, and then introducing thereinto a gene encoding said CAR;
preparing CAR-T cells by the following (3) or (4):
(3) preparing multipotent blood progenitor cells from a cell population comprising hematopoietic stem cells, introducing a gene encoding said CAR into the multipotent blood progenitor cells to thereby prepare multipotent blood progenitor cells expressing said CAR, inducing differentiation of the CAR-expressing multipotent blood progenitor cells into T cells, and then subjecting the T cells to TCR stimulation to thereby induce differentiation into CD8-positive cytotoxic TCR-T or CAR-T cells;
(4) preparing induced leukocyte stem cells or T/NK progenitor cells from a cell population comprising hematopoietic stem cells, inducing differentiation thereof into T cells, subjecting the T cells to TCR stimulation, and then introducing thereinto a gene encoding said CAR; and
administering the CAR-macrophages and the CAR-T cells in combination to a patient having a tumor expressing said tumor antigen.

23. A method of treating a tumor in a patient, comprising the steps of:
preparing CAR-macrophages by the following step (1) or (2):
(1) preparing multipotent blood progenitor cells from a cell population comprising hematopoietic stem cells, introducing a gene encoding a CAR that specifically recognizes a tumor antigen into the multipotent blood progenitor cells to thereby prepare multipotent blood progenitor cells expressing said CAR, and then inducing differentiation of the CAR-expressing multipotent blood progenitor cells into macrophages;
(2) preparing induced leukocyte stem cells or T/NK progenitor cells from a cell population comprising hematopoietic stem cells, inducing differentiation thereof into macrophages, subjecting the macrophages to receptor stimulation, and then introducing a gene encoding said CAR;
preparing CAR-NK cells by the following step (5) or (6):
(5) preparing multipotent blood progenitor cells from a cell population comprising hematopoietic stem cells, introducing a gene encoding said CAR into the multipotent blood progenitor cells to thereby prepare multipotent blood progenitor cells expressing said CAR, and then inducing differentiation of the CAR-expressing multipotent blood progenitor cells into NK cells;
(6) preparing induced leukocyte stem cells or T/NK progenitor cells from a cell population comprising hematopoietic stem cells, inducing differentiation thereof into NK cells, subjecting the NK cells to receptor stimulation, and then introducing a gene encoding said CAR; and
administering the CAR-macrophages and the CAR-NK cells in combination to a patient having a tumor expressing said tumor antigen.

24. The method according to claim 23, wherein the step (5) further comprises, after inducing differentiation of the CAR-expressing multipotent blood progenitor cells into NK cells to prepare the CAR-NK cells, subjecting the CAR-NK cells to receptor stimulation.

25. The method according to any one of claims 22 to 24, wherein the step (1) further comprises, after inducing differentiation of the CAR-expressing multipotent blood progenitor cells into macrophages to prepare the CAR-macrophages, subjecting the CAR-macrophages to receptor stimulation.

26. A method of producing human T/NK progenitor cells, comprising a step of culturing a cell population comprising human hematopoietic stem cells in the presence of a Notch ligand in a medium comprising SCF, Flt3-L, and IL-7 at concentrations of 1 ng/mL or more and less than 10 ng/mL each.

27. The method according to claim 26, wherein the medium further comprises at least one of TPO and M-CSF.

28. The method according to any one of claims 26 to 27, wherein the cell population comprising human hematopoietic stem cells is a cell population obtained after culturing a cell population comprising human hematopoietic stem cells in a medium comprising SCF, TPO, and G-CSF at concentrations of 100 ng/mL or more each in the absence of a Notch ligand.

29. A method of producing human NK cells, comprising the steps of:
producing human T/NK progenitor cells by the method according to any one of claims 26 to 28; and
culturing the produced human T/NK progenitor cells in a medium comprising IL-7, Flt3-L, SCF, and IL-15 in the absence of a Notch ligand to induce differentiation into human NK cells.

30. The method of producing said immune cell according to claim 6 or 7, wherein the T/NK progenitor cell is a human T/NK progenitor cell, and in the step of preparing multipotent blood progenitor cells, human T/NK progenitor cells are produced from a cell population comprising human hematopoietic stem cells by the method according to any one of claims 26 to 28.

31. The method of producing said immune cell according to claim 30, wherein the immune cell is a human NK cell, and in the step of inducing differentiation of the multipotent blood progenitor cells into immune cells, differentiation into human NK cells is induced by culturing the human T/NK progenitor cells in a medium comprising IL-7, Flt3-L, SCF, and IL-15 in the absence of a Notch ligand.

32. A cultured human T/NK progenitor cell, **characterized by** being CD122-positive.

33. A cultured human NK cell, **characterized by** being more immature and having a lower degree of exhaustion than human umbilical cord blood NK cells isolated from human umbilical cord blood, due to lower expression levels of maturity indicators CD16 and KIR and exhaustion marker TIGIT compared to those of said human umbilical cord blood NK cells.
